# EUROPEAN PATENT APPLICATION

(11) **EP 4 382 525 A1**
(43) Date of publication of application: **12.06.2024**
(21) Application number: 22852251.2
(22) Date of filing: 03.08.2022
(51) Int. Cl.: C07D 405/14, C07D 407/14, A61K 31/435, A61K 31/4184, A61K 31/4188, A61P 3/10, A61P 3/04

(54) **CYCLOALKENE DERIVATIVE REGULATOR, PREPARATION METHOD THEREFOR, AND APPLICATION THEREOF**

(30) Priority: 04.08.2021 CN 202110892791; 09.11.2021 CN 202111322097; 14.01.2022 CN 202210041958; 17.05.2022 CN 202210540040
(71) Applicant: Shanghai Hansoh Biomedical Co., Ltd., Shanghai 201203 (CN); Jiangsu Hansoh Pharmaceutical Group Co., Ltd., Lianyungang, Jiangsu 222047 (CN)
(72) Inventor: SU, Yidong, Shanghai 201203 (CN); MAO, Xiaofeng, Shanghai 201203 (CN); WANG, Jun, Shanghai 201203 (CN); ZHOU, Xiaohan, Shanghai 201203 (CN); YU, Wensheng, Shanghai 201203 (CN)
(74) Representative: EP&C
(86) International application number: PCT/CN2022/110017
(87) International publication number: WO 2023/011539

(57) **Abstract**

Provided are a polycyclic derivative regulator, a preparation method therefor, and an application thereof. In particular, provided are a compound represented by general formula (I'), a preparation method therefor, a pharmaceutical composition comprising the compound, and an application thereof as a regulator in the preparation of a drug for treating metabolic diseases and related diseases; the substituents in general formula (I') are the same as those defined in the description.

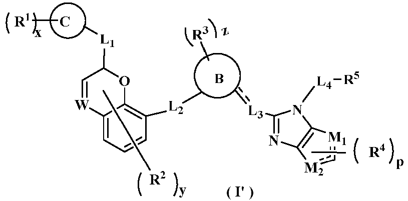

## Description

### FIELD OF THE INVENTION

The present disclosure belongs to the field of biomedicine, specifically related to a cycloalkene derivative regulator, preparation method therefor and application thereof.

### BACKGROUND OF THE INVENTION

Diabetes mellitus is a common endocrine and metabolic disease, which causes metabolic disorders due to multiple reasons, leading to damage to multiple systems and organs. The incidence is high, there are about 425 million diabetes patients in the world, the incidence of diabetes in China is about 10%, of which type II diabetes accounts for 90%, moreover, the prevalence is still increasing, and the age of disease is becoming younger and younger.

At present, there are several types of drugs on the market for type II diabetes, including insulin, biguanides, glucagon-likepeptide 1 (GLP-1) receptor agonists, dipeptidyl peptidase (DPP-IV) inhibitors, sodium-glucose cotransporter 2 (SGLT-2) inhibitors, α-glucosidase inhibitors, etc. among them, GLP-1 receptor agonists are the most concerned.

GLP-1 is a peptide hormone secreted by human intestinal L cells, and its receptors are distributed in islet cells, various gastrointestinal cells, central nervous system and peripheral nervous system neurons. After activation, GLP-1 receptor has physiological effects such as promoting insulin secretion, inhibiting glucagon secretion, inhibiting appetite and delaying gastric emptance. Clinical evidence shows that compared to other hypoglycemic drugs, GLP-1 receptor agonists have better hypoglycemic effects and are less prone to side effects such as hypoglycemia. In addition, it also has additional cardiovascular benefits and can reduce food intake and delay gastric emptying, which is beneficial for weight control.

Currently marketed GLP-1 receptor agonists are polypeptide drugs, most of which require subcutaneous administration, resulting in poor patient compliance, and the bioavailability of oral peptides is very low. So there is a great clinical demand for developing oral small molecule GLP-1 receptor agonists.

At present, there are no approved small molecule GLP-1 receptor agonists, and three small molecule GLP-1 receptor agonists have entered the clinical research stage, such as PF-06882961 and PF-07081532 developed by Pifzer, and TTP273 developed by vTv, all of which are currently in the clinical phase I/II research stage. Among them, PF-06882961 has shown significant hypoglycemic and weight reducing effects in early clinical practice, and its safety is similar to that of polypeptide GLP-1 receptor agonist, which is expected to bring more treatment options for diabetes, obesity and NASH patients in the future.

There is a huge clinical demand for GLP-1 receptor agonists. Oral small molecule GLP-1 receptor agonists with lower costs and better compliance have the potential to treat various metabolic diseases and have broad market prospects.

### SUMMARY OF THE INVENTION

The object of the present invention is to provide a compound of general formula (I-A), a stereoisomer thereof or a pharmaceutically acceptable salt thereof: 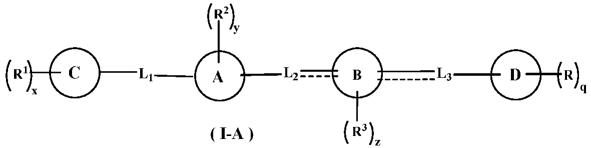 wherein:
L₁ is selected from the group consisting of a bond, alkenylene, alkynylene, - (CH₂)ₙ₁-, -(CH₂)ₙ₁(CRₐₐR_{bb})ₙ₂-, -(CRₐₐR_{bb})ₙ₁O(CH₂)ₙ₂-, -(CH₂)ₙ₁O(CRₐₐR_{bb})ₙ₂-, - (CRₐₐR_{bb})ₙ₁S(CH₂)ₙ₂-, -(CH₂)ₙ₁S(CRₐₐR_{bb})ₙ₂-, -(CRₐₐR_{bb})ₙ₁(CH₂)ₙ₂NR_{cc}-, - (CH₂)ₙ₁NRₐₐ(CR_{bb}R_{cc})ₙ₂-, -(CH₂)ₙ₁C(O)(CRₐₐR_{bb})ₙ₂-, -(CH₂)ₙ₁NRₐₐC(O)(CRₐₐR_{bb})ₙ₂-, - (CH₂)ₙ₁P(O)Rₐₐ-, -(CH₂)ₙ₁S(O)ₘ₁-, -(CH₂)ₙ₁S(O)ₘ₁NRₐₐ-, -(CH₂)ₙ₁NRₐₐS(O)ₘ₁- and - (CH₂)ₙ₁C(O)NRₐₐ-;
Rₐₐ, R_{bb}, and R_{cc} are each independently selected from the group consisting of hydrogen, deuterium, halogen, amino, nitro, hydroxy, cyano, carboxy, alkyl, deuterated alkyl, haloalkyl, hydroxyalkyl, alkoxy, haloalkoxy, deuterated alkoxy, alkenyl, alkynyl, heterocyclylalkyl, cycloalkyl, heterocyclyl, aryl and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, any two of Rₐₐ, R_{bb}, and R_{cc} are bonded to form a cycloalkyl, heterocyclyl, aryl or heteroaryl, wherein the cycloalkyl, heterocyclyl, aryl and heteroaryl can be each optionally further substituted;
L₂ is selected from the group consisting of a bond, alkenylene, alkynylene, - (CH₂)n3-, -(CH₂)ₙ₃(CRₐ₁R_{b1})n4-, -(CRₐ₁R_{b1})ₙ₃O(CH₂)n4-, -(CH₂)ₙ₃O(CRₐ₁R_{b1})ₙ₄-, - (CRₐ₁R_{b1})ₙ₃S(CH₂)ₙ₄-, -(CH₂)ₙ₃S(CRₐ₁R_{b1})ₙ₄-, -(CRₐ₁R_{b1})ₙ₃(CH₂)ₙ₄NR_{c1}-, - (CH₂)ₙ₃NRₐ₁(CR_{b1}R_{c1})ₙ₄-, -(CH₂)ₙ₃C(O)(CRₐ₁R_{b1})ₙ₄-, -(CH₂)ₙ₃NRₐ₁C(O)(CRₐ₁R_{b1})ₙ₄-, - (CH₂)ₙ₃P(O)Rₐ₁-, -(CH₂)ₙ₃S(O)ₘ₂-, -(CH₂)ₙ₃S(O)ₘ₂NRₐ₁-, -(CH₂)ₙ₃NRₐ₁S(O)ₘ₂- and - (CH₂)ₙ₃C(O)NRₐ₁-;
Rₐ₁, R_{b1} and R_{c1} are each independently selected from the group consisting of hydrogen, deuterium, halogen, amino, nitro, hydroxy, cyano, carboxy, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, Rₐ₁ is absent;
or, any two of Rₐ₁, R_{b1} and R_{c1} are bonded to form a cycloalkyl, heterocyclyl, aryl or heteroaryl, wherein the cycloalkyl, heterocyclyl, aryl and heteroaryl can be each optionally further substituted;
L₃ is selected from the group consisting of a bond, alkenylene, alkynylene, - (CH₂)ₙ₅-, -(CH₂)ₙ₅(CRₐ₂R_{b2})ₙ₆-, -(CRₐ₂R_{b2})ₙ₅O(CH₂)ₙ₆-, -(CH₂)ₙ₅O(CRₐ₂R_{b2})ₙ₆-, - (CRₐ₂R_{b2})ₙ₅S(CH₂)ₙ₆-, -(CH₂)ₙ₅S(CRₐ₂R_{b2})ₙ₆-, -(CRₐ₂R_{b2})ₙ₅(CH₂)ₙ₆NR_{c2}-, - (CH₂)ₙ₅NRₐ₂(CR_{b2}R_{c2})ₙ₆-, -(CH₂)ₙ₅C(O)(CRₐ₂R_{b2})ₙ₆-, -(CH₂)ₙ₅NRₐ₂C(O)(CRₐ₂R_{b2})ₙ₆-, - (CH₂)ₙ₅P(O)Rₐ₂-, -(CH)ₙ(CH₂)ₙ₅S(O)ₘ₃-, -(CH₂)ₙ₅S(O)ₘ₃NRₐ₂-, -(CH₂)ₙ₅NRₐ₂S(O)ₘ₃-and -(CH₂)ₙ₅C(O)NRₐ₂-;
Rₐ₂, R_{b2} and R_{c2} are each independently selected from the group consisting of hydrogen, deuterium, halogen, amino, nitro, hydroxy, cyano, oxo, thioxo, carboxy, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, Rₐ₂ is absent;
or, any two of Rₐ₂, R_{b2} and R_{c2} are bonded to form a cycloalkyl, heterocyclyl, aryl or heteroaryl, wherein the cycloalkyl, heterocyclyl, aryl and heteroaryl can be each optionally further substituted;
ring A is selected from the group consisting of cycloalkyl, heterocyclyl, aryl and heteroaryl, wherein the cycloalkyl, heterocyclyl, aryl, and heteroaryl can be each optionally further substituted;
or, ring A is absent;
ring B is selected from the group consisting of cycloalkyl, heterocyclyl, aryl and heteroaryl, wherein the cycloalkyl, heterocyclyl, aryl, and heteroaryl can be each optionally further substituted;
or, ring B is absent;
ring C is selected from the group consisting of cycloalkyl, heterocyclyl, aryl and heteroaryl, wherein the cycloalkyl, heterocyclyl, aryl, and heteroaryl can be each optionally further substituted;
or, ring C is absent;
ring D is selected from the group consisting of cycloalkyl, heterocyclyl, aryl and heteroaryl, wherein the cycloalkyl, heterocyclyl, aryl, and heteroaryl can be each optionally further substituted;
or, ring D is absent;
R¹ is each independently selected from the group consisting of hydrogen, deuterium, halogen, amino, nitro, hydroxy, cyano, alkyl, deuterated alkyl, haloalkyl, hydroxyalkyl, alkoxy, haloalkoxy, deuterated alkoxy, alkenyl, alkynyl, heterocyclylalkyl, cycloalkyl, heterocyclyl, aryl, and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, any two of R¹ are bonded to form a cycloalkyl, heterocyclyl, aryl or heteroaryl, wherein the cycloalkyl, heterocyclyl, aryl and heteroaryl can be each optionally further substituted;
or, R¹ and L₁ are bonded to form a cycloalkyl, heterocyclyl, aryl or heteroaryl, wherein the cycloalkyl, heterocyclyl, aryl and heteroaryl can be each optionally further substituted;
R² is each independently selected from the group consisting of hydrogen, deuterium, halogen, amino, nitro, hydroxy, cyano, oxo, thioxo, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, any two of R² are bonded to form a cycloalkyl, heterocyclyl, aryl or heteroaryl, wherein the cycloalkyl, heterocyclyl, aryl and heteroaryl can be each optionally further substituted;
or, R² and L₁ are bonded to form a cycloalkyl, heterocyclyl, aryl or heteroaryl, wherein the cycloalkyl, heterocyclyl, aryl and heteroaryl can be each optionally further substituted;
or, R² and L₂ are bonded to form a cycloalkyl, heterocyclyl, aryl or heteroaryl, wherein the cycloalkyl, heterocyclyl, aryl and heteroaryl can be each optionally further substituted;
or, R² and R³ are bonded to form a cycloalkyl, heterocyclyl, aryl or heteroaryl, wherein the cycloalkyl, heterocyclyl, aryl and heteroaryl can be each optionally further substituted;
R³ is each independently selected from the group consisting of hydrogen, deuterium, halogen, amino, nitro, hydroxy, cyano, thiol, oxo, thioxo, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, any two of R³ are bonded to form a cycloalkyl, heterocyclyl, aryl or heteroaryl, wherein the cycloalkyl, heterocyclyl, aryl and heteroaryl can be each optionally further substituted;
or, R³ and L₂ are bonded to form a cycloalkyl, heterocyclyl, aryl or heteroaryl, wherein the cycloalkyl, heterocyclyl, aryl and heteroaryl can be each optionally further substituted;
or, R³ and L₃ are bonded to form a cycloalkyl, heterocyclyl, aryl or heteroaryl, wherein the cycloalkyl, heterocyclyl, aryl and heteroaryl can be each optionally further substituted;
R is each independently selected from the group consisting of hydrogen, deuterium, halogen, amino, nitro, hydroxy, carboxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted; 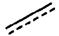
is a single bond or a double bond;
x is an integer from 0 to 12;
y is an integer from 0 to 12;
z is an integer from 0 to 12;
m1~m3 are integers from 0 to 2; and
n1~n6 are integers from 0 to 3.

In some further preferred embodiments of the present invention, the compound is further shown as general formula (I') or (I"): 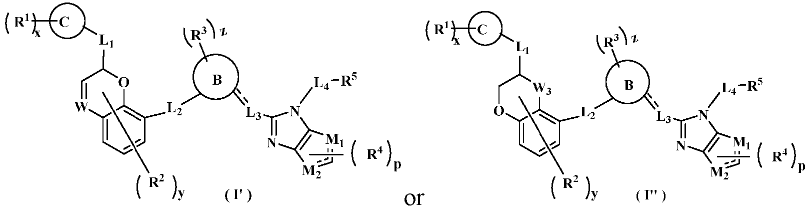
R¹, R², R³, L₁, L₂, L₃, x, y, z, and ring B are as defined above;
W is selected from CRₘ or N;
W₃ is selected from CRₘ₁Rₘ₂ or NRₘ₃;
ring C is selected from aryl or heteroaryl, preferably phenyl or pyridinyl ;
L₄ is selected from the group consisting of a bond, -(CH₂)ₙ₇-, -(CH₂)ₙ₇(CRₐ₃R_{b3})ₙ₈-, -(CRₐ₃R_{b3})ₙ₇O(CH₂)ₙ₈-, -(CH₂)ₙ₇O(CRₐ₃R_{b3})ₙ₈-, -(CRₐ₃R_{b3})ₙ₇S(CH₂)ₙ₈-, - (CH₂)ₙ₇S(CRₐ₃R_{b3})ₙ₈-, -(CRₐ₃R_{b3})ₙ₇(CH₂)ₙ₈NR_{c3}-, -(CH₂)ₙ₇NRₐ₃(CR_{b3}R_{c3})ₙ₈-, - (CH₂)ₙ₇C(O)(CRₐ₃R_{b3})ₙ₈-, -(CH₂)ₙ₇NRₐ₃C(O)(CRₐ₃R_{b3})ₙ₈-, -(CH₂)ₙ₇P(O)Rₐ₃-, - (CH₂)ₙ₇S(O)ₘ₄-, -(CH₂)ₙ₇S(O)ₘ₄NRₐ₃-, -(CH₂)ₙ₇NRₐ₃S(O)ₘ₄- and -(CH₂)ₙ₇C(O)NRₐ₃-;
Rₐ₃, R_{b3} and R_{c3} are each independently selected from the group consisting of hydrogen, deuterium, halogen, amino, nitro, hydroxy, cyano, oxo, thioxo, carboxy, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
or, any two of Rₐ₃, R_{b3} and R_{c3} are bonded to form a cycloalkyl, heterocyclyl, aryl or heteroaryl, optionally can be further substituted;
R⁴ is each independently selected from the group consisting of hydrogen, deuterium, halogen, amino, nitro, hydroxy, carboxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
R⁵ is selected from the group consisting of hydrogen, deuterium, halogen, amino, nitro, hydroxy, cyano, oxo, thioxo, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
Rₘ, Rₘ₁, Rₘ₂ and Rₘ₃ are each independently selected from the group consisting of hydrogen, deuterium, halogen, amino, nitro, hydroxy, cyano, oxo, thioxo, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
M₁ is selected from the group consisting of O, S, N, C, (CH)ₙ₉, (CH₂)ₙ₉O, (CH₂)ₙ₉S, (CH₂)ₙ₉NH and (CH₂)ₙ₉;
M₂ is selected from the group consisting of O, S, N, C, (CH)ₙ₁₀, (CH₂)ₙ₁₀O, (CH₂)ₙ₁₀S, (CH₂)ₙ₁₀NH and (CH₂)ₙ₁₀;
p is an integer from 0 to 4;
n7~n10 are each independently integer from 0 to 3;
m4 is an integer from 0 to 2.

In some further preferred embodiments of the present invention, the compound is further shown as general formula (I'): 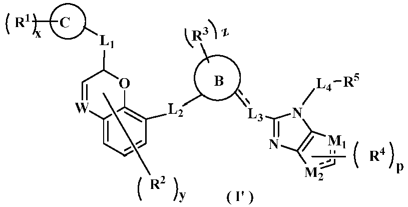 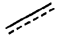 is a single bond or a double bond;
ring B is selected from the group consisting of cycloalkyl, heterocyclyl, aryl and heteroaryl, wherein the cycloalkyl, heterocyclyl, aryl, and heteroaryl can be each optionally further substituted;
or, ring B is absent;
ring C is selected from aryl or heteroaryl, preferably phenyl or pyridinyl ;
L₁ is selected from the group consisting of a bond, alkenylene, alkynylene, - (CH₂)ₙ₁-, -(CH₂)ₙ₁(CRₐₐR_{bb})ₙ₂-, -(CRₐₐR_{bb})ₙ₁O(CH₂)ₙ₂-, -(CH₂)ₙ₁O(CRₐₐR_{bb})ₙ₂-, - (CRₐₐR_{bb})ₙ₁S(CH₂)ₙ₂-, -(CH₂)ₙ₁S(CRₐₐR_{bb})ₙ₂-, -(CRₐₐR_{bb})ₙ₁(CH₂)ₙ₂NR_{cc}-, - (CH₂)ₙ₁NRₐₐ(CR_{bb}R_{cc})ₙ₂-, -(CH₂)ₙ₁C(O)(CRₐₐR_{bb})ₙ₂-, -(CH₂)ₙ₁NRₐₐC(O)(CRₐₐR_{bb})ₙ₂-, - (CH₂)ₙ₁P(O)Rₐₐ-, -(CH₂)ₙ₁S(O)ₘ₁-, -(CH₂)ₙ₁S(O)ₘ₁NRₐₐ-, -(CH₂)ₙ₁NRₐₐS(O)ₘ₁- and - (CH₂)ₙ₁C(O)NRₐₐ-;
Rₐₐ, R_{bb}, and R_{cc} are each independently selected from the group consisting of hydrogen, deuterium, halogen, amino, nitro, hydroxy, cyano, carboxy, alkyl, deuterated alkyl, haloalkyl, hydroxyalkyl, alkoxy, haloalkoxy, deuterated alkoxy, alkenyl, alkynyl, heterocyclylalkyl, cycloalkyl, heterocyclyl, aryl and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, any two of Rₐₐ, R_{bb}, and R_{cc} are bonded to form a cycloalkyl, heterocyclyl, aryl or heteroaryl, wherein the cycloalkyl, heterocyclyl, aryl and heteroaryl can be each optionally further substituted;
L₂ is selected from the group consisting of a bond, alkenylene, alkynylene, - (CH₂)ₙ₃-, -(CH₂)ₙ₃(CRₐ₁R_{b1})ₙ₄-, -(CRₐ₁R_{b1})ₙ₃O(CH₂)ₙ₄-, -(CH₂)ₙ₃O(CRₐ₁R_{b1})ₙ₄-, - (CRₐ₁R_{b1})ₙ₃S(CH₂)ₙ₄-, -(CH₂)ₙ₃S(CR₁ₐR_{b1})ₙ₄-, -(CRₐ₁R_{b1})ₙ₃(CH₂)ₙ₄NR_{c1}-, - (CH₂)ₙ₃NRₐ₁(CR_{b1}R_{c1})ₙ₄-, -(CH₂)ₙ₃C(O)(CRₐ₁R_{b1})ₙ₄-, -(CH₂)ₙ₃NRₐ₁C(O)(CRₐ₁R_{b1})ₙ₄-, - (CH₂)ₙ₃P(O)Rₐ₁-, -(CH₂)ₙ₃S(O)ₘ₂-, -(CH₂)ₙ₃S(O)ₘ₂NRₐ₁-, -(CH₂)ₙ₃NRₐ₁S(O)ₘ₂- and - (CH₂)ₙ₃C(O)NRₐ₁-;
Rₐ₁, R_{b1} and R_{c1} are each independently selected from the group consisting of hydrogen, deuterium, halogen, amino, nitro, hydroxy, cyano, carboxy, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, Rₐ₁ is absent;
or, any two of Rₐ₁, R_{b1} and R_{c1} are bonded to form a cycloalkyl, heterocyclyl, aryl or heteroaryl, wherein the cycloalkyl, heterocyclyl, aryl and heteroaryl can be each optionally further substituted;
L₃ is selected from the group consisting of a bond, alkenylene, alkynylene, - (CH₂)ₙ₅-, -(CH₂)ₙ₅(CRₐ₂R_{b2})ₙ₆-, -(CRₐ₂R_{b2})ₙ₅O(CH₂)ₙ₆-, -(CH₂)ₙ₅O(CRₐ₂R_{b2})ₙ₆-, - (CRₐ₂R_{b2})ₙ₅S(CH₂)ₙ₆-, -(CH₂)ₙ₅S(CRₐ₂R_{b2})ₙ₆-, -(CRₐ₂R_{b2})ₙ₅(CH₂)ₙ₆NR_{c2}-, - (CH₂)ₙ₅NRₐ₂(CR_{b2}R_{c2})ₙ₆-, -(CH₂)ₙ₅C(O)(CRₐ₂R_{b2})ₙ₆-, -(CH₂)ₙ₅NRₐ₂C(O)(CRₐ₂R_{b2})ₙ₆-, - (CH₂)ₙ₅P(O)Rₐ₂-, -(CH)ₙ(CH₂)ₙ₅S(O)ₘ₃-, -(CH₂)ₙ₅S(O)ₘ₃NRₐ₂-, -(CH₂)ₙ₅NRₐ₂S(O)ₘ₃-and -(CH₂)ₙ₅C(O)NRₐ₂-;
Rₐ₂, R_{b2} and R_{c2} are each independently selected from the group consisting of hydrogen, deuterium, halogen, amino, nitro, hydroxy, cyano, oxo, thioxo, carboxy, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, Rₐ₂ is absent;
W is selected from CRₘ or N;
Rₘ is selected from the group consisting of hydrogen, deuterium, halogen, amino, nitro, hydroxy, cyano, oxo, thioxo, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
L₄ is selected from the group consisting of a bond, -(CH₂)ₙ₇-, -(CH₂)ₙ₇(CRₐ₃R_{b3})ₙ₈-, -(CRₐ₃R_{b3})ₙ₇O(CH₂)ₙ₈-, -(CH₂)ₙ₇O(CRₐ₃R_{b3})ₙ₈-, -(CRₐ₃R_{b3})ₙ₇S(CH₂)ₙ₈-, - (CH₂)ₙ₇S(CRₐ₃R_{b3})ₙ₈-, -(CRₐ₃R_{b3})ₙ₇(CH₂)ₙ₈NR_{c3}-, -(CH₂)ₙ₇NRₐ₃(CR_{b3}R_{c3})ₙ₈-, - (CH₂)ₙ₇C(O)(CRₐ₃R_{b3})ₙ₈-, -(CH₂)ₙ₇NRₐ₃C(O)(CRₐ₃R_{b3})ₙ₈-, -(CH₂)ₙ₇P(O)Rₐ₃-, - (CH₂)ₙ₇S(O)ₘ₄-, -(CH₂)ₙ₇S(O)ₘ₄NRₐ₃-, -(CH₂)ₙ₇NRₐ₃S(O)ₘ₄- and -(CH₂)ₙ₇C(O)NRₐ₃-;
Rₐ₃, R_{b3} and R_{c3} are each independently selected from the group consisting of hydrogen, deuterium, halogen, amino, nitro, hydroxy, cyano, oxo, thioxo, carboxy, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
or, any two of Rₐ₃, R_{b3} and R_{c3} are bonded to form a cycloalkyl, heterocyclyl, aryl or heteroaryl, optionally can be further substituted;
R¹ is each independently selected from the group consisting of hydrogen, deuterium, halogen, amino, nitro, hydroxy, cyano, alkyl, deuterated alkyl, haloalkyl, hydroxyalkyl, alkoxy, haloalkoxy, deuterated alkoxy, alkenyl, alkynyl, heterocyclylalkyl, cycloalkyl, heterocyclyl, aryl, heteroaryl, -(CH₂)ₙ₁C(O)Rₐₐ and - (CH₂)ₙ₁C(O)NRₐₐR_{bb}, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, any two of R¹ are bonded to form a cycloalkyl, heterocyclyl, aryl or heteroaryl, wherein the cycloalkyl, heterocyclyl, aryl and heteroaryl can be each optionally further substituted;
or, R¹ and L₁ are bonded to form a cycloalkyl, heterocyclyl, aryl or heteroaryl, wherein the cycloalkyl, heterocyclyl, aryl and heteroaryl can be each optionally further substituted;
R² is each independently selected from the group consisting of hydrogen, deuterium, halogen, amino, nitro, hydroxy, cyano, oxo, thioxo, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, any two of R² are bonded to form a cycloalkyl, heterocyclyl, aryl or heteroaryl, wherein the cycloalkyl, heterocyclyl, aryl and heteroaryl can be each optionally further substituted;
or, R² and L₁ are bonded to form a cycloalkyl, heterocyclyl, aryl or heteroaryl, wherein the cycloalkyl, heterocyclyl, aryl and heteroaryl can be each optionally further substituted;
or, R² and L₂ are bonded to form a cycloalkyl, heterocyclyl, aryl or heteroaryl, wherein the cycloalkyl, heterocyclyl, aryl and heteroaryl can be each optionally further substituted;
or, R² and R³ are bonded to form a cycloalkyl, heterocyclyl, aryl or heteroaryl, wherein the cycloalkyl, heterocyclyl, aryl and heteroaryl can be each optionally further substituted;
R³ is each independently selected from the group consisting of hydrogen, deuterium, halogen, amino, nitro, hydroxy, cyano, thiol, oxo, thioxo, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, any two of R³ are bonded to form a cycloalkyl, heterocyclyl, aryl or heteroaryl, wherein the cycloalkyl, heterocyclyl, aryl and heteroaryl can be each optionally further substituted;
or, R³ and L₂ are bonded to form a cycloalkyl, heterocyclyl, aryl or heteroaryl, wherein the cycloalkyl, heterocyclyl, aryl and heteroaryl can be each optionally further substituted;
or, R³ and L₃ are bonded to form a cycloalkyl, heterocyclyl, aryl or heteroaryl, wherein the cycloalkyl, heterocyclyl, aryl and heteroaryl can be each optionally further substituted;
R⁴ is each independently selected from the group consisting of hydrogen, deuterium, halogen, oxo, amino, nitro, hydroxy, carboxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
R⁵ is selected from the group consisting of hydrogen, deuterium, halogen, amino, nitro, hydroxy, cyano, oxo, thioxo, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
M₁ is selected from the group consisting of O, S, N, C, (CH)ₙ₉, (CH₂)ₙ₉O, (CH₂)ₙ₉S, (CH₂)ₙ₉NH and (CH₂)ₙ₉;
M₂ is selected from the group consisting of O, S, N, C, (CH)ₙ₁₀, (CH₂)ₙ₁₀O, (CH₂)ₙ₁₀S, (CH₂)ₙ₁₀NH and (CH₂)ₙ₁₀;
x is an integer from 0 to 12;
y is an integer from 0 to 12;
z is an integer from 0 to 12;
p is an integer from 0 to 4;
n1~n6 are each integer from 0 to 3; and
n7~n10 are each integer from 0 to 3;
m1~m3 are each integer from 0 to 2;
m4 is an integer from 0 to 2.

In some further preferred embodiments of the present invention, the compound is further shown as general formula (I): 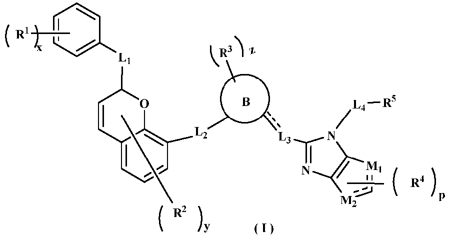
R¹, R², R³, L₁, L₂, L₃, x, y, z, and ring B are as defined above;
L₄ is selected from the group consisting of a bond, -(CH₂)ₙ₇-, -(CH₂)ₙ₇(CRₐ₃R_{b3})ₙ₈-, -(CRₐ₃R_{b3})ₙ₇O(CH₂)ₙ₈-, -(CH₂)ₙ₇O(CRₐ₃R_{b3})ₙ₈-, -(CRₐ₃R_{b3})ₙ₇S(CH₂)ₙ₈-, - (CH₂)ₙ₇S(CRₐ₃R_{b3})ₙ₈-, -(CRₐ₃R_{b3})ₙ₇(CH₂)ₙ₈NR_{c3}-, -(CH₂)ₙ₇NRₐ₃(CR_{b3}R_{c3})ₙ₈-, - (CH₂)ₙ₇C(O)(CRₐ₃R_{b3})ₙ₈-, -(CH₂)ₙ₇NRₐ₃C(O)(CRₐ₃R_{b3})ₙ₈-, -(CH₂)ₙ₇P(O)Rₐ₃-, - (CH₂)ₙ₇S(O)ₘ₄-, -(CH₂)ₙ₇S(O)ₘ₄NRₐ₃-, -(CH₂)ₙ₇NRₐ₃S(O)ₘ₄- and -(CH₂)ₙ₇C(O)NRₐ₃-;
Rₐ₃, R_{b3} and R_{c3} are each independently selected from the group consisting of hydrogen, deuterium, halogen, amino, nitro, hydroxy, cyano, oxo, thioxo, carboxy, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
or, any two of Rₐ₃, R_{b3} and R_{c3} are bonded to form a cycloalkyl, heterocyclyl, aryl or heteroaryl, optionally can be further substituted;
R⁴ is each independently selected from the group consisting of hydrogen, deuterium, halogen, amino, nitro, hydroxy, carboxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
R⁵ is selected from the group consisting of hydrogen, deuterium, halogen, amino, nitro, hydroxy, cyano, oxo, thioxo, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
M₁ is selected from the group consisting of O, S, N, C, (CH)ₙ₉, (CH₂)ₙ₉O, (CH₂)ₙ₉S, (CH₂)ₙ₉NH and (CH₂)ₙ₉;
M₂ is selected from the group consisting of O, S, N, C, (CH)ₙ₁₀, (CH₂)ₙ₁₀O, (CH₂)ₙ₁₀S, (CH₂)ₙ₁₀NH and (CH₂)ₙ₁₀;
p is an integer from 0 to 4;
n7~n10 are each integer from 0 to 3;
m4 is an integer from 0 to 2.

In some further preferred embodiments of the present invention, in the compounds of the aforementioned general formulas (I-A), (I'), (I"), and (I):
ring B is selected from the group consisting of C₃₋₁₂ cycloalkyl, 3 to 12 membered heterocyclyl, C₆₋₁₄ aryl and 5 to 14 membered heteroaryl;
preferably C₃₋₈ cycloalkyl, 4 to 12 membered heterocyclyl, C₆₋₁₀ aryl and 5 to 10 membered heteroaryl;
more preferably C₅₋₈ cycloalkyl, 5 to 10 membered heterocyclyl comprising 1 to 3 nitrogen atoms, oxygen atoms, or sulfur atoms, C₆₋₈ aryl and 5 to 10 membered heteroaryl comprising 1 to 3 nitrogen atoms, oxygen atoms, or sulfur atoms; further preferably 5 to 10 membered heterocyclyl comprising nitrogen atoms; the most preferably piperidinyl and piperazinyl.

In some further preferred embodiments of the present invention, in the compounds of the aforementioned general formulas (I-A), (I'), (I"), and (I):
ring B is selected from the group consisting of C₃₋₁₂ cycloalkyl, 3 to 12 membered heterocyclyl, C₆₋₁₄ aryl and 5 to 14 membered heteroaryl;
preferably C₃₋₈ cycloalkyl, 4 to 12 membered heterocyclyl, C₆₋₁₀ aryl and 5 to 10 membered heteroaryl;
more preferably C₅₋₈ cycloalkyl, 5 to 10 membered heterocyclyl comprising 1 to 3 nitrogen atoms, oxygen atoms, or sulfur atoms, C₆₋₈ aryl and 5 to 10 membered heteroaryl comprising 1 to 3 nitrogen atoms, oxygen atoms, or sulfur atoms; further preferably 5 to 10 membered heterocyclyl comprising nitrogen atoms; the most preferably piperidinyl.

In some further preferred embodiments of the present invention, the compound is further shown as general formula (II') and (IV): 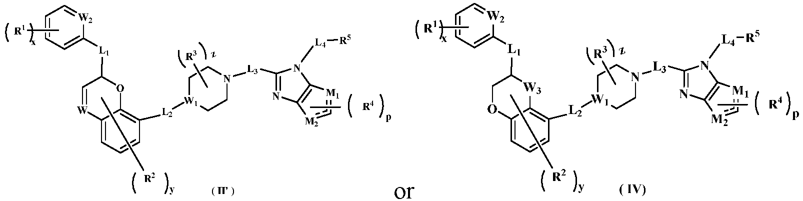
W is selected from CRₘ or N;
W₁ is selected from CH or N;
W₂ is selected from CRₘ₅ or N;
W₃ is selected from CRₘ₁Rₘ₂ or NRₘ₃;
R¹ is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, C₁₋₆ deuterated alkoxy, and C₁₋₆ haloalkoxy, preferably hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy, more preferably hydrogen, deuterium, fluorine, chlorine, cyano, -OCD₃ and methoxy;
R² is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, and C₁₋₆ haloalkoxy, preferably hydrogen, deuterium, fluorine, chlorine, cyano, C₁₋₃ alkyl, C₁₋₃ deuterated alkyl, C₁₋₃ haloalkyl, C₁₋₃ alkoxy, and C₁₋₃ haloalkoxy, more preferably hydrogen, deuterium, fluorine, chlorine, and methyl;
R³ is each independently selected from the group consisting of hydrogen, deuterium, fluorine, methyl, deuterated methyl, and halomethyl, preferably hydrogen and methyl;
Rₘ₅ is selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, C₁₋₆ deuterated alkoxy, and C₁₋₆ haloalkoxy, preferably hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy, more preferably hydrogen, and deuterium;
R⁴ is each independently selected from the group consisting of hydrogen, deuterium, fluorine, chlorine, cyano, carboxy, C₁₋₆ alkyl, C₁₋₆ deuterated alkyl, and C₁₋₆ haloalkyl, preferably carboxy;
R⁵ is selected from the group consisting of C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3 to 8 membered heterocyclyl, C₆₋₁₀ aryl and 5 to 10 membered heteroaryl, optionally further substituted by one or more substituents selected from halogen, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl and 3 to 8 membered heterocyclyl, preferably C₁₋₆ alkyl, C₁₋₆ deuterated alkyl, C₁₋₆ haloalkyl, C₃₋₈ cycloalkyl and 3 to 8 membered heterocyclyl, optionally further substituted by one or more substituents selected from halogen, C₁₋₆ alkyl, C₁₋₆ deuterated alkyl, C₁₋₆ haloalkyl, C₃₋₈ cycloalkyl and 3 to 8 membered heterocyclyl, more preferably 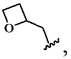 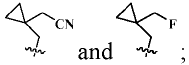
L₁ and L₂ are each independently selected from a bond or -(CH₂)ₙ₁-, preferably bond;
L₃ is selected from a bond or -(CH₂)ₙ₁-, preferably -CH₂-;
L₄ is selected from a bond or -(CH₂)ₙ₇-, preferably bond or -CH₂-;
M₁ is selected from the group consisting of N, C and (CH)ₙ₉;
M₂ is selected from the group consisting of N, C and (CH)ₙ₁₀;
n9 or n10 is each independently integer from 0 to 2;
x, y, and z are as defined above.

In some further preferred embodiments of the present invention, the compound is further shown as general formula (II') and (IV): 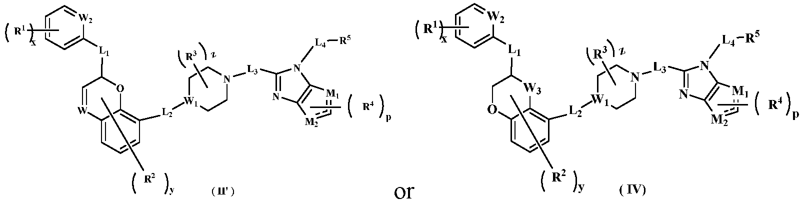
W₁ is selected from CH or N;
W₂ is selected from CRₘ₅ or N;
R¹ is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, C₁₋₆ deuterated alkoxy, and C₁₋₆ haloalkoxy;
R² is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, and C₁₋₆ haloalkoxy;
R³ is each independently selected from the group consisting of hydrogen, deuterium, fluorine, methyl, deuterated methyl, and halomethyl;
Rₘ₅ is selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, C₁₋₆ deuterated alkoxy, and C₁₋₆ haloalkoxy, preferably hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy, more preferably hydrogen, and deuterium;
R⁴ is each independently selected from the group consisting of hydrogen, deuterium, fluorine, chlorine, cyano, oxo, C₁₋₆ carboxy, C₁₋₆ alkyl, C₁₋₆ deuterated alkyl, C₁₋₆ haloalkyl, and 5 to 6 membered heteroaryl comprising 1 to 4 nitrogen atoms, oxygen atoms, or sulfur atoms, wherein the C₁₋₆ carboxy or 5 to 6 membered heteroaryl comprising 1 to 4 nitrogen atoms, oxygen atoms or sulfur atoms is optionally further substituted by one or more substituents selected from the group consisting of oxo, halogen cyano, C₁₋₆ deuterated alkyl, C₁₋₆ haloalkyl, C₁₋₆ alkoxy, C₁₋₆ deuterated alkoxy, and C₁₋₆ haloalkoxy, preferably carboxy and -C(O)OCH₃;
R⁵ is selected from the group consisting of C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ cyanoalkyl, C₃₋₈ cycloalkyl, and 3 to 8 membered heterocyclyl, preferably C₁₋₆ alkyl, C₁₋₆ deuterated alkyl, C₁₋₆ haloalkyl, C₃₋₈ cycloalkyl, and 3 to 8 membered heterocyclyl, optionally further substituted by one or more substituents selected from the group consisting of halogen, C₁₋₆ alkyl, C₁₋₆ deuterated alkyl, C₁₋₆ haloalkyl, C₁₋₆ cyanoalkyl, C₃₋₈ cycloalkyl, and 3 to 8 membered heterocyclyl;
L₁ and L₂ are each independently selected from a bond or -(CH₂)ₙ₁-, preferably bond;
L₃ is selected from a bond or -(CH₂)ₙ₁-, preferably -CH₂-;
L₄ is selected from a bond or -(CH₂)ₙ₇-, preferably bond or -CH₂-;
M₁ is selected from the group consisting of N, NH, O, S, C and (CH)ₙ₉;
M₂ is selected from the group consisting of N, NH, O, S, C and (CH)ₙ₁₀;
n9 or n10 is each independently integer from 0 to 2;
x, y, and z are as defined above.

In some further preferred embodiments of the present invention, the compound is further shown as general formula (II'): 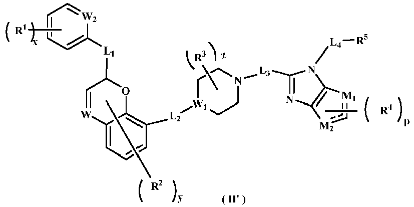
W₁ is selected from CH or N;
W₂ is selected from CRₘ₅ or N;
R¹ is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, -C(O)Rₐₐ and -C(O)NRₐₐR_{bb};
Rₐₐ, and R_{bb} are each independently selected from the group consisting of hydrogen, deuterium, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3 to 8 membered heterocyclyl, C₆₋₁₀ aryl and 5 to 10 membered heteroaryl;
R² is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, and C₁₋₆ haloalkoxy;
R³ is each independently selected from the group consisting of hydrogen, deuterium, fluorine, methyl, deuterated methyl, and halomethyl;
Rₘ₅ is selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, C₁₋₆ deuterated alkoxy, and C₁₋₆ haloalkoxy, preferably hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy, more preferably hydrogen, and deuterium;
R⁴ is each independently selected from the group consisting of hydrogen, deuterium, fluorine, chlorine, cyano, oxo, C₁₋₆ carboxy, C₁₋₆ alkyl, C₁₋₆ deuterated alkyl, C₁₋₆ haloalkyl, and substituted or unsubstituted 5 to 6 membered heteroaryl, wherein the C₁₋₆ carboxy is optionally further substituted by one or more substituents selected from halogen or C₁₋₆ alkyl, preferably carboxy and -C(O)OCH₃;
R⁵ is selected from the group consisting of C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ cyanoalkyl, C₃₋₈ cycloalkyl, and 3 to 8 membered heterocyclyl, preferably C₁₋₆ alkyl, C₁₋₆ deuterated alkyl, C₁₋₆ haloalkyl, C₃₋₈ cycloalkyl, and 3 to 8 membered heterocyclyl, optionally further substituted by one or more substituents selected from the group consisting of halogen, C₁₋₆ alkyl, C₁₋₆ deuterated alkyl, C₁₋₆ haloalkyl, C₁₋₆ cyanoalkyl, C₃₋₈ cycloalkyl, and 3 to 8 membered heterocyclyl;
L₁ and L₂ are each independently selected from a bond or -(CH₂)ₙ₁-, preferably bond;
L₃ is selected from a bond or -(CH₂)ₙ₁-, preferably -CH₂-;
L₄ is selected from a bond or -(CH₂)ₙ₇-, preferably bond or -CH₂-;
M₁ is selected from the group consisting of N, NH, O, S, C and (CH)ₙ₉;
M₂ is selected from the group consisting of N, NH, O, S, C and (CH)ₙ₁₀;
n9 or n10 is each independently integer from 0 to 2.

In some further preferred embodiments of the present invention, Rₐₐ, and R_{bb} are each independently selected from the group consisting of hydrogen, deuterium, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl, 3 to 6 membered heterocyclyl, C₆₋₁₀ aryl and 5 to 6 membered heteroaryl.

In some further preferred embodiments of the present invention, the compound is further shown in the following general formula:

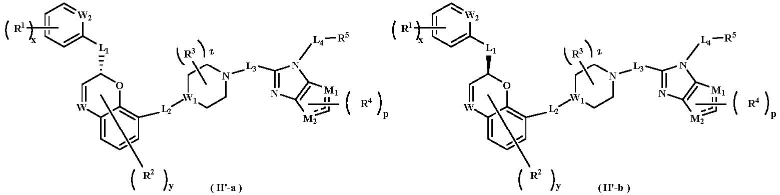

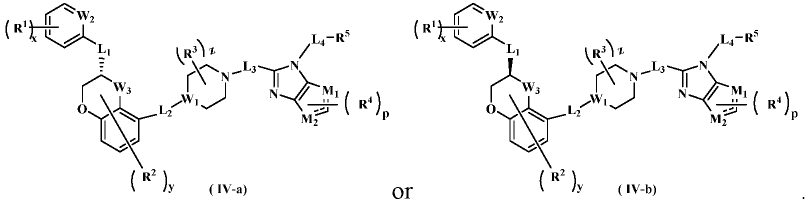

In some further preferred embodiments of the present invention, the compound is further shown as general formula (II): 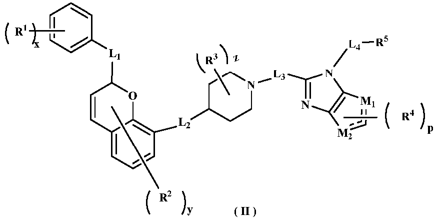
R¹ is each independently selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ deuterated alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
or, any two of R¹ are bonded to form a C₃₋₈ cycloalkyl, 3 to 8 membered heterocyclyl, C₆₋₁₀ aryl and 5 to 10 membered heteroaryl, wherein the C₃₋₈ cycloalkyl, 3 to 8 membered heterocyclyl, C₆₋₁₀ aryl and 5 to 10 membered heteroaryl are each optionally further substituted by one or more substituents selected from the group consisting of halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
or, R² is each independently selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
or, any two of R² are bonded to form a C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
or, R³ is each independently selected from the group consisting of hydrogen, deuterium, halogen, amino, nitro, hydroxy, cyano, carboxy, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, carboxy, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
or, R⁴ is each independently selected from the group consisting of hydrogen, deuterium, halogen, amino, nitro, hydroxy, cyano, carboxy, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, carboxy, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
or, any two of R⁴ are bonded to form a C₃₋₈ cycloalkyl, 3 to 8 membered heterocyclyl, C₆₋₁₀ aryl and 5 to 10 membered heteroaryl, wherein the C₃₋₈ cycloalkyl, 3 to 8 membered heterocyclyl, C₆₋₁₀ aryl and 5 to 10 membered heteroaryl are each optionally further substituted by one or more substituents selected from the group consisting of halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
or, R⁵ is each independently selected from the group consisting of hydrogen, deuterium, halogen, amino, nitro, hydroxy, cyano, carboxy, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, carboxy, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
or, M₁ is selected from the group consisting of O, S, N, C, CH and (CH)₂;
or, M₂ is selected from the group consisting of O, S, N, C, CH and (CH)₂;
or, x is 0, 1, 2, 3 ,4 or 5;
or, y is 0, 1, 2, 3 or 4;
or, z is 0, 1, 2, 3 or 4;
or, p is 0, 1, 2, or 3.

In some further preferred embodiments of the present invention, the compound is further shown as general formula (II): 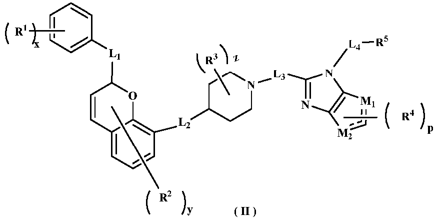
R¹ is each independently selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ deuterated alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
or, any two of R¹ are bonded to form a C₃₋₈ cycloalkyl, 3 to 8 membered heterocyclyl, C₆₋₁₀ aryl and 5 to 10 membered heteroaryl, wherein the C₃₋₈ cycloalkyl, 3 to 8 membered heterocyclyl, C₆₋₁₀ aryl and 5 to 10 membered heteroaryl are each optionally further substituted by one or more substituents selected from the group consisting of halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
R² is each independently selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
or, any two of R² are bonded to form a C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
R³ is each independently selected from the group consisting of hydrogen, deuterium, halogen, amino, nitro, hydroxy, cyano, carboxy, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, carboxy, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
R⁴ is each independently selected from the group consisting of hydrogen, deuterium, halogen, amino, nitro, hydroxy, cyano, carboxy, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, carboxy, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
or, any two of R⁴ are bonded to form a C₃₋₈ cycloalkyl, 3 to 8 membered heterocyclyl, C₆₋₁₀ aryl and 5 to 10 membered heteroaryl, wherein the C₃₋₈ cycloalkyl, 3 to 8 membered heterocyclyl, C₆₋₁₀ aryl and 5 to 10 membered heteroaryl are each optionally further substituted by one or more substituents selected from the group consisting of halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
R⁵ is selected from the group consisting of hydrogen, deuterium, halogen, amino, nitro, hydroxy, cyano, carboxy, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, carboxy, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ cyanoalkyl, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
M₁ is selected from the group consisting of O, S, N, NH, C, CH and (CH)₂;
M₂ is selected from the group consisting of O, S, N, NH, C, CH and (CH)₂;
x is 0, 1, 2, 3 ,4 or 5;
y is 0, 1, 2, 3 or 4;
z is 0, 1, 2, 3 or 4;
p is 0, 1, 2, or 3.

In some further preferred embodiments of the present invention, in the aforementioned general formulas, R¹ is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, C₁₋₆ deuterated alkoxy, and C₁₋₆ haloalkoxy, preferably hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy, more preferably hydrogen, deuterium, fluorine, chlorine, cyano, -OCD₃, and methoxy.

In some further preferred embodiments of the present invention, in the aforementioned general formulas, R² is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, C₁₋₆ deuterated alkoxy, and C₁₋₆ haloalkoxy, preferably hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy, more preferably hydrogen, deuterium, fluorine, chlorine, and methyl.

In some further preferred embodiments of the present invention, in the aforementioned general formulas, R³ is each independently selected from the group consisting of hydrogen, deuterium, fluorine, methyl, deuterated methyl, and halomethyl, preferably hydrogen and methyl.

In some further preferred embodiments of the present invention, in the aforementioned general formulas, R⁴ is each independently selected from the group consisting of hydrogen, deuterium, fluorine, chlorine, cyano, carboxy, C₁₋₆ alkyl, C₁₋₆ deuterated alkyl, and C₁₋₆ haloalkyl, preferably carboxy.

In some further preferred embodiments of the present invention, in the aforementioned general formulas, R⁴ is selected from the group consisting of hydrogen, deuterium, fluorine, chlorine, cyano, oxo, C₁₋₆ carboxy, C₁₋₆ alkyl, C₁₋₆ deuterated alkyl, C₁₋₆ haloalkyl and 5 to 6 membered heteroaryl comprising 1 to 4 nitrogen atoms, oxygen atoms, or sulfur atoms, wherein the C₁₋₆ carboxy and 5 to 6 membered heteroaryl comprising 1 to 4 nitrogen atoms, oxygen atoms are each optionally further substituted by one or more substituents selected from the group consisting of oxo, halogen, cyano, C₁₋₃ deuterated alkyl, C₁₋₃ haloalkyl, C₁₋₃ alkoxy, C₁₋₃ deuterated alkoxy, and C₁₋₃ haloalkoxy, preferably carboxy and -C(O)OCH₃.

In some further preferred embodiments of the present invention, in the aforementioned general formulas, R⁵ is selected from the group consisting of C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3 to 8 membered heterocyclyl, C₆₋₁₀ aryl and 5 to 10 membered heteroaryl, optionally further substituted by one or more substituents selected from halogen, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ cyanoalkyl, C₃₋₈ cycloalkyl, and 3 to 8 membered heterocyclyl, preferably C₁₋₃ alkyl, C₁₋₃ deuterated alkyl, C₁₋₃ haloalkyl, C₃₋₆ cycloalkyl, and 3 to 6 membered heterocyclyl, optionally further substituted by one or more substituents selected from the group consisting of halogen, C₁₋₃ alkyl, C₁₋₃ deuterated alkyl, C₁₋₃ haloalkyl, C₁₋₃ cyanoalkyl, C₃₋₆ cycloalkyl, and 3 to 6 membered heterocyclyl, more preferably C₃₋₆ cycloalkyl, and 3 to 6 membered heterocyclyl comprising 1 to 2 nitrogen atoms, oxygen atoms, or sulfur atoms, optionally further substituted by one or more substituents selected from the group consisting of halogen, C₁₋₃ alkyl, C₁₋₃ deuterated alkyl, C₁₋₃ cyanoalkyl, and C₁₋₃ haloalkyl, further preferably

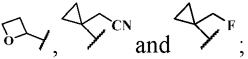

In some further preferred embodiments of the present invention, in the aforementioned general formulas, R⁵ is selected from the group consisting of C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, and 3 to 8 membered heterocyclyl, preferably C₁₋₃ alkyl, C₁₋₃ deuterated alkyl, C₁₋₃ haloalkyl, C₃₋₆ cycloalkyl, and 3 to 6 membered heterocyclyl, optionally further substituted by one or more substituents selected from the group consisting of halogen, C₁₋₃ alkyl, C₁₋₃ deuterated alkyl, C₁₋₃ haloalkyl, C₃₋₆ cycloalkyl, and 3 to 6 membered heterocyclyl, more preferably

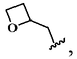

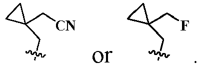

In some further preferred embodiments of the present invention, in the aforementioned general formulas, M₁ is selected from the group consisting of N, C, and (CH)ₙ₉.

In some further preferred embodiments of the present invention, in the aforementioned general formulas, M₂ is selected from the group consisting of N, C, and (CH)ₙ₁₀.

In some further preferred embodiments of the present invention, in the aforementioned general formulas, n9 or n10 is each independently integer from 0 to 2.

In some further preferred embodiments of the present invention, in the aforementioned general formulas, L₁ and L₂ are each independently selected from a bond or -(CH₂)ₙ₁-, preferably bond.

L₃ is selected from a bond or -(CH₂)ₙ₁-, preferably -CH₂-.

L₄ is selected from a bond or -(CH₂)ₙ₇-, preferably bond or -CH₂-.

In some further preferred embodiments of the present invention, in the aforementioned general formulas, R¹ is each independently selected from the group consisting of hydrogen, deuterium, fluorine, chlorine, cyano, methoxy and -OCD₃;
R² is each independently selected from the group consisting of hydrogen, deuterium, fluorine, and methyl;
R³ is each independently selected from hydrogen or methyl;
R⁴ is each independently selected from hydrogen or carboxy;
R⁵ is selected from the group consisting of 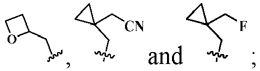
R⁵ is selected from the group consisting of 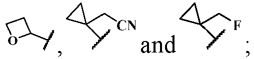
L₁ and L₂ are each independently selected from a bond; L₃ is-CH₂-; L₄ is bond.

In some further preferred embodiments of the present invention, R¹ is each independently selected from the group consisting of hydrogen, deuterium, fluorine, chlorine, cyano, and methoxy;
R² is each independently selected from the group consisting of hydrogen, deuterium, fluorine, and methyl;
R³ is each independently hydrogen;
R⁴ is each independently selected from hydrogen or carboxy;
R⁵ is selected from 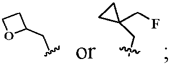
R⁵ is selected from 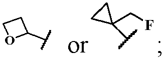
L₁ and L₂ are each independently a bond; L₃ is-CH₂-; L₄ is bond.

In some further preferred embodiments of the present invention, the compound is further shown as general formula (III)~(III-3): 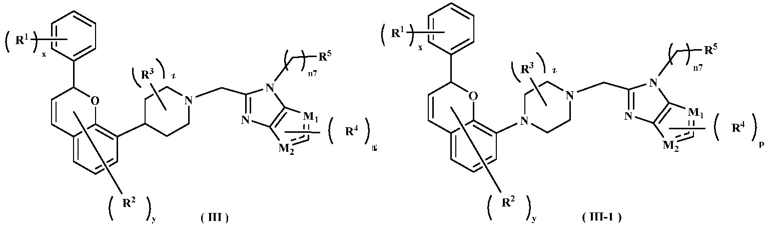 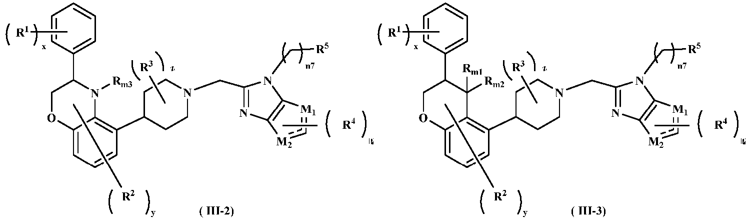
R¹ is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, C₁₋₆ deuterated alkoxy, and C₁₋₆ haloalkoxy;
R² is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, and C₁₋₆ haloalkoxy;
R³ is each independently selected from the group consisting of hydrogen, deuterium, fluorine, methyl, deuterated methyl, and halomethyl;
R⁴ is each independently selected from the group consisting of hydrogen, deuterium, fluorine, chlorine, cyano, carboxy, C₁₋₆ alkyl, C₁₋₆ deuterated alkyl, and C₁₋₆ haloalkyl;
R⁵ is selected from C₃₋₈ cycloalkyl, or 3 to 8 membered heterocyclyl, optionally further substituted by one or more substituents selected from the group consisting of halogen, C₁₋₆ alkyl, C₁₋₆ deuterated alkyl and C₁₋₆ haloalkyl;
M₁ is selected from the group consisting of O, S, N, C, CH and (CH)₂;
M₂ is selected from the group consisting of O, S, N, C, CH and (CH)₂;
n7 is 0, 1, 2, or 3;
x is 0, 1, 2, 3 ,4 or 5;
y is 0, 1, 2, 3 or 4;
z is 0, 1, 2, 3 or 4;
p is 0, 1, 2, or 3.

In a further preferred embodiment of the present invention, the compound is further shown as general formula (III)~(III-7):
wherein:
R¹ is each independently selected from the group consisting of hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy;
R² is each independently selected from the group consisting of hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy;
R³ is each independently selected from the group consisting of hydrogen, deuterium, and fluorine;
R⁴ is each independently selected from 5 to 6 membered heteroaryl comprising 1 to 4 nitrogen atoms, oxygen atoms, or sulfur atoms, optionally further substituted by one or more substituents selected from the group consisting of oxo, halogen, cyano, C₁₋₃ alkyl, and C₁₋₃ haloalkyl, -(CH₂)ₙCOOH and -(CH₂)ₙC(O)OCH₃;
R⁵ is selected from C₃₋₆ cycloalkyl or 3 to 6 membered heterocyclyl comprising 1 to 2 nitrogen atoms, oxygen atoms, or sulfur atoms, optionally further substituted by one or more substituents selected from the group consisting of halogen, C₁₋₃ alkyl, C₁₋₃ deuterated alkyl, C₁₋₃ cyanoalkyl, and C₁₋₃ haloalkyl;
M₁ and M₂ are each independently selected from the group consisting of N, CH and (CH)₂;
n is 0, 1, 2, or 3.

In some further preferred embodiments of the present invention, the compound is further shown as general formula (III): 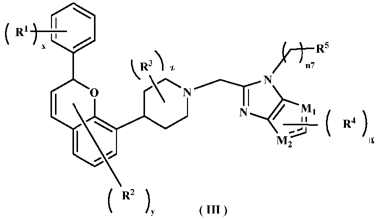
R¹ is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, C₁₋₆ deuterated alkoxy, and C₁₋₆ haloalkoxy;
R² is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, and C₁₋₆ haloalkoxy;
R³ is each independently selected from the group consisting of hydrogen, deuterium, and fluorine;
R⁴ is each independently selected from the group consisting of hydrogen, deuterium, fluorine, chlorine, cyano, carboxy, C₁₋₆ alkyl, C₁₋₆ deuterated alkyl, and C₁₋₆ haloalkyl;
R⁵ is selected from C₃₋₈ cycloalkyl, or 3 to 8 membered heterocyclyl, optionally further substituted by one or more substituents selected from the group consisting of halogen, C₁₋₆ alkyl, C₁₋₆ deuterated alkyl and C₁₋₆ haloalkyl;
M₁ is selected from the group consisting of O, S, N, C, CH and (CH)₂;
M₂ is selected from the group consisting of O, S, N, C, and CH;
n7 is 0, 1, 2, or 3;
x is 0, 1, 2, 3 ,4 or 5;
y is 0, 1, 2, 3 or 4;
z is 0, 1, 2, 3 or 4;
p is 0, 1, 2, or 3.

In some further preferred embodiments of the present invention, in compounds of general formulas (III)~(III-7), the stereoisomer thereof or the pharmaceutically acceptable salt thereof, wherein:
In some embodiments, R¹ is each independently selected from the group consisting of hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy, preferably hydrogen, deuterium, fluorine, chlorine, cyano, methoxy and -OCD₃;
In some embodiments, R² is each independently selected from the group consisting of hydrogen, deuterium, fluorine, chlorine, cyano, C₁₋₃ alkyl, C₁₋₃ deuterated alkyl, C₁₋₃ haloalkyl, C₁₋₃ alkoxy, and C₁₋₃ haloalkoxy, preferably hydrogen, deuterium, fluorine and methyl;
In some embodiments, R³ is each independently selected from the group consisting of hydrogen, deuterium, fluorine and methyl, preferably hydrogen and methyl;
In some embodiments, R⁴ is each independently carboxy;
In some embodiments, R⁵ is each independently selected from the group consisting of 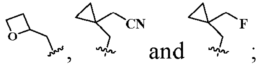
In some embodiments, x is 0; In some embodiments, x is 1; In some embodiments, x is 2; In some embodiments, x is 3; In some embodiments, x is 4; In some embodiments, x is 5;
In some embodiments, y is 0; In some embodiments, y is 1; In some embodiments, y is 2; In some embodiments, y is 3;
In some embodiments, z is 0; In some embodiments, z is 1; In some embodiments, z is 2; In some embodiments, z is 3;
In some embodiments, n7 is 0; In some embodiments, n7 is 1; In some embodiments, n7 is 2;
In some embodiments, p is 0; In some embodiments, p is 1; In some embodiments, p is 2; In some embodiments, p is 3;

In some further preferred embodiments of the present invention, in compounds of general formulas (III), the stereoisomer thereof or the pharmaceutically acceptable salt thereof, wherein:
R¹ is each independently selected from the group consisting of hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy;
R² is each independently selected from the group consisting of hydrogen, deuterium, fluorine, chlorine, cyano, C₁₋₃ alkyl, C₁₋₃ deuterated alkyl, C₁₋₃ haloalkyl, C₁₋₃ alkoxy, and C₁₋₃ haloalkoxy;
R³ is each independently selected from the group consisting of hydrogen, deuterium, and fluorine;
R⁴ is carboxy.

In a further preferred embodiment of the present invention, the compound is further shown as general formula (III'): 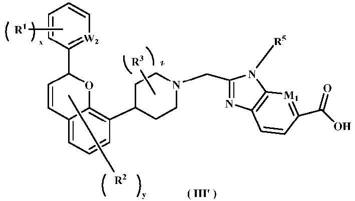
R¹ is each independently selected from the group consisting of hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy, preferably hydrogen, deuterium, fluorine, chlorine, methoxy and -OCD₃;
R² is each independently selected from the group consisting of hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy, preferably hydrogen, deuterium, fluorine, chlorine, and methyl;
R³ is each independently selected from the group consisting of hydrogen, deuterium, and fluorine, preferably hydrogen;
M₁ is selected from N or CH, preferably N;
W₂ is selected from N or CH, preferably CH;
x, y and z are each independently 0, 1, or 2;
R⁵ is selected from the group consisting of 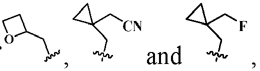 preferably

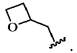

In a further preferred embodiment of the present invention, the compound is further shown as general formula (V-1) and (V-2): 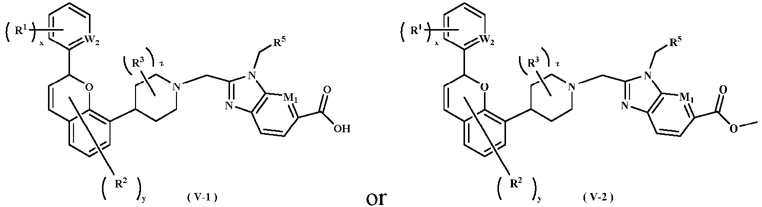
R¹ is each independently selected from the group consisting of hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy, preferably hydrogen, deuterium, fluorine, chlorine, methoxy and -OCD₃;
R² is each independently selected from the group consisting of hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy, preferably hydrogen, deuterium, fluorine, chlorine, and methyl;
R³ is each independently selected from the group consisting of hydrogen, deuterium, and fluorine, preferably hydrogen;
M₁ is selected from N or CH, preferably N;
W₂ is selected from N or CH, preferably CH;
x, y and z are each independently 0, 1, or 2;
R⁵ is selected from the group consisting of 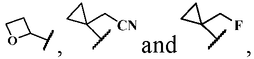 preferably 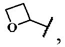 more preferably

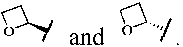

In a further preferred embodiment of the present invention, the compound is further shown as general formula (V-3) and (V-4):

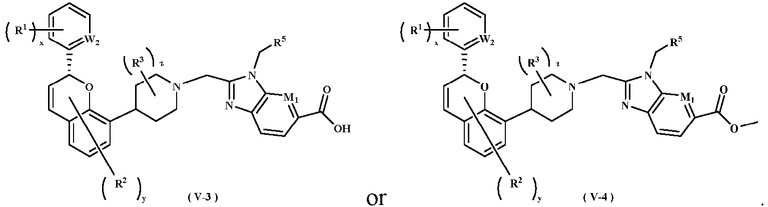

In a further preferred embodiment of the present invention, the compound is further shown as general formula (V-5) and (V-6):

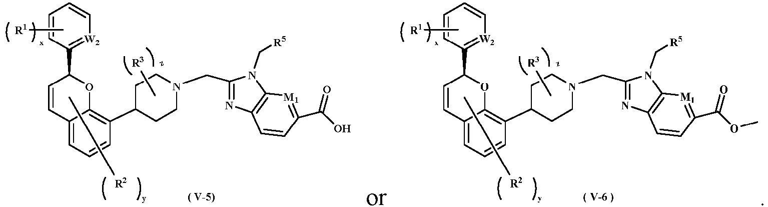

The present invention further relates to a pharmaceutical composition comprising a therapeutically effective dose of any one of the compound of the general formula, the stereoisomer thereof or the pharmaceutically acceptable salt thereof, and one or more pharmaceutically acceptable carriers, diluents or excipients.

The present invention further relates to a use of any one of the compound of the general formula, the stereoisomer thereof or the pharmaceutically acceptable salt thereof, or the pharmaceutical composition in the preparation of a GLP-1 receptor agonist medicament.

The present invention further relates to a use of any one of the compound of the general formula, the stereoisomer thereof or the pharmaceutically acceptable salt thereof, or the pharmaceutical composition comprising the same in the preparation of a medicament for treating metabolic related diseases, wherein the metabolic related diseases are selected from diabetes, obesity or nonalcoholic steatohepatitis related diseases or other related diseases caused by diabetes, obesity or nonalcoholic steatohepatitis.

The present invention further relates to a method for treating metabolic related diseases by the compound of the general formulas, the stereoisomer thereof or the pharmaceutically acceptable salt thereof or the pharmaceutical composition comprising the same.

The present invention also relates to a method for treating, preventing, and/or treating metabolic diseases and related diseases, comprising administering to a patient a therapeutic effective dose of the compound of the general formula, the stereoisomer thereof or the pharmaceutically acceptable salt thereof or the pharmaceutical composition comprising the same.

The present invention also provides a method for treating a disease condition by using the compound or pharmaceutical composition according to the present invention, wherein the disease condition includes, but is not limited to a condition related to a modulator of GLP-1 receptor.

The present invention also relates to a method for treating metabolic related diseases in a mammal, comprising administering a therapeutically effective amount of the compound or the pharmaceutically acceptable salt, ester, prodrug, solvate, hydrate or derivative thereof according to the present invention to the mammal.

### DEFINITIONS

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 8 carbon atoms, more preferably an alkyl having 1 to 6 carbon atoms, most preferably an alkyl having 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl group can be substituted or unsubstituted. When substituted, the substituent group(s) can be substituted at any available connection point. The substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl. The alkyl of the present invention is preferably selected from the group consisting of methyl, ethyl, isopropyl, tert-butyl, haloalkyl, deuterated alkyl, alkoxy-substituted alkyl and hydroxy-substituted alkyl.

The term "alkylene" refers to an alkyl with one hydrogen being further substituted, for example, "methylene" refers to -CH₂-, "ethylene" refers to -(CH₂)₂-, "propylene" refers to -(CH₂)₃-, "butylene" refers to -(CH₂)₄- and the like.

The term "alkenylene" refers to an alkenyl group with one hydrogen being further substituted, for example, "ethenylene" refers to -CH₂=CH₂-, "propenylene" refers to - CH₂-=CH₂-CH₂-, etc. The alkenylene group can be substituted or unsubstituted, and when substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, and heterocyclylthio.The term "cycloalkyl" refers to a saturated or partially unsaturated monocyclic or polycyclic hydrocarbon substituent group having 3 to 20 carbon atoms, preferably 3 to 12 carbon atoms, more preferably 3 to 8 carbon atoms, further preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes cycloalkyl having a spiro ring, fused ring or bridged ring. The cycloalkyl is preferably cyclopropyl, cyclobutyl, cyclohexyl, cyclopentyl and cycloheptyl.

The term "cycloalkyl" refers to a saturated or partially unsaturated monocyclic or polycyclic hydrocarbon substituent group having 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes cycloalkyl having a spiro ring, fused ring or bridged ring. The cycloalkyl is preferably cyclopropyl, cyclobutyl, cyclohexyl, cyclopentyl and cycloheptyl.

The term "spiro cycloalkyl" refers to a 5 to 20 membered polycyclic group with individual rings connected through one shared carbon atom (called a spiro atom), wherein the rings can contain one or more double bonds, but none of the rings has a completely conjugated π-electron system. The spiro cycloalkyl is preferably 6 to 14 membered spiro cycloalkyl, and more preferably 7 to 10 membered spiro cycloalkyl. According to the number of the spiro atoms shared between the rings, the spiro cycloalkyl can be divided into mono-spiro cycloalkyl, di-spiro cycloalkyl, or poly-spiro cycloalkyl, and the spiro cycloalkyl is preferably a mono-spiro cycloalkyl or di-spiro cycloalkyl, and more preferably 4-membered/4-membered, 4-membered/5-membered, 4-membered/6-membered, 5-membered/5-membered, or 5-membered/6-membered mono-spiro cycloalkyl. Non-limiting examples of spiro cycloalkyl include: 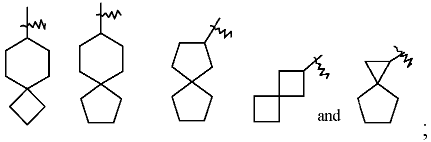 and also include spiro cycloalkyl in which a cycloalkyl and a heterocyclyl are connected through one spiro atom, non-limiting examples thereof include:

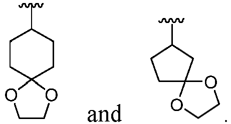

The term "fused cycloalkyl" refers to a 5 to 20 membered all-carbon polycyclic group, wherein each ring in the system shares an adjacent pair of carbon atoms with another ring, wherein one or more rings can contain one or more double bonds, but none of the rings has a completely conjugated π-electron system. The fused cycloalkyl is preferably 6 to 14 membered fused cycloalkyl, and more preferably 7 to 10 membered fused cycloalkyl. According to the number of membered rings, the fused cycloalkyl can be divided into bicyclic, tricyclic, tetracyclic or polycyclic fused cycloalkyl, and the fused cycloalkyl is preferably bicyclic or tricyclic fused cycloalkyl, and more preferably 5-membered/5-membered, or 5-membered/6-membered bicyclic fused cycloalkyl. Non-limiting examples of fused cycloalkyl include:

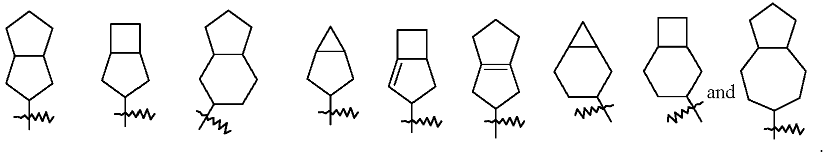

The term "bridged cycloalkyl" refers to a 5 to 20 membered all-carbon polycyclic group, wherein every two rings in the system share two disconnected carbon atoms, wherein the rings can have one or more double bonds, but none of the rings has a completely conjugated π-electron system. The bridged cycloalkyl is preferably 6 to 14 membered bridged cycloalkyl, and more preferably 7 to 10 membered bridged cycloalkyl. According to the number of membered rings, the bridged cycloalkyl can be divided into bicyclic, tricyclic, tetracyclic or polycyclic bridged cycloalkyl, and the bridged cycloalkyl is preferably bicyclic, tricyclic or tetracyclic bridged cycloalkyl, and more preferably bicyclic or tricyclic bridged cycloalkyl. Non-limiting examples of bridged cycloalkyl include:

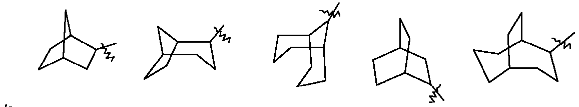

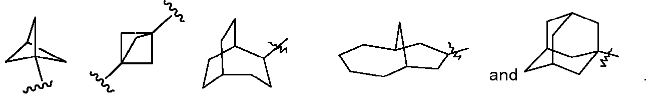

The cycloalkyl ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like. The cycloalkyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "heterocyclyl" refers to a 3 to 20 membered saturated or partially unsaturated monocyclic or polycyclic hydrocarbon group, wherein one or more ring atoms are heteroatoms selected from the group consisting of N, O and S(O)ₘ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; more preferably, 3 to 10 ring atoms; and further preferably 3 to 8 ring atoms; and more further preferably 3 to 6 ring atoms wherein 1 to 2 atoms are heteroatoms selected from the group consisting of N, O and S. Non-limiting examples of monocyclic heterocyclyl include pyrrolidinyl, azacyclobutanyl, oxocyclobutanyl, oxocyclohexyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomolinyl, homopiazinyl and pyranyl and the like, and preferably pyrrolidinyl, azacyclobutanyl, oxocyclobutanyl, tetrahydrofuranyl, pyrazolidinyl, morpholinyl, 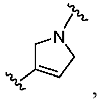 piperazinyl and pyranyl, and more preferably pyrrolidinyl, azacyclobutanyl, oxocyclobutanyl, piperidinyl, 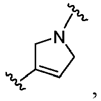 piperazinyl and pyranyl. Polycyclic heterocyclyl includes a heterocyclyl having a spiro ring, fused ring or bridged ring; wherein the involved heterocyclyl having a spiro ring, fused ring or bridged ring is optionally bonded to other group(s) through single bond, or further bonded to other cycloalkyl, heterocyclyl, aryl and heteroaryl through any two or more atoms in the ring.

The term "spiro heterocyclyl" refers to a 5 to 20 membered polycyclic heterocyclyl group with individual rings connected through one shared atom (called a spiro atom), wherein one or more ring atoms are heteroatoms selected from the group consisting of N, O and S(O)ₘ (wherein m is an integer of 0 to 2), with the remaining ring atoms being carbon atoms, wherein the rings can contain one or more double bonds, but none of the rings has a completely conjugated π-electron system. The spiro heterocyclyl is preferably 6 to 14 membered spiro heterocyclyl, and more preferably 7 to 10 membered spiro heterocyclyl. According to the number of the spiro atoms shared between the rings, the spiro heterocyclyl can be divided into mono-spiro heterocyclyl, di-spiro heterocyclyl, or poly-spiro heterocyclyl, and the spiro heterocyclyl is preferably mono-spiro heterocyclyl or di-spiro heterocyclyl, and more preferably 4-membered/4-membered, 4-membered/5-membered, 4-membered/6-membered, 5-membered/5-membered, or 5-membered/6-membered mono-spiro heterocyclyl. Non-limiting examples of spiro heterocyclyl include: 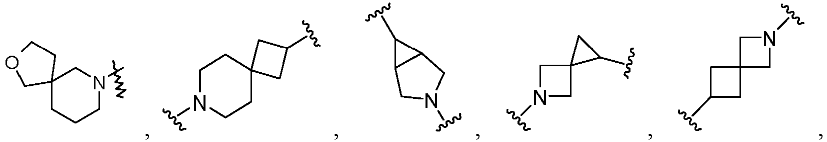 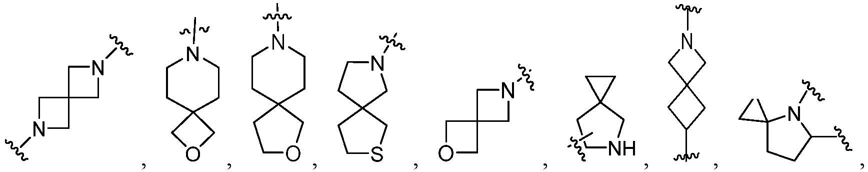 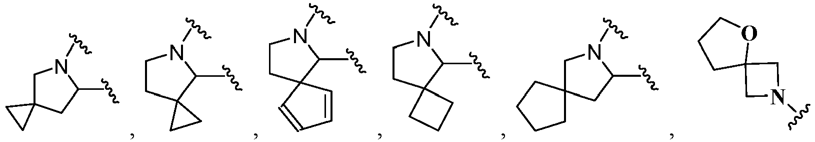 and

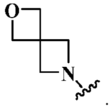

The term "fused heterocyclyl" refers to a 5 to 20 membered polycyclic heterocyclyl group, wherein each ring in the system shares an adjacent pair of atoms with another ring, wherein one or more rings can contain one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The fused heterocyclyl is preferably 6 to 14 membered fused heterocyclyl, and more preferably 7 to 10 membered fused heterocyclyl. According to the number of membered rings, the fused heterocyclyl can be divided into bicyclic, tricyclic, tetracyclic or polycyclic fused heterocyclyl, and the fused heterocyclyl is preferably bicyclic or tricyclic fused heterocyclyl, and more preferably 5-membered/5-membered or 5-membered/6-membered bicyclic fused heterocyclyl. Non-limiting examples of fused heterocyclyl include:

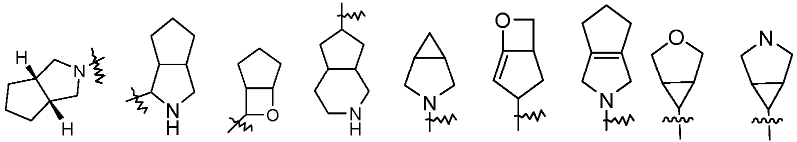

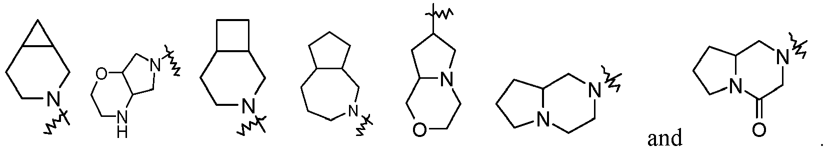

The term "bridged heterocyclyl" refers to a 5 to 14 membered polycyclic heterocyclyl group, wherein every two rings in the system share two disconnected atoms, wherein the rings can have one or more double bonds, but none of the rings has a completely conjugated π-electron system, and wherein one or more ring atoms are heteroatoms selected from the group consisting of N, O and S(O)m (wherein m is an integer of 0 to 2), with the remaining ring atoms being carbon atoms. The bridged heterocyclyl is preferably 6 to 14 membered bridged heterocyclyl, and more preferably 7 to 10 membered bridged heterocyclyl. According to the number of membered rings, the bridged heterocyclyl can be divided into bicyclic, tricyclic, tetracyclic or polycyclic bridged heterocyclyl, and the bridged heterocyclyl is preferably bicyclic, tricyclic or tetracyclic bridged heterocyclyl, and more preferably bicyclic or tricyclic bridged heterocyclyl. Non-limiting examples of bridged heterocyclyl include:

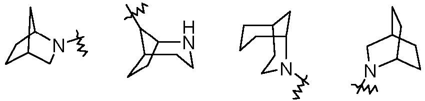

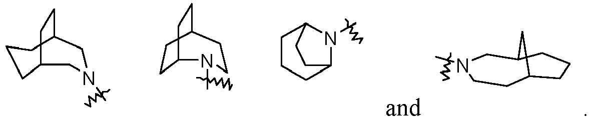

The heterocyclyl ring can be fused to the ring of aryl, heteroaryl or cycloalkyl, wherein the ring bound to the parent structure is heterocyclyl. Non-limiting examples thereof include:

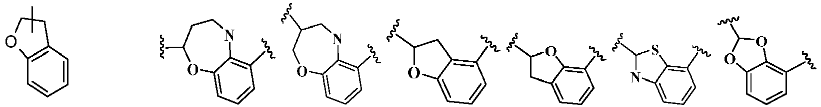

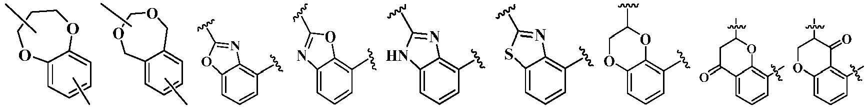

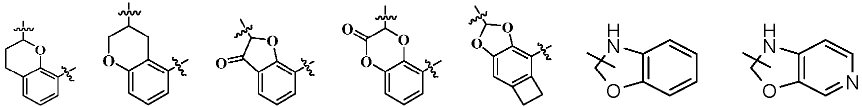

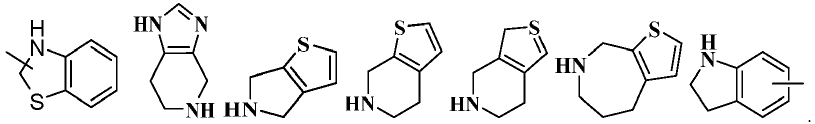

The heterocyclyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "aryl" refers to a 6 to 14 membered all-carbon monocyclic ring or polycyclic fused ring (i.e. each ring in the system shares an adjacent pair of carbon atoms with another ring in the system) having a conjugated π-electron system, preferably 6 to 10 membered aryl, more preferably 6 to 8 membered aryl, for example, phenyl and naphthyl. The aryl is more preferably phenyl. The aryl ring can be fused to the ring of heteroaryl, heterocyclyl or cycloalkyl, wherein the ring bound to the parent structure is aryl ring. Non-limiting examples thereof include:

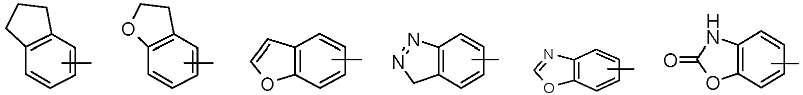

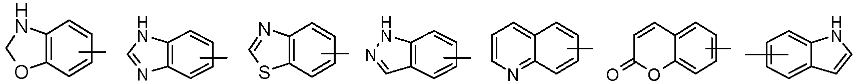

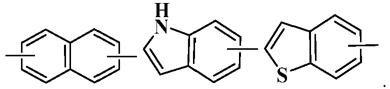

The aryl can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "heteroaryl" refers to a 5 to 14 membered heteroaromatic system having 1 to 4 heteroatoms selected from the group consisting of O, S and N. The heteroaryl is preferably 5 to 10 membered heteroaryl, more preferably 5 to 10 membered heteroaryl, most preferably 5 or 6 membered heteroaryl, for example imidazolyl, furanyl, thienyl, thiazolyl, pyrazolyl, oxazolyl, pyrrolyl, triazolyl, tetrazolyl, pyridyl, pyrimidinyl, thiadiazolyl, pyrazinyl and the like; preferably triazolyl, thienyl, imidazolyl, pyrazolyl or pyrimidinyl, thiazolyl; and more preferably triazolyl, pyrazolyl thienyl, thiazolyl and pyrimidinyl. The heteroaryl ring can be fused to the ring of aryl, heterocyclyl or cycloalkyl, wherein the ring bound to the parent structure is heteroaryl ring. Non-limiting examples thereof include:

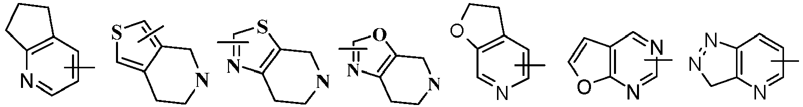

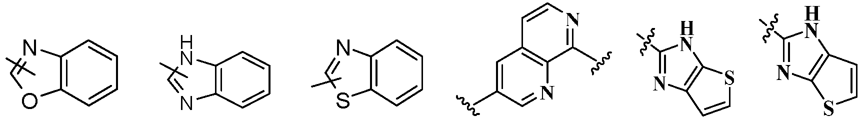

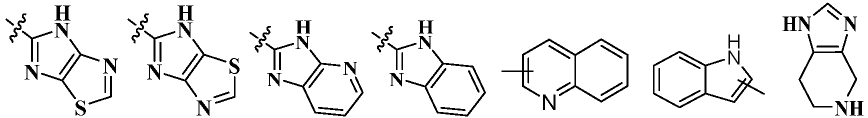

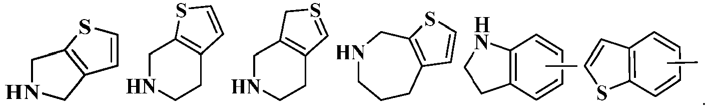

The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "alkoxy" refers to an -O-(alkyl) or an -O-(unsubstituted cycloalkyl) group, wherein the alkyl is as defined above, preferably the alkyl containing 1 to 8 carbon atoms, more preferably the alkyl containing 1 to 6 carbon atoms, and more preferably the alkyl containing 1 to 3 carbon atoms. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The "alkenyl" refers to a chain alkene group, also known as an alkene group, preferably containing 2 to 8 carbon atoms, more preferably 2 to 6 carbon atoms, and most preferably 2 to 3 carbon atoms, for example, ethenyl, 1-propenyl, 2-propenyl, 1-, 2- or 3-butenyl and the like. The alkenyl group can be further substituted by other related group, for example, alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The "alkynyl" refers to (CH=C-), preferably an alkyne group containing 2 to 8 carbon atoms, more preferably an alkyne group containing 2 to 6 carbon atoms, and most preferably an alkyne group containing 2 to 3 carbon atoms. The alkynyl group can be further substituted by other related group, for example, alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "alkynylene" refers to an alkynyl group with one hydrogen being further substituted, for example, "ethynylene" refers to -C≡C-, "propynylene" refers to -C≡C-CH₂-, etc. The alkynylene group can be substituted or unsubstituted, and when substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, and heterocyclylthio.

The "haloalkyl" refers to an alkyl group substituted by one or more halogens, wherein the alkyl is as defined above.

The "haloalkoxy" refers to an alkoxy group substituted by one or more halogens, wherein the alkoxy is as defined above.

The "hydroxyalkyl" refers to an alkyl group substituted by hydroxy(s), wherein the alkyl is as defined above.

The "C₁₋₆ cyanoalkyl" refers to -C₁₋₆ alkyl CN, for example, -CH₂CN, - CH₂CH₂CN, etc.

The "C₁₋₆ carboxy" refers to -C₁₋₅ alkyl COOH.

The "hydroxy" refers to an -OH group.

The "halogen" refers to fluorine, chlorine, bromine or iodine.

The "amino" refers to -NH₂.

The "cyano" refers to -CN.

The "nitro" refers to -NO₂.

The "carboxy" refers to -C(O)OH.

The "THF" refers to tetrahydrofuran.

The "EtOAc" refers to ethyl acetate.

The "MeOH" refers to methanol.

The "DMF" refers to N,N-dimethylformamide.

The "DIPEA" refers to diisopropylethylamine.

The "TFA" refers to trifluoroacetic acid.

The "MeCN" refers to acetonitrile.

The "DMA" refers to N, N-dimethylacetamide.

The "Et₂O" refers to diethyl ether.

The "DCE" refers to 1,2 dichloroethane.

The "DIPEA" refers to N,N-diisopropylethylamine.

The "NBS" refers to N-bromosuccinimide.

The "NIS" refers to N-iodosuccinimide.

The "Cbz-Cl" refers to benzyl chloroformate.

The "Pd₂(dba)₃" refers to tris(dibenzylideneacetone)dipalladium.

The "Dppf' refers to 1,1'-bis(diphenylphosphino)ferrocene.

The "HATU" refers to 2-(7-azabenzotriazol-1-yl)-N,N,N',N'-tetramethyluronium hexafluorophosphate.

The "KHMDS" refers to potassium hexamethyldisilazide.

The "LiHMDS" refers to lithium bis(trimethylsilyl)amide.

The "MeLi" refers to methyl lithium.

The "n-BuLi" refers to n-butyl lithium.

The "NaBH(OAc)₃" refers to sodium triacetoxyborohydride.

The " 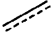 " represents a single bond or a double bond.

Different expressions such as "X is selected from the group consisting of A, B, or C", "X is selected from the group consisting of A, B and C", "X is A, B or C", "X is A, B and C" and the like, express the same meaning, that is, X can be any one or more of A, B and C.

The hydrogen described in the present invention can all be substituted by its isotope deuterium, and any hydrogen in a compound involved in the examples of the present invention can also be substituted by deuterium.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without paying excessive efforts. For example, the combination of amino or hydroxy having free hydrogen and carbon atoms having unsaturated bonds (such as olefinic) may be unstable.

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

A "pharmaceutically acceptable salt" refers to a salt of the compound of the present invention, which is safe and effective in mammals and has the desired biological activity.

### DETAILED DESCRIPTION OF THE INVENTION

### Intermediate Im-1

### Synthesis of methyl (S)-2-(chloromethyl)-1-(oxetan-2-ylmethyl)-1H-benzo[d]imidazole-6-carboxylate

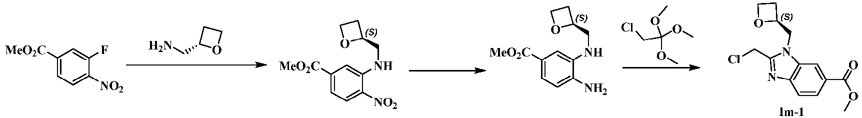

### Step 1

### Methyl (S)-4-nitro-3-((oxetan-2-ylmethyl)amino)benzoate

3-Fluoro-4-(1-(6-(piperidin-4-yl)pyridine-2-yl)oxo)cyclopropyl)benzonitrile (2 g, 10.04 mmol) and K₂CO₃ (2.78 g, 20.08 mmol) were dissolved in tetrahydrofuran (30 mL) in a 50 mL reaction flask, then (*S*)-oxetan-2-ylmethanamine (874 mg, 10.04 mmol) was added, and the reaction solution was stirred at room temperature for 12 hours. The reaction was stopped, water (20 mL) was added to quench the reaction, and the reaction solution was extracted with ethyl acetate (15 mL×2). The combined organic phases were washed with saturated sodium chloride (10 mL), dried over anhydrous sodium sulfate, filtered, and the resulting residue was purified by silica gel column chromatography with petroleum ether and ethyl acetate as eluent system to obtain the title product methyl (*S*)-4-nitro-3-((oxetan-2-ylmethyl)amino)benzoate (2.0 g, yellow solid) with a yield of 74.8%.

MS m/z (ESI): 267.0 [M+1].

### Step 2

### Methyl (S)-4-amino-3-((oxetan-2-ylmethyl)amino)benzoate

Methyl (*S*)-4-nitro-3-((2-oxetan-2-ylmethyl)amino)benzoate (3 g, 11.27 mmol) was dissolved in methanol (30 mL), then 10% Pd/C (300 mg) was added, the reaction system was purged with hydrogen gas three times, and the reaction was stirred for 3 hours. The reaction solution was filtered, the organic phase was dried, and then concentrated to dryness by rotary evaporation to obtain the title product methyl (S)-4-amino-3-((oxetan-2-ylmethyl)amino)benzoate (2.6 g, yellow solid) with a yield of 97.7%.

MS m/z (ESI): 237.1 [M+1].

### Step 3

### Methyl (S)-2-(chloromethyl)-1-(oxetan-2-ylmethyl)-1H-benzo[d]imidazole-6-carboxylate

In a 50 mL reaction flask, methyl (5)-4-nitro-3-((ox etan-2-ylmethyl)amino)benzoate (2 g, 8.46 mmol) and p-toluenesulfonic acid (86 mg, 499.41) were dissolved in tetrahydrofuran (100 mL), then 2-chloro-1,1,1-trimethoxyethane (1.3 g, 8.41 mmol) was added, and the reaction solution was stirred at 60°C for 1 hour. The reaction was stopped, cooled to room temperature, concentrated under reduced pressure, and the resulting residue was purified by silica gel column chromatography with petroleum ether and ethyl acetate as eluent system to obtain the title product methyl (*S*)-2-(chloromethyl)-1-(oxetan-2-ylmethyl)-1*H*-benzo[d]imidazole-6-carboxylate (Im-1) (1.3 g, yellow solid) with a yield of 52.0%.

MS m/z (ESI): 295.0 [M+1].

### Intermediate Im-2

### Synthesis of methyl (S)-2-(chloromethyl)-3-(oxetan-2-ylmethyl)-3H-imidazo[4,5-b]pyridine-5-carboxylate

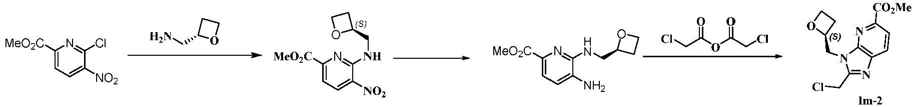

### Step 1

### Methyl (S)-5-nitro-6-((oxetan-2-ylmethyl)amino)picolinate

Using methyl 6-chloro-5-nitro-2-nitropicolinate as the starting material and referring to Step 1 of intermediate Im-1, the title product methyl (*S*)-5-nitro-6-((oxetan-2-ylmethyl)amino)picolinate was obtained.

MS m/z (ESI): 268.1 [M+1].

### Step 2

### Methyl (5)-5-amino-6-((oxetan-2-ylmethyl)amino)picolinate

Using methyl (*S*)-5-nitro-6-((oxetan-2-ylmethyl)amino)picolinate as the starting material and referring to Step 2 of intermediate Im-1, the title product methyl (*S*)-5-amino-6-((oxetan-2-ylmethyl)amino)picolinate was obtained.

MS m/z (ESI): 238.1 [M+1].

### Step 3

### Methyl (S)-2-(chloromethyl)-3-(oxetan-2-ylmethyl)-3H-imidazo[4,5-b]pyridine-5-carboxylate

At room temperature, methyl (*S*)-5-amino-6-((oxetan-2-ylmethyl)amino)picolinate (340 mg, 1.43 mmol) was dissolved in tetrahydrofuran (5 mL), then a solution of chloroacetic anhydride (257.27 mg, 1.50 mmol) in tetrahydrofuran (5 mL) was added dropwise. The reaction solution was stirred at room temperature for 30 minutes, heated to 60°C, reacted for 2 hours, cooled to room temperature, and LCMS showed the end of the reaction. The reaction solution was diluted with ethyl acetate (30 mL), then washed with saturated sodium bicarbonate solution (15 mL×3) and saturated sodium chloride solution (15 mL×3) The organic phase was dried over anhydrous sodium sulfate, filtered, and concentrated to dryness by rotary evaporation to obtain the title product methyl (*S*)-2-(chloromethyl)-3-(oxetan-2-ylmethyl)-3*H*-imidazo[4,5-b]pyridine-5-carboxylate (Im-2) (yellow oil, 0.4 g) with a yield of 94.4%. This crude product was directly used in the next step.

MS m/z (ESI): 296.1 [M+1].

### Intermediate Im-3

### Methyl (S)-2-(chloromethyl)-1-(oxetan-2-ylmethyl)-1H-imidazolo[4,5-b]pyridine-6-carboxylate

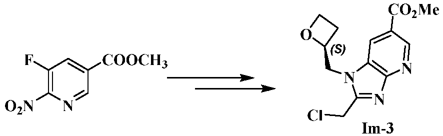

Using methyl 5-fluoro-6-nitronicotinate as the starting material and referring to the synthesis of intermediate Im-2, the product methyl (*S*)-2-(chloromethyl)-1-(oxetan-2-ylmethyl)-1*H*-imidazolo[4,5-b]pyridine-6-carboxylate was obtained.

MS m/z (ESI): 296.1 [M+1].

### Intermediate Im-4

### Synthesis of ethyl 2-(chloromethyl)-1-((1-(fluoromethyl)cyclopropyl)methyl)-1H-benzo[d]imidazole-6-carboxylate

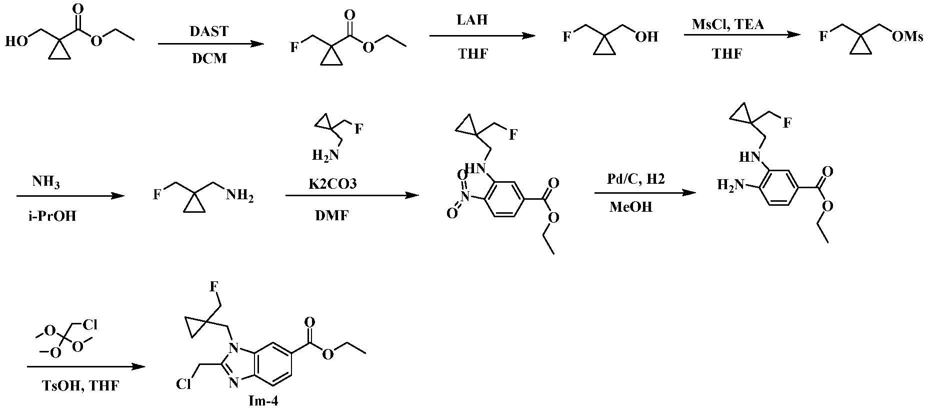

### Step 1

### Ethyl 1-(fluoromethyl)cyclopropane-1-carboxylate

Ethyl 1-(hydroxymethyl)cyclopropane-1-carboxylate (7 g, 48.55 mmol) was dissolved in DCM (100 mL), and DAST (8.61 g, 53.41 mmol) was added thereto at - 78°C. The reaction system was naturally raised to room temperature and stirred for 16 hours. After the reaction was completed, 50 mL of water was added thereto. The reaction solution was extracted with dichloromethane (50 mL×2), washed with saturated sodium chloride solution (30 mL×2), dried over anhydrous sodium sulfate, and concentrated to obtain the title product ethyl 1-(fluoromethyl)cyclopropane-1-carboxylate (6.5 g, yellow oil) was with a yield of 91.6%.

¹H NMR (400 MHz, CDCl₃): δ 4.52 (dd, 1.5 Hz, 2H), 4.17 (q, 2H), 1.41 - 1.33 (m, 2H), 1.26 (t, 3H), 1.05 - 0.95 (m, 2H).

### Step 2

### (1-(Fluoromethyl)cyclopropyl)methanol

Ethyl 1-(fluoromethyl)cyclopropane-1-carboxylate (6.5 g, 44.47 mmol) was dissolved in THF (60 mL), and LiAlH4 (2.53 g, 66.71 mmol) was added thereto in an ice water bath. The reaction system was naturally raised to room temperature and stirred for 16 hours. After the reaction was completed, 15 g of sodium sulfate decahydrate was added thereto to quench the reaction. The reaction solution was filtered and concentrated to obtain the title product (1-(fluoromethyl)cyclopropyl)methanol (3.5 g, yellow oil) with a yield of 75.6%.

¹H NMR (400 MHz, CDCl₃): δ 4.36 (d, 2H), 3.58 (s, 2H), 0.60 (m, 4H).

### Step 3

### (1-(Fluoromethyl)cyclopropyl)methylmethanesulfonate

(1-(Fluoromethyl)cyclopropyl)methanol (1.3 g, 12.49 mmol) was dissolved in DCM (30 mL), and methylsulfonyl chloride (1.86 g, 16.23 mmol, 1.26 mL) and triethylamine (2.53 g, 24.97 mmol, 3.48 mL) were added dropwise thereto in an ice water bath. The reaction system was stirred at 20°C. After the reaction was completed, saturated NaHCO₃ (10 mL) was added dropwise to quench the reaction. The reaction solution was extracted with dichloromethane (20 mL×3), washed with saturated sodium chloride solution (20 mL×2), dried over anhydrous sodium sulfate, and concentrated to obtain the crude (1-(fluoromethyl)cyclopropyl)methylmethanesulfonate (2.0g, light yellow oil) with a yield of 87.0%.

¹H NMR (400 MHz, CDCl₃) δ 4.32 (d, 2H), 4.19 (s, 2H), 3.05 (s, 3H), 0.81 - 0.72 (m, 4H).

### Step 4

### (1-(Fluoromethyl)cyclopropyl)methanamine

(1-(Fluoromethyl)cyclopropyl)methylmethanesulfonate (1.0 g, 5.49 mmol) was dissolved in NH3/i-PrO*H*-(10 mL), and the reaction system was heated under microwave at 60°C for 6 hours. After the reaction was completed, the reaction solution was concentrated to obtain the title product (1-(fluoromethyl)cyclopropyl)methanamine (600 mg, yellow oil). The crude product is directly used for the next reaction.

### Step 5

### Ethyl 3-(((1-(fluoromethyl)cyclopropyl)methyl)amino)-4-nitrobenzoate

Ethyl 3-fluoro-4-nitrobenzoate (1.24 g, 5.82 mmol) was dissolved in DMF (30 mL), and (1-(fluoromethyl)cyclopropyl)methanamine (600 mg, 5.82 mmol) and K₂CO₃ (1.61 g, 11.64 mmol) were added thereto. The reaction system was stirred at 20°C for four hours. After the reaction was completed, 10 mL of water was added thereto. The reaction solution was extracted with ethyl acetate (20 mL×2), washed with saturated sodium chloride solution (20 mL×2), dried over anhydrous sodium sulfate, and concentrated to obtain the crude product. The resulting residue was purified by silica gel column chromatography with petroleum ether and ethyl acetate as eluent system to obtain the title product ethyl 3-(((1-(fluoromethyl)cyclopropyl)methyl)amino)-4-nitrobenzoate (1.2 g, light yellow solid) with a yield of 69.6%.

MS m/z (ESI): 297.1 [M+1].

¹H NMR (400 MHz, CDCl₃): δ 8.22 (d, 1H), 7.56 (s, 1H), 7.25 (d, 1H), 4.41 (q, 2H), 4.30 (d, 2H), 3.42 (d, 2H), 1.41 (t, 3H), 0.76 (m, 4H).

### Step 6

### Ethyl 4-amino-3-(((1-(fluoromethyl)cyclopropyl)methyl)amino)benzoate

Ethyl 3-(((1-(fluoromethyl)cyclopropyl)methyl)amino)-4-nitrobenzoate (1.2 g, 4.05 mmol) was dissolved in MeO*H*-(30 mL), and Pd/C (200 mg, 10% purity) was added thereto. The reaction system was purged with hydrogen gas three times, and stirred at 20°C for 2 hours. After the reaction was completed, the reaction solution was filtered through diatomaceous earth, and the filtrate was concentrated to obtain the title product ethyl 4-amino-3-(((1-(fluoromethyl)cyclopropyl)methyl)amino)benzoate (1.0 g, yellow solid) with a yield of 92.7%.

MS m/z (ESI): 267.1 [M+1].

### Step 7

### Ethyl 2-(Chloromethyl)-1-((1-(fluoromethyl)cyclopropyl)methyl)-1H-benzo[d]imidazole-6-carboxylate

Ethyl 4-amino-3-(((1-(fluoromethyl)cyclopropyl)methyl)amino) benzoate (1.0 g, 3.76 mmol) was dissolved in MeCN (30 mL), and p-toluenesulfonic acid (193.99 mg, 1.13 mmol) and 2-chloro-1,1,1-trimethoxyethane (1.16 g, 7.51 mmol) were added thereto. The reaction system was stirred for 4 hours in an oil bath at 60°C. After the reaction was completed, the reaction solution was concentrated to obtain the crude product. The resulting residue was purified by silica gel column chromatography with petroleum ether and ethyl acetate as eluent system to obtain ethyl 2-(chloromethyl)-1-((1-(fluoromethyl)cyclopropyl)methyl)-1*H*-benzo[d]imidazole-6-carboxylate (700 mg, yellow solid) with a yield of 57.4%.

MS m/z (ESI): 325.1 [M+1].

¹H NMR (400 MHz, CDCl₃): δ 8.21 (s, 1H), 8.04(d,1H), 7.81 (d, 1H), 4.97 (s, 2H), 4.47 (s, 2H), 4.44 (q, 2H), 4.03 (d, 2H), 1.43 (t, 3H), 0.92 - 0.82 (m, 4H).

### Intermediate Im-5

### Synthesis of 2-(chloromethyl)-3-((1-(fluoromethyl)cyclopropyl)methyl)-3H-imidazolo[4,5-b]pyridine-5-carboxylate

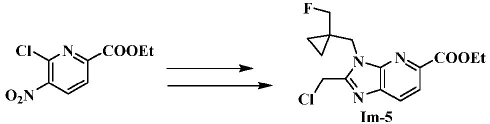

Using ethyl 6-chloro-5-nitropicolinate as the starting material and referring to the synthesis of intermediate Im-4, the title product 2-(chloromethyl)-3-(((1-(fluoromethyl)cyclopropyl)methyl)-3*H*-imidazolo[4,5-b]pyridine-5-carboxylate was obtained.

MS m/z (ESI): 326.10 [M+1].

### Intermediate Im-6

### Methyl 2-(chloromethyl)-1-((1-(fluoromethyl)cyclopropyl)methyl)-1H-imidazolo[4,5-b]pyridine-6-carboxylate

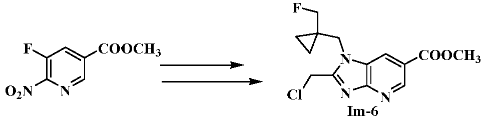

Using methyl 5-fluoro-6-nitronicotinate as the starting material and referring to the synthesis of intermediate Im-4, the product methyl 2-(chloromethyl)-1-((1-(fluoromethyl)cyclopropyl)methyl)-1*H*-imidazolo[4,5-b]pyridine-6-carboxylate was obtained.

MS m/z (ESI): 312.1 [M+1].

### Intermediate Im-7

### Tert-butyl 4-(3-acetyl-2-hydroxyphenyl)piperidine-1-carboxylate

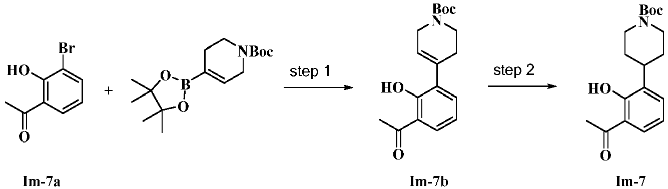

### Step 1

### Tert-butyl 4-(3-acetyl-2-hydroxyphenyl)-3,6-dihydropyridine-1(2H)-carboxylate

1-(3-Bromo-2-hydroxyphenyl)ethan-1-one **Im-7a** (20 g, 0.09 mol), tert-butyl 4-(4,4,5,5-tetramethyl-l,3,2-dioxaborolan-2-yl)-3,6-dihydropyridine-1(2H)-carboxylate (32 g, 0.10 mol), Pd(dppf)Cl₂·CH₂Cl₂ (7.6 g, 9.40 mmol), and anhydrous potassium carbonate (39 g, 0.28 mol) were dissolved in a mixed solvent of 250 mL of dioxane and water (4:1). The reaction was heated to 100°C and stirred for 8 hours. The reaction was stopped, the reaction solution was cooled to ambient temperature, filtered through diatomaceous earth. The filtrate was concentrated under reduced pressure, and the residue was purified by silica gel column chromatography with petroleum ether and ethyl acetate as eluent system to obtain the title product tert-butyl 4-(3-acetyl-2-hydroxyphenyl)-3,6-dihydropyridine-1(2H)-carboxylate **Im-7b** (26 g, colorless oil) with a yield of 88.1%.

MS m/z (ESI): 318.1 [M+1].

### Step 2

### Tert-butyl 4-(3-acetyl-2-hydroxyphenyl)piperidine-1-carboxylate

Tert-butyl 4-(3-acetyl-2-hydroxyphenyl)-3,6-dihydropyridine-1(2H)carboxylate Im-7b (26 g, 0.08 mol) and Pd/C (2.6 g, 10% wt.) were dispersed in methanol (300 mL), and the reaction system was purged with hydrogen gas three times to discharge air. The reaction solution was stirred for 12 hours. The reaction was stopped, and the reaction solution was filtered through diatomaceous earth. The filtrate was concentrated under reduced pressure, and the residue was purified by silica gel column chromatography with petroleum ether and ethyl acetate as eluent system to obtain the title product, tert-butyl 4-(3-acetyl-2-hydroxyphenyl)piperidine-1-carboxylate **Im-7** (25 g, white solid) with a yield of 95.6%.

MS m/z (ESI): 320.1 [M+1].

### Example 1

### 2-((4-(2-(4-Chloro-2-fluorophenyl)-4-fluoro-2H-chromen-8-yl)piperidin-1-yl)methyl)-1-(((S)-oxetan-2-yl)methyl)-1H-benzo[d]imidazole-6-carboxylic acid

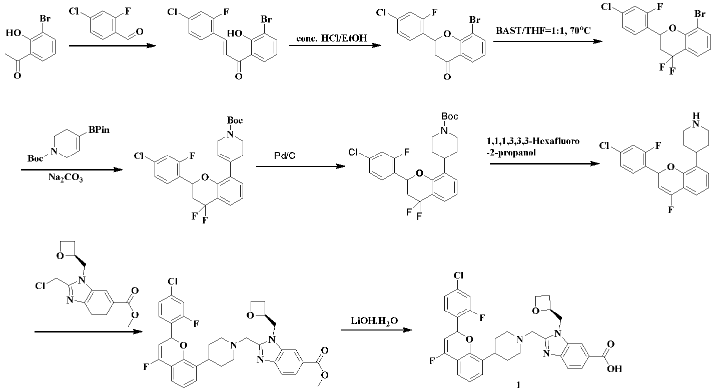

### Step 1

### (E)-1-(3-Bromo-2-hydroxyphenyl)-3-(4-chloro-2-fluorophenyl)prop-2-en-1-one

Sodium tetraborate decahydrate (45.97 g, 66.50 mmol) was added to a solution of 1-(3-bromo-2-hydroxyphenyl)ethan-1-one (13 g, 60.45 mmol) and 4-chloro-2-fluorobenzaldehyde (10.06 g, 63.48 mmol) in ethanol (125 mL) and water (200 mL), and stirred at 90°C for 12 hours. The reaction solution was cooled, and filtered. The filter cake was washed with water, and dried to obtain the title product (*E*)-1-(3-bromo-2-hydroxyphenyl)-3-(4-chloro-2-fluorophenyl)prop-2-en-1-one (20 g, yellow solid) with a yield of 93.0%.

MS m/z (ESI): 354.9 [M+1]

### Step 2

### 8-Bromo-2-(4-chloro-2-fluorophenyl)chroman-4-one

A solution of (*E*)-1-(3-bromo-2-hydroxyphenyl)-3-(4-chloro-2-fluorophenyl)prop-2-en-1-one (2 g, 5.62 mmol) and concentrated hydrochloric acid (4 mL) in ethanol (10 mL) was stirred under microwave at 110°C for 20 hours, cooled, and concentrated to dryness by rotary evaporation. The resulting residue was purified by silica gel column chromatography with petroleum ether and ethyl acetate as eluent system to obtain the title product 8-bromo-2-(4-chloro-2-fluorophenyl)chroman-4-one (1.8 g, yellow solid), with a yield of 90.0%.

MS m/z (ESI): 355.0 [M+1]

### Step 3

### 8-Bromo-2-(4-chloro-2-fluorophenyl)-4,4-difluorochromane

Bis 2-Methoxy-N-(2-methoxyethyl)-N-(trifluoro-sulfoalkyl) ethylamine (10 mL) was added dropwise into a solution of 8-bromo-2-(4-chloro-2-fluorophenyl)chroman-4-one (1.8 g, 5.06 mmol) in tetrahydrofuran (10 mL), then stirred at 70°C for 12 hours, and cooled. Water was added to quench the reaction, and the reaction solution was extracted with dichloromethane (50 mL×3). The organic phase was washed with saturated sodium bicarbonate solution (50 mL×2) and saturated sodium chloride solution (50 mL×2), dried over anhydrous sodium sulfate, filtered, and concentrated to dryness by rotary evaporation. The resulting residue was purified by silica gel column chromatography with petroleum ether and ethyl acetate as eluent system to obtain the title product 8-bromo-2-(4-chloro-2-fluorophenyl)-4,4-difluorochromane (400 mg, yellow solid) with a yield of 20.9%.

MS m/z (ESI): 377.0 [M+1]

### Step 4

### Tert-butyl 4-[2-(4-chloro-2-fluorophenyl)-4,4-difluorochroman-8-yl]-3,6-dihydropyridine-1(2H)-carboxylate

A mixture of 8-bromo-2-(4-chloro-2-fluorophenyl)-4,4-difluorochromane (400 mg, 1.06 mmol), tert-butyl 4-(4,4,5,5-tetramethyl-1,3,2-dioxaborolan-2-yl)-3,6-dihydropyridine-1(2H)-carboxylate (360.32 mg, 1.17 mmol), sodium carbonate (280.71 mg, 2.65 mmol), [1,1'-Bis(diphenylphosphino)ferrocene]palladium chloride (77.44 mg, 105.94 µmol), 1,4-dioxane (10 mL), and water (2 mL) was stirred at 100°C under a nitrogen atmosphere for 2 hours, cooled, and water was added. The reaction solution was extracted with dichloromethane (10 mL×2), and the organic phase was washed with saturated sodium chloride solution (20 mL×2), dried over anhydrous sodium sulfate, filtered, and concentrated to dryness by rotary evaporation. The resulting residue was purified by silica gel column chromatography with petroleum ether and ethyl acetate as eluent system to obtain the title product tert-butyl 4-[2-(4-chloro-2-fluorophenyl)-4,4-difluorochroman-8-yl]-3,6-dihydropyridine-1(2H)-carboxylate (450 mg, yellow oil) with a yield of 88.5%.

MS m/z (ESI): 480.1 [M+1]

### Step 5

### Tert-butyl 4-[2-(4-chloro-2-fluorophenyl)-4,4-difluorochroman-8-yl]piperidine-1-carboxylate

A mixture of tert-butyl 4-[2-(4-chloro-2-fluorophenyl)-4,4-difluorochroman-8-yl]-3,6-dihydropyridine-1(2H)-carboxylate (450 mg, 937.66 µmol), palladium on carbon (80 mg, 10%), and ethyl acetate (30 mL) was purged with hydrogen gas three times and stirred for 5 hours. The reaction solution was filtered and concentrated to dryness by rotary evaporation to obtain the title product tert-butyl 4-[2-(4-chloro-2-fluorophenyl)-4,4-difluorochroman-8-yl]piperidine-1-carboxylate (380 mg, light yellow oil) with a yield of 84.1%.

MS m/z (ESI): 482.2 [M+1]

### Step 6

### 4-[2-(4-Chloro-2-fluorophenyl)-4-fluoro-2H-chromen-8-yl]piperidine

A mixture of tert-butyl 4-[2-(4-chloro-2-fluorophenyl)-4,4-difluorochroman-8-yl]piperidine-1-carboxylate (340 mg, 705.49 µmol), and hexafluoroisopropanol (10 mL) was stirred at 80°C under microwave for 4 hours, cooled, and the filtrate was concentrated under reduced pressure. The resulting residue was purified by silica gel column chromatography with petroleum ether and ethyl acetate as eluent system to obtain the title product 4-[2-(4-chloro-2-fluorophenyl)-4-fluoro-2*H*-chromen-8-yl]piperidine (40 mg, colorless oil) with a yield of 15.7%.

MS m/z (ESI): 362.1 [M+1]

### Step 7

### Methyl 2-[4-[2-(4-chloro-2-fluorophenyl)-4-fluoro-2H-chromen-8-yl]piperidin-1-yl]methyl]-3-[[1-(fluoromethyl)cyclopropyl]methyl]benzimidazol-5-carboxylate

A mixture of 4-[2-(4-chloro-2-fluorophenyl)-4-fluoro-2*H*-chromen-8-yl]piperidine (40 mg, 110.55 µmol), Im-1 (46.68 mg, 143.72 µmol), potassium carbonate (61.03 mg, 442.22 µmol) and acetonitrile (5 mL) was stirred at 50°C for 3 hours, cooled, and 5 mL of water was added. The reaction solution was extracted with dichloromethane (20 mL×3), and the organic phase was washed with saturated sodium chloride solution (20 mL×2), dried over anhydrous sodium sulfate, filtered, and the filtrate was concentrated under reduced pressure. The resulting residue was purified by silica gel column chromatography with petroleum ether and ethyl acetate as eluent system to obtain the title product methyl 2-[4-[2-(4-chloro-2-fluorophenyl)-4-fluoro-2*H*-chromen-8-yl]piperidin-1-yl]methyl]-3-[[1-(fluoromethyl)cyclopropyl]methyl]benzimidazole-5-carboxylate (30 mg, colorless oil) with a yield of 43.7%.

MS m/z (ESI): 620.2 [M+1]

### Step 8

### 2-((4-(2-(4-Chloro-2-fluorophenyl)-4-fluoro-2H-chromen-8-yl)piperidin-1-yl)methyl)-1-(((S)-oxetan-2-yl)methyl)-1H-benzo[d]imidazole-6-carboxylic acid

A mixture of methyl 2-[4-[2-(4-chloro-2-fluorophenyl)-4-fluoro-2*H*-chromen-8-yl]piperidin-1-yl]methyl]-3-[[1-(fluoromethyl)cyclopropyl]methyl]benzimidazol-5-carboxylate (30 mg, 48.38 µmol, lithium hydroxide monohydrate (20 mg, 476.62 µmol), methanol (2 mL), water (2 mL), and tetrahydrofuran (3 mL) was stirred at room temperature for 12 hours, adjusted to pH=6 with formic acid, and concentrated to dryness by rotary evaporation. The resulting residue was purified by silica gel column chromatography with petroleum ether and ethyl acetate as eluent system to obtain 2-((4-(2-(4-chloro-2-fluorophenyl)-4-fluoro-2*H*-chromen-8-yl)piperidin-1-yl)methyl)-1-(((*S*)-oxetan-2-yl)methyl)-1*H*-benzo[d]imidazole-6-carboxylic acid (20 mg, white solid) with a yield of 68.2%.

MS m/z (ESI): 606.2 [M+1].

2-((4-(2-(4-Chloro-2-fluorophenyl)-4-fluoro-2*H*-chromen-8-yl)piperidin-1-yl)methyl)-1-(((*S*)-oxetan-2-yl)methyl)-1*H*-benzo[d]imidazol-6-carboxylic acid was subjected to chiral separation to obtain

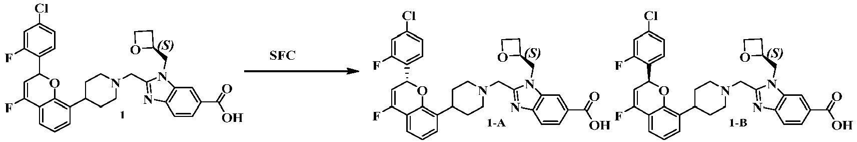

### 2-((4-((R)-2-(4-chloro-2-fluorophenyl)-4-fluoro-2H-chromen-8-yl)piperidin-1-yl)methyl)-1-(((S)-oxetan-2-yl)methyl)-1H-benzo[d]imidazole-6-carboxylic acid (1-A)

MS m/z (ESI): 606.2 [M+1].

### 2-((4-((S)-2-(4-chloro-2-fluorophenyl)-4-fluoro-2H-chromen-8-yl)piperidin-1-yl)methyl)-1-(((S)-oxetan-2-yl)methyl)-1H-benzo[d]imidazole-6-carboxylic acid (1-B)

MS m/z (ESI): 606.2 [M+1].

### Example 2

### 2-((4-(2-(4-Chloro-2-fluorophenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid

Replacing Im-1 with Im-2, and referring to Example 1, 2-((4-(2-(4-chloro-2-fluorophenyl)-4-fluoro-2*H*-chromen-8-yl)piperidin-1-yl)methyl)-3-(((*S*)-oxetan-2-yl)methyl)-3*H*-imidazolo[4,5-b]pyridine-5-carboxylic acid was obtained.

MS m/z (ESI): 607.2 [M+1].

2-((4-(2-(4-Chloro-2-fluorophenyl)-4-fluoro-2*H*-chromen-8-yl)piperidin-1-yl)methyl)-3-(((*S*)-oxetan-2-yl)methyl)-3*H*-imidazolo[4,5-b]pyridine-5-carboxylic acid was subjected to chiral separation to obtain

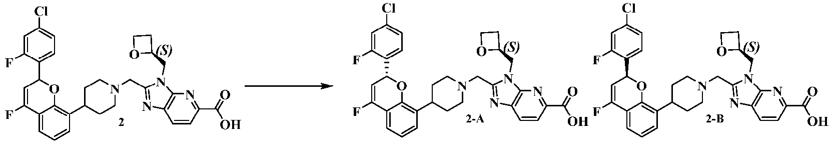

### 2-((4-((R)-2-(4-chloro-2-fluorophenyl)-4-fluoro-2H-chroman-8-yl)piperidin-1-yl)methyl)-3-(((S)-oxetan-2-yl)methyl)-3H-imidazolo[4,5-b]pyridine-5-carboxylic acid (Example 2-A)

MS m/z (ESI): 607.2 [M+1].

### 2-((4-((S)-2-(4-chloro-2-fluorophenyl)-4-fluoro-2H-chroman-8-yl)piperidin-1-yl)methyl)-3-(((S)-oxetan-2-yl)methyl)-3H-imidazolo[4,5-b]pyridine-5-carboxylic acid (Example 2-B)

MS m/z (ESI): 607.2 [M+1].

### Example 3

### 2-((4-(2-(4-Chloro-2-fluorophenyl)-4-fluoro-2H-chromen-8-yl)piperidin-1-yl)methyl)-1-((1-(fluoromethyl)cyclopropyl)methyl)-1H-benzo[d]imidazole-6-carboxylic acid

Replacing Im-1 with Im-4 and referring to Example 1, 2-((4-(2-(4-chloro-2-fluorophenyl)-4-fluoro-2*H*-chromen-8-yl)piperidin-1-yl)methyl)-1-((1-(fluoromethyl)cyclopropyl)methyl)-1*H*-benzo[d]imidazole-6-carboxylic acid was obtained.

MS m/z (ESI): 622.2 [M+1].

2-((4-(2-(4-Chloro-2-fluorophenyl)-4-fluoro-2*H*-chromen-8-yl)piperidin-1-yl)methyl)-1-(1-(fluoromethyl)cyclopropyl)methyl)-1*H*-benzo[d]imidazole-6-carboxylic acid was subjected to chiral separation to obtain

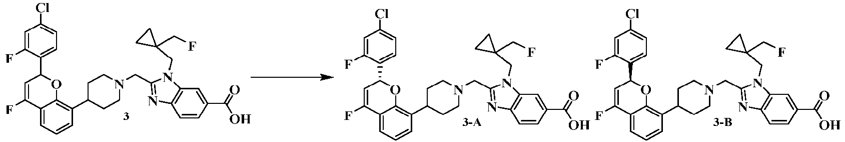

### (R)-2-((4-(2-(4-chloro-2-fluorophenyl)-4-fluoro-2H-chromen-8-yl)piperidin-1-yl)methyl)-1-((1-(fluoromethyl)cyclopropyl)methyl)-1H-benzo[d]imidazole-6-carboxylic acid (Example 3-A)

MS m/z (ESI): 622.2 [M+1].

### (S)-2-((4-(2-(-4-chloro-2-fluorophenyl)-4-fluoro-2H-chromen-8-yl)piperidin-1-yl)methyl)-1-((1-(fluoromethyl)cyclopropyl)methyl)-1H-benzo[d]imidazole-6-carboxylic acid (Example 3-B)

MS m/z (ESI): 622.2 [M+1].

### Example 4

### 2-((4-(2-(4-Chloro-2-fluorophenyl)-4-fluoro-2H-chromen-8-yl)piperidin-1-yl)methyl)-3-((1-(fluoromethyl)cyclopropyl)methyl)-3H-imidazolo[4,5-b]pyridine-5-carboxylic acid

Replacing Im-1 with Im-5 and referring to Example 1, 2-((4-(2-(4-chloro-2-fluorophenyl)-4-fluoro-2*H*-chromen-8-yl)piperidin-1-yl)methyl)-3-((1-(fluoromethyl)cyclopropyl)methyl)-3*H*-imidazolo[4,5-b]pyridine-5-carboxylic acid was obtained.

MS m/z (ESI): 623.2 [M+1].

2-((4-(2-(4-Chloro-2-fluorophenyl)-4-fluoro-2*H*-chromen-8-yl)piperidin-1-yl)methyl)-3-(1-(fluoromethyl)cyclopropyl)methyl)-3*H*-imidazolo[4,5-b]pyridine-5-carboxylic acid was subjected to chiral separation to obtain

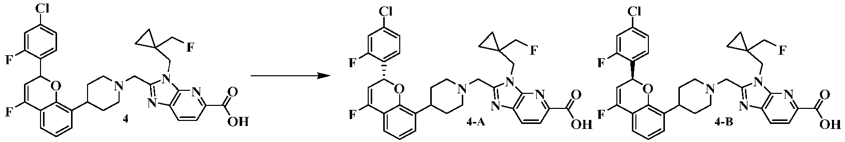

### (R)-2-((4-(2-(4-chloro-2-fluorophenyl)-4-fluoro-2H-chromen-8-yl)piperidin-1-yl)methyl)-3-(1-(fluoromethyl)cyclopropyl)methyl)-3H-imidazolo[4,5-b]pyridine-5-carboxylic acid (Example 4-A)

MS m/z (ESI):623.2 [M+1].

### (S)-2-((4-(2-(4-chloro-2-fluorophenyl)-4-fluoro-2H-chromen-8-yl)piperidin-1-yl)methyl)-3-(1-(fluoromethyl)cyclopropyl)methyl)-3H-imidazolo[4,5-b]pyridine-5-carboxylic acid (Example 4-B)

MS m/z (ESI):623.2 [M+1].

### Example 5

### 2-((4-(2-(2,4-Dichlorophenyl)-4-fluoro-2H-chromen-8-yl)piperidin-1-yl)methyl)-3-(1-(fluoromethyl)cyclopropyl)methyl)-3H-imidazolo[4,5-b]pyridine-5-carboxylic acid

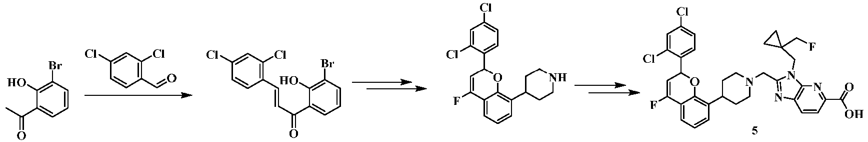

### Step 1

(*E*)-1-(3-Bromo-2-hydroxyphenyl)-3-(2,4-dichlorophenyl)prop-2-en-1-one Using 2,4-dichlorobenzaldehyde as the starting material, and referring to Step 1 of Example 1, (*E*)-1-(3-bromo-2-hydroxyphenyl)-3-(2,4-dichlorophenyl)prop-2-en-1-one was obtained.

MS m/z (ESI): 371.0 [M+1]

### Step 2

### 4-(2-(2,4-Dichlorophenyl)-4-fluoro-2H-chromen-8-yl)piperidine

Using (*E*)-1-(3-bromo-2-hydroxyphenyl)-3 -(2,4-dichlorophenyl)prop-2-en-1-one as the starting material, and referring to Step 2 to Step 6 of Example 1, 4-(2-(2,4-dichlorophenyl)-4-fluoro-2*H*-chromen-8-yl)piperidine was obtained.

MS m/z (ESI): 378.1 [M+1]

### Step 3

### 2-((4-(2-(2,4-Dichlorophenyl)-4-fluoro-2H-chromen-8-yl)piperidin-1-yl)methyl)-3-(1-(fluoromethyl)cyclopropyl)methyl)-3H-imidazolo[4,5-b]pyridine-5-carboxylic acid

Using 4-(2,4-dichlorophenyl)-4-fluoro-2*H*-chromen-8-yl)piperidine and Im-5 as the starting materials, and referring to Step 7 to Step 8 of Example 1, 2-((4-(2,4-dichlorophenyl)-4-fluoro-2*H*-chromen-8-yl)piperidine-1-1)methyl)-3-(1-(fluoromethyl)cyclopropyl)methyl)-3*H*-imidazolo[4,5-b]pyridine-5-carboxylic acid was obtained.

MS m/z (ESI):639.2 [M+1].

2-((4-(2-(2,4-Dichlorophenyl)-4-fluoro-2*H*-chromen-8-yl)piperidin-1-yl)methyl)-3-(1-(fluoromethyl)cyclopropyl)methyl)-3*H*-imidazolo[4,5-b]pyridine-5-carboxylic acid was subjected to chiral separation to obtain

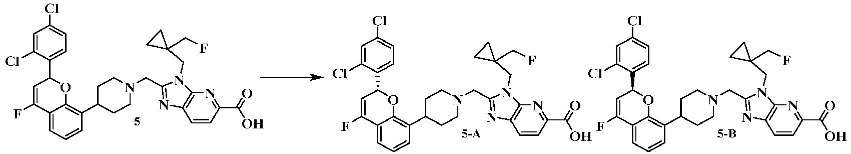

### (R)-2-((4-(2-(2,4-dichlorophenyl)-4-fluoro-2H-chromen-8-yl)piperidin-1-yl)methyl)-3-(1-(fluoromethyl)cyclopropyl)methyl)-3H-imidazolo[4,5-b]pyridine-5-carboxylic acid (Example 5-A)

MS m/z (ESI):639.2 [M+1].

### (S)-2-((4-(2-(2,4-dichlorophenyl)-4-fluoro-2H-chromen-8-yl)piperidin-1-yl)methyl)-3-(1-(fluoromethyl)cyclopropyl)methyl)-3H-imidazolo[4,5-b]pyridine-5-carboxylic acid (Example 5-B)

MS m/z (ESI):639.2 [M+1].

### Example 6

### 2-((4-(2-(4-Chloro-2-fluorophenyl)-4-fluoro-2H-chromen-8-yl)piperidin-1-yl)methyl)-1-(1-(fluoromethyl)cyclopropyl)methyl)-1H-imidazolo[4,5-b]pyridine-6-carboxylic acid

Replacing Im-1 with Im-6 and referring to Example 1, 2-(4-(2-(4-chloro-2-fluorophenyl)-4-fluoro-2*H*-chromen-8-yl)piperidin-1-yl)methyl)-1-(1-(fluoromethyl)cyclopropyl)methyl)-1*H*-imidazolo[4,5-b]pyridine-6-carboxylic acid was obtained.

MS m/z (ESI): 623.2 [M+1].

2-((4-(2-(4-Chloro-2-fluorophenyl)-4-fluoro-2*H*-chromen-8-yl)piperidin-1-yl)methyl)-1-(1-(fluoromethyl)cyclopropyl)methyl)-1*H*-imidazolo[4,5-b]pyridine-6-carboxylic acid was subjected to chiral separation to obtain

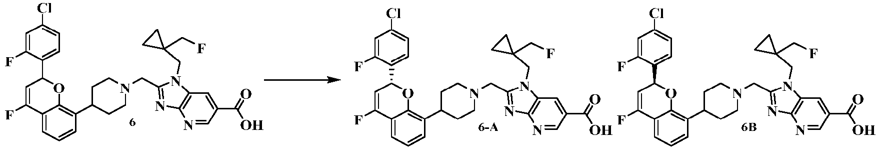

### (R)-2-((4-(2-(4-chloro-2-fluorophenyl)-4-fluoro-2H-chromen-8-yl)piperidin-1-yl)methyl)-1-(1-(fluoromethyl)cyclopropyl)methyl)-1H-imidazolo[4,5-b]pyridine-6-carboxylic acid (Example 6-A)

MS m/z (ESI):623.2 [M+1].

### (S)-2-((4-(2-(4-chloro-2-fluorophenyl)-4-fluoro-2H-chromen-8-yl)piperidin-1-yl)methyl)-1-(1-(fluoromethyl)cyclopropyl)methyl)-1H-imidazolo[4,5-b]pyridine-6-carboxylic acid (Example 6-B)

MS m/z (ESI): 623.2 [M+1].

### Example 7

### 2-((4-(2-(4-Cyano-2-fluorophenyl)-4-fluoro-2H-chromen-8-yl)piperidin-1-yl)methyl)-1-((1-(fluoromethyl)cyclopropyl)methyl)-1H-benzo[d]imidazole-6-carboxylic acid

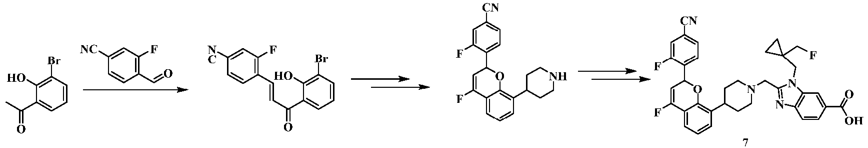

### Step 1

### (E)-4-(3-(3-Bromo-2-hydroxyphenyl)-3-oxoprop-1-en-1-yl)-3-fluorobenzonitrile

Using 3-fluoro-4-formylbenzonitrile as the starting material, and referring to Step 1 of Example 1, (*E*)-4-(3-(3-bromo-2-hydroxyphenyl)-3-oxoprop-1-en-1-yl)-3-fluorobenzonitrile was obtained.

MS m/z (ESI): 346.0 [M+1]

### Step 2

### 3-Fluoro-4-(4-fluoro-8-(piperidin-4-yl)-2H-chromen-2-yl)benzonitrile

Using (*E*)-1-(3-bromo-2-hydroxyphenyl)-3-(2,4-dichlorophenyl)prop-2-en-1-one as the starting material, and referring to Step 2 to Step 6 of Example 1, 3-fluoro-4-(4-fluoro-8-(piperidin-4-yl)-2*H*-chromen-2-yl)benzonitrile was obtained.

MS m/z (ESI): 353.1 [M+1].

### Step 3

### 2-((4-(2-(4-Cyano-2-fluorophenyl)-4-fluoro-2H-chromen-8-yl)piperidin-1-yl)methyl)-1-((1-(fluoromethyl)cyclopropyl)methyl)-1H-benzo[d]imidazole-6-carboxylic acid

Using 3-fluoro-4-(4-fluoro-8-(piperidin-4-yl)-2*H*-chromen-2-yl)benzonitrile and Im-4 as the starting materials, and referring to steps Step 7 to Step 8 of Example 1, 2-((4-(2-(4-cyano-2-fluorophenyl)-4-fluoro-2*H*-chromen-8-yl)piperidin-1-yl)methyl)-1-((1-(fluoromethyl)cyclopropyl)methyl)-1*H*-benzo[d]imidazole-6-carboxylic acid was obtained.

MS m/z (ESI):613.2 [M+1].

2-((4-(2-(4-Cyano-2-fluorophenyl)-4-fluoro-2*H*-chromen-8-yl)piperidin-1-yl)methyl)-1-((1-(fluoromethyl)cyclopropyl)methyl)-1*H*-benzo[d]imidazole-6-carboxylic acid was subjected to chiral separation to obtain

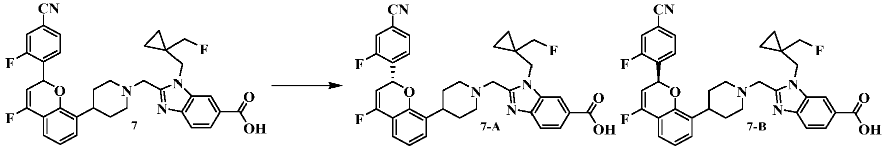

### (R)-2-((4-(2-(4-cyano-2-fluorophenyl)-4-fluoro-2H-chromen-8-yl)piperidin-1-yl)methyl)-1-((1-(fluoromethyl)cyclopropyl)methyl)-1H-benzo[d]imidazole-6-carboxylic acid (Example 7-A)

MS m/z (ESI):613.2 [M+1].

### (S)-2-((4-(2-(4-cyano-2-fluorophenyl)-4-fluoro-2H-chromen-8-yl)piperidin-1-yl)methyl)-1-((1-(fluoromethyl)cyclopropyl)methyl)-1H-benzo[d]imidazole-6-carboxylic acid (Example 7-B)

MS m/z (ESI):613.2 [M+1].

### Example 8

### 2-((4-(2-(4-Cyano-2-fluorophenyl)-4-fluoro-2H-chromen-8-yl)piperidin-1-yl)methyl)-1-(((S)-oxetan-2-yl)methyl)-1H-benzo[d]imidazole-6-carboxylic acid

Using 3-fluoro-4-(4-fluoro-8-(piperidin-4-yl)-2*H*-chromen-2-yl)benzonitrile and Im-1 as the starting materials, and referring to Example 1, 2-((4-(2-(4-cyano-2-fluorophenyl)-4-fluoro-2*H*-chromen-8-yl)piperidin-1-yl)methyl)-1-(((*S*)-oxetan-2-yl)methyl)-1*H*-benzo[d]imidazole-6-carboxylic acid was obtained.

MS m/z (ESI):597.2 [M+1].

2-((4-(2-(4-Cyano-2-fluorophenyl)-4-fluoro-2*H*-chromen-8-yl)piperidin-1-yl)methyl)-1-(((*S*)-oxetan-2-yl)methyl)-1*H*-benzo[d]imidazole-6-carboxylic acid was subjected to chiral separation to obtain

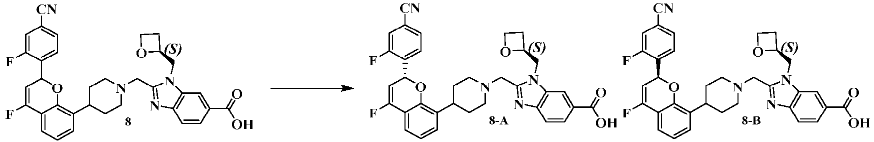

### 2-((4-((R)-2-(4-cyano-2-fluorophenyl)-4-fluoro-2H-chromen-8-yl)piperidin-1-yl)methyl)-1-(((S)-oxetan-2-yl)methyl)-1H-benzo[d]imidazole-6-carboxylic acid (Example 8-A)

MS m/z (ESI):597.2 [M+1].

### 2-((4-((S)-2-(4-cyano-2-fluorophenyl)-4-fluoro-2H-chromen-8-yl)piperidin-1-yl)methyl)-1-(((S)-oxetan-2-yl)methyl)-1H-benzo[d]imidazole-6-carboxylic acid (Example 8-B)

MS m/z (ESI):597.2 [M+1].

### Example 9

### 2-((4-(2-(2,4-Dichlorophenyl)-4-fluoro-2H-chromen-8-yl)piperidin-1-yl)methyl)-1-((1-(fluoromethyl)cyclopropyl)methyl)-1H-imidazolo[4,5-b]pyridine-6-carboxylic acid

Using 4-(2,4-dichlorophenyl)-4-fluoro-2*H*-chromen-8-yl)piperidine and Im-6 as the starting materials, and referring to Example 1, 2-((4-(2-(2,4-dichlorophenyl)-4-fluoro-2*H*-chromen-8-yl)piperidin-1-yl)methyl)-1-((1-(fluoromethyl)cyclopropyl)methyl)-1*H*-imidazolo[4,5-b]pyridine-6-carboxylic acid was obtained.

MS m/z (ESI):639.2 [M+1].

2-((4-(2-(2,4-Dichlorophenyl)-4-fluoro-2*H*-chromen-8-yl)piperidin-1-yl)methyl)-1-(1-(fluoromethyl)cyclopropyl)methyl)-1*H*-imidazolo[4,5-b]pyridine-6-carboxylic acid was subjected to chiral separation to obtain

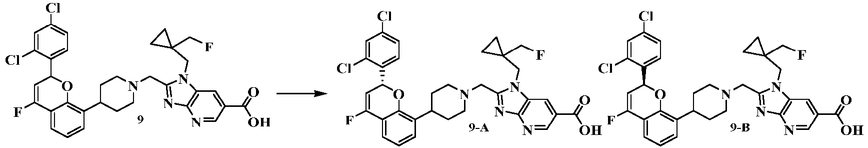

### (R)-2-((4-(2-(2,4-dichlorophenyl)-4-fluoro-2H-chromen-8-yl)piperidin-1-yl)methyl)-1-((1-(fluoromethyl)cyclopropyl)methyl)-1H-imidazolo[4,5-b]pyridine-6-carboxylic acid (Example 9-A)

MS m/z (ESI):639.2 [M+1].

### (S)-2-((4-(2-(2,4-dichlorophenyl)-4-fluoro-2H-chromen-8-yl)piperidin-1-yl)methyl)-1-((1-(fluoromethyl)cyclopropyl)methyl)-1H-imidazolo[4,5-b]pyridine-6-carboxylic acid (Example 9-B)

MS m/z (ESI):639.2 [M+1].

### Example 10

2-((4-(2-(4-Chloro-2-fluorophenyl)-2*H*-chromen-8-yl)piperidin-1-yl)methyl)-3-((1-(fluoromethyl)cyclopropyl)methyl)-3*H*-imidazolo[4,5-b]pyridine-5-carboxylic acid

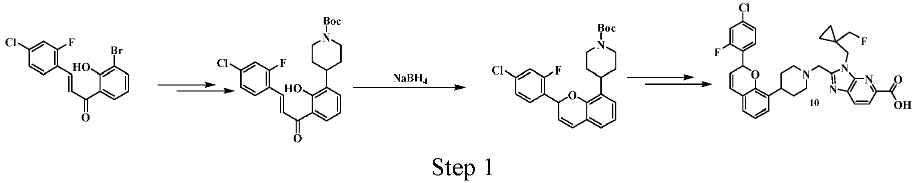

### Tert-butyl (E)-4-(3-(3-(4-chloro-2-fluorophenyl)acryloyl)-2-hydroxyphenyl)piperidine-1-carboxylate

Using (*E*)-1-(3-bromo-2-hydroxyphenyl)-3-(4-chloro-2-fluorophenyl)prop-2-en-1-one as the starting material, and referring to Step 4 and Step 5 of Example 1, tert-butyl (*E*)-4-(3-(4-chloro-2-fluorophenyl)acryloyl)-2-hydroxyphenyl)piperidine-1-carboxylate was obtained.

MS m/z (ESI): 460.2 [M+1].

### Step 2

### Tert-butyl 4-(2-(4-chloro-2-fluorophenyl)-2H-chromen-8-yl)piperidine-1-carboxylate

Sodium borohydride (248 mg, 6.54 mmol) was added to a solution of tert-butyl (*E*)-4-(3-(3-(4-chloro-2-fluorophenyl)acryloyl)-2-hydroxyphenyl)piperidine-1-carboxylate (1 g, 2.18 mmol) in isopropanol (25 mL) at 50°C, then stirred at room temperature for 16 hours. 10 mL of water was added to quench the reaction, and the reaction solution was extracted with dichloromethane (50 mL×3). The organic phase was washed with saturated sodium chloride solution (20 mL×3), dried over anhydrous sodium sulfate, and filtered. The filtrate was concentrated under reduced pressure. The resulting residue was purified by silica gel column chromatography with petroleum ether and ethyl acetate as eluent system to obtain tert-butyl 4-(2-(4-chloro-2-fluorophenyl)-2*H*-chromen-8-yl)piperidine-1-carboxylate (260 mg, light yellow oil) with a yield of 26.9%.

MS m/z (ESI):444.2 [M+1].

### Step 3

### 2-((4-(2-(4-Chloro-2-fluorophenyl)-2H-chromen-8-yl)piperidin-1-yl)methyl)-3-((1-(fluoromethyl)cyclopropyl)methyl)-3H-imidazolo[4,5-b]pyridine-5-carboxylic acid

Using tert-butyl 4-(2-(4-chloro-2-fluorophenyl)-2*H*-chromen-8-yl)piperidine-1-carboxylate and Im-5 as the starting materials, and referring to Step 6 to Step 8 of Example 1, 2-((4-(2-(4-chloro-2-fluorophenyl)-2*H*-chromen-8-yl)piperidin-1-yl)methyl)-3-(1-(fluoromethyl)cyclopropyl)methyl)-3*H*-imidazolo[4,5-b]pyridine-5-carboxylic acid was obtained.

MS m/z (ESI):605.2 [M+1].

2-((4-(2-(4-Chloro-2-fluorophenyl)-2*H*-chromen-8-yl)piperidin-1-yl)methyl)-3-(1-(fluoromethyl)cyclopropyl)methyl)-3*H*-imidazolo[4,5-b]pyridine-5-carboxylic acid was subjected to chiral separation to obtain

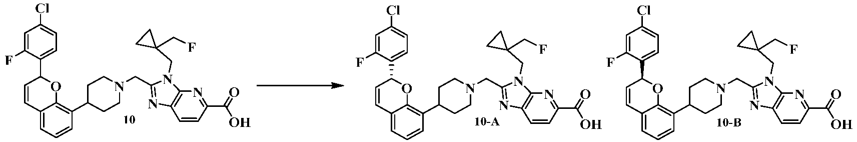

### (R)-2-((4-(2-(4-chloro-2-fluorophenyl)-2H-chromen-8-yl)piperidin-1-yl)methyl)-3-((1-(fluoromethyl)cyclopropyl)methyl)-3H-imidazolo[4,5-b]pyridine-5-carboxylic acid (Example 10-A)

MS m/z (ESI):605.2 [M+1].

### (S)-2-((4-(2-(4-chloro-2-fluorophenyl)-2H-chromen-8-yl)piperidin-1-yl)methyl)-3-((1-(fluoromethyl)cyclopropyl)methyl)-3H-imidazolo[4,5-b]pyridine-5-carboxylic acid (Example 10-B)

MS m/z (ESI):605.2 [M+1].

### Example 11

### 2-((4-(2-(4-Chloro-2-fluorophenyl)-4-fluoro-2H-chromen-8-yl)piperidin-1-yl)methyl)-1-(((S)-oxetan-2-yl)methyl)-1H-imidazolo[4,5-b]pyridine-6-carboxylic acid

Using 4-[2-(4-chloro-2-fluorophenyl)-4-fluoro-2*H*-chromen-8-yl]piperidine and Im-3 as the starting materials, and referring to Example 1, 2-((4-(2-(4-chloro-2-fluorophenyl)-4-fluoro-2*H*-chromen-8-yl)piperidin-1-yl)methyl)-1-(((*S*)-oxetan-2-yl)methyl)-1*H*-imidazolo[4,5-b]pyridine-6-carboxylic acid was obtained.

MS m/z (ESI):607.2 [M+1].

2-((4-(2-(4-Chloro-2-fluorophenyl)-4-fluoro-2*H*-chromen-8-yl)piperidin-1-yl)methyl)-1-(((*S*)-oxetan-2-yl)methyl)-1*H*-imidazolo[4,5-b]pyridine-6-carboxylic acid was subjected to chiral separation to obtain

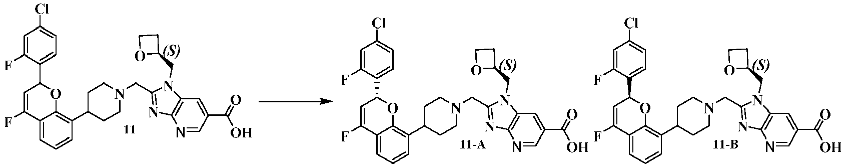

### 2-((4-((R)-2-(4-chloro-2-fluorophenyl)-4-fluoro-2H-chromen-8-yl)piperidin-1-yl)methyl)-1-(((S)-oxetan-2-yl)methyl)-1H-imidazolo[4,5-b]pyridine-6-carboxylic acid (Example 11-A)

MS m/z (ESI):607.2 [M+1].

### 2-((4-((S)-2-(4-chloro-2-fluorophenyl)-4-fluoro-2H-chromen-8-yl)piperidin-1-yl)methyl)-1-(((S)-oxetan-2-yl)methyl)-1H-imidazolo[4,5-b]pyridine-6-carboxylic acid (Examples 11-B)

MS m/z (ESI):607.2 [M+1].

### Example 12

### 2-((4-(2-(2,4-Dichlorophenyl)-4-fluoro-2H-chromen-8-yl)piperidin-1-yl)methyl)-1-((1-(fluoromethyl)cyclopropyl)methyl)-1H-benzo[d]imidazole-6-carboxylic acid

Using 4-(2,4-dichlorophenyl)-4-fluoro-2*H*-chromen-8-yl)piperidine and Im-4 as the starting materials, and referring to Example 1, 2-((4-(2-(2,4-dichlorophenyl)-4-fluoro-2*H*-chromen-8-yl)piperidin-1-yl)methyl)-1-((1-(fluoromethyl)cyclopropyl)methyl)-1*H*-benzo[d]imidazole-6-carboxylic acid was obtained.

MS m/z (ESI):638.2 [M+1].

2-((4-(2-(2,4-Dichlorophenyl)-4-fluoro-2*H*-chromen-8-yl)piperidin-1-yl)methyl)-1-((1-(fluoromethyl)cyclopropyl)methyl)-1*H*-benzo[d]imidazole-6-carboxylic acid was subjected to chiral separation to obtain

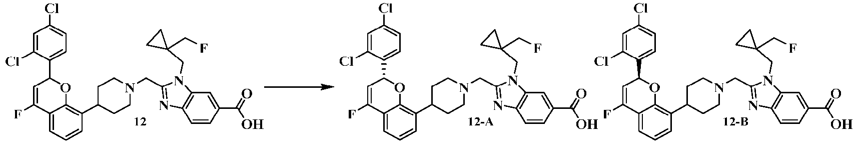

### (R)-2-((4-(2-(2,4-dichlorophenyl)-4-fluoro-2H-chromen-8-yl)piperidin-1-yl)methyl)-1-((1-(fluoromethyl)cyclopropyl)methyl)-1H-benzo[d]imidazole-6-carboxylic acid (Example 12-A)

MS m/z (ESI):638.2 [M+1].

### (S)-2-((4-(2-(2,4-dichlorophenyl)-4-fluoro-2H-chromen-8-yl)piperidin-1-yl)methyl)-1-((1-(fluoromethyl)cyclopropyl)methyl)-1H-benzo[d]imidazole-6-carboxylic acid (Example 12-B)

MS m/z (ESI):638.2 [M+1].

### Example 13

### 2-((4-(2-(2,4-Dichlorophenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid

Using 4-(2,4-dichlorophenyl)-4-fluoro-2*H*-chromen-8-yl)piperidine and Im-2 as the starting materials, and referring to Example 1, 2-((4-(2-(2,4-dichlorophenyl)-4-fluoro-2*H*-chromen-8-yl)piperidin-1-yl)methyl)-3-(((*S*)-oxetan-2-yl)methyl)-3*H-*imidazolo[4,5-b]pyridine-5-carboxylic acid was obtained.

MS m/z (ESI):623.2 [M+1].

2-((4-(2-(2,4-Dichlorophenyl)-4-fluoro-2H-chromen-8-yl)piperidin-1-yl)methyl)-3-(((*S*)-oxetan-2-yl)methyl)-3*H*-imidazolo[4,5-b]pyridine-5-carboxylic acid was subjected to chiral separation to obtain

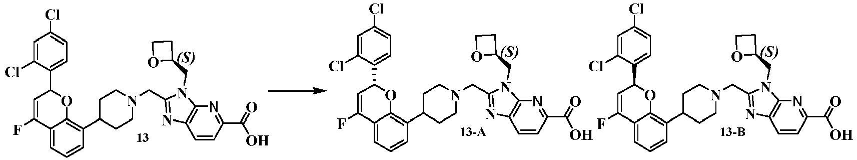

### 2-((4-((R)-2-(2,4-dichlorophenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid (Example 13-A)

MS m/z (ESI):623.2 [M+1].

### 2-((4-((S)-2-(2,4-dichlorophenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid (Example 13-B)

MS m/z (ESI):623.2 [M+1].

### Example 14

### 2-((4-(2-(2,4-Dichlorophenyl)-4-fluoro-2H-chromen-8-yl)piperidin-1-yl)methyl)-1-(((S)-oxetan-2-yl)methyl)-1H-benzo[d]imidazole-6-carboxylic acid

Using 4-(2,4-dichlorophenyl)-4-fluoro-2*H*-chromen-8-yl)piperidine and Im-1 as the starting materials, and referring to Example 1, 2-((4-(2-(2,4-dichlorophenyl)-4-fluoro-2*H*-chromen-8-yl)piperidin-1-yl)methyl)-1-(((*S*)-oxetan-2-yl)methyl)-1*H-*benzo[d]imidazole-6-carboxylic acid was obtained.

MS m/z (ESI):622.2 [M+1].

2-((4-(2-(2,4-Dichlorophenyl)-4-fluoro-2*H*-chromen-8-yl)piperidin-1-yl)methyl)-1-(((*S*)-oxetan-2-yl)methyl)-1*H*-benzo[d]imidazole-6-carboxylic acid was subjected to chiral separation to obtain

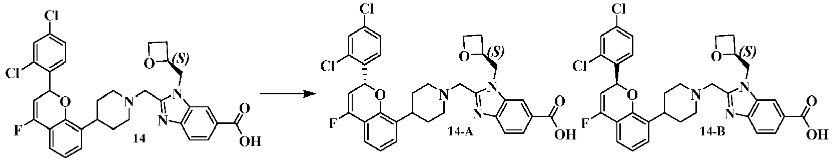

### 2-((4-((R)-2-(2,4-dichlorophenyl)-4-fluoro-2H-chromen-8-yl)piperidin-1-yl)methyl)-1-(((S)-oxetan-2-yl)methyl)-1H-benzo[d]imidazole-6-carboxylic acid (Example 14-A)

MS m/z (ESI):622.2 [M+1].

### 2-((4-((S)-2-(2,4-dichlorophenyl)-4-fluoro-2H-chromen-8-yl)piperidin-1-yl)methyl)-1-(((S)-oxetan-2-yl)methyl)-1H-benzo[d]imidazole-6-carboxylic acid (Example 14-B)

MS m/z (ESI):622.2 [M+1].

### Example 15

### 2-((4-(2-(2,4-Dichlorophenyl)-4-fluoro-2H-chromen-8-yl)piperidin-1-yl)methyl)-1-(((S)-oxetan-2-yl)methyl)-1H-imidazolo[4,5-b]pyridine-6-carboxylic acid

Using 4-(2,4-dichlorophenyl)-4-fluoro-2*H*-chromen-8-yl)piperidine and Im-3 as the starting materials, and referring to Example 1, 2-((4-(2-(2,4-dichlorophenyl)-4-fluoro-2*H*-chromen-8-yl)piperidin-1-yl)methyl)-1-(((*S*)-oxetan-2-yl)methyl)-1*H-*imidazolo[4,5-b]pyridine-6-carboxylic acid was obtained.

MS m/z (ESI):623.2 [M+1].

2-((4-(2-(2,4-Dichlorophenyl)-4-fluoro-2*H*-chromen-8-yl)piperidin-1-yl)methyl)-1-(((*S*)-oxetan-2-yl)methyl)-1*H*-imidazolo[4,5-b]pyridine-6-carboxylic acid was subjected to chiral separation to obtain

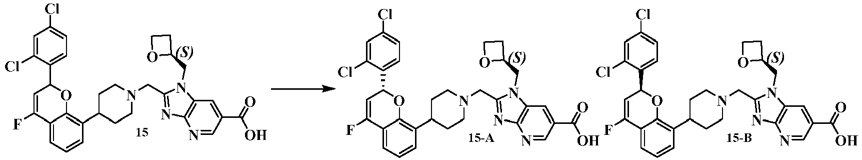

### 2-((4-((R)-2-(2,4-dichlorophenyl)-4-fluoro-2H-chromen-8-yl)piperidin-1-yl)methyl)-1-(((S)-oxetan-2-yl)methyl)-1H-imidazolo[4,5-b]pyridine-6-carboxylic acid (Example 15-A)

MS m/z (ESI):623.2 [M+1].

### 2-((4-((S)-2-(2,4-dichlorophenyl)-4-fluoro-2H-chromen-8-yl)piperidin-1-yl)methyl)-1-(((S)-oxetan-2-yl)methyl)-1H-imidazolo[4,5-b]pyridine-6-carboxylic acid (Example 15-B)

MS m/z (ESI):623.2 [M+1].

### Example 16

### 2-((4-(4-Chloro-2-(4-chloro-2-fluorophenyl)-2H-chromen-8-yl)piperidin-1-yl)methyl)-1-(((S)-oxetan-2-yl)methyl)-1H-benzo[d]imidazole-6-carboxylic acid

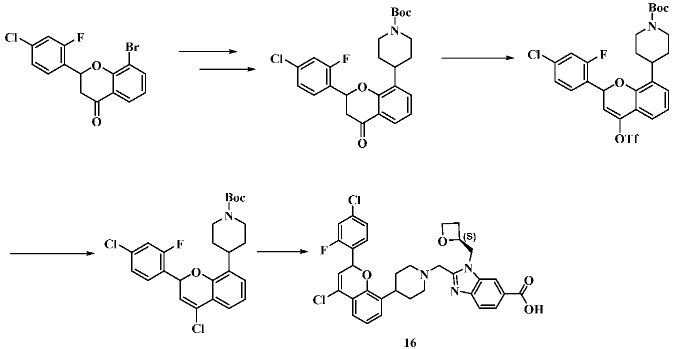

### Step 1

### Tert-butyl 4-(2-(4-chloro-2-fluorophenyl)-4-oxochroman-8-yl)piperidine-1-carboxylate

Using 8-bromo-2-(4-chloro-2-fluorophenyl)chroman-4-one as the starting material, and referring to the Step 4 and Step 5 of Example 1 the product tert-butyl 4-(2-(4-chloro-2-fluorophenyl)-4-oxochroman-8-yl)piperidine-1-carboxylate was obtained.

MS m/z (ESI): 460.1 [M+1]

### Step 2

### Tert-butyl 4-(2-(4-chloro-2-fluorophenyl)-4-(((trifluoromethyl)sulfonyl)oxy)-2H-chromen-8-yl)piperidine-1 -carboxylate

Tert-butyl 4-(2-(4-chloro-2-fluorophenyl)-4-oxochroman-8-yl)piperidine-1-carboxylate (1 g, 2.17 mmol) was dissolved in 20 mL of THF, lithium bis(trimethylsilyl)amide (2.4 mL, 2.40 mmol) was added at -78°C, and reacted for 1 hour. A solution of 2-[N,N-bis(trifluoromethylsulphonyl)amino]-5-chloropyridine (0.94 g, 2.4 mmol) in THF (20 mL) was added at -78°C. The reaction solution was slowly raised to room temperature and stirred for 5 hours. Sodium bicarbonate (10 mL) was added to quench the reaction. The reaction solution was extracted with ethyl acetate (20 mL×3). The combined organic phases were washed with saturated sodium chloride solution (20 mL×2), dried over anhydrous sodium sulfate and concentrated under reduced pressure to obtain the crude product. The resulting residue was purified by silica gel column chromatography with petroleum ether and ethyl acetate as eluent system to obtain tert-butyl 4-(2-(4-chloro-2-fluorophenyl)-4-(((trifluoromethyl)sulfonyl)oxy)-2*H*-chromen-8-yl)piperidine-1-carboxylate (456 mg, yield: 35.5%).

MS m/z (ESI): 592.1[M+1].

### Step 3

### Tert-butyl 4-(4-chloro-2-(4-chloro-2-fluorophenyl)-2H-chromen-8-yl)piperidine-1-carboxylate

Tert-butyl 4-(2-(4-chloro-2-fluorophenyl)-4-(((trifluoromethyl)sulfonyl)oxy)-2*H-*chromen-8-yl)piperidine-1-carboxylate (0.5 g, 0.84 mmol) was dissolved in 10 mL of DMA/THF (v: v=1:3), nickel acetate tetrahydrate (24.8 mg, 0.1 mmol), Zn powder (10 mg, 0.16 mmol), 1,5-cyclooctadiene (11 mg, 0.1 mmol), and lithium chloride (53 mg, 1.3 mmol) were added, and reacted at room temperature under a nitrogen atmosphere for 16 hours. 10 mL of water was added to quench the reaction, and the reaction solution was extracted with ethyl acetate (20 mL×3). The combined organic phases were washed with saturated sodium chloride solution (20 mL×2), dried over anhydrous sodium sulfate and concentrated under reduced pressure to obtain the crude product. The resulting residue was purified by silica gel column chromatography with petroleum ether and ethyl acetate as eluent system to obtain tert-butyl 4-(4-chloro-2-(4-chloro-2-fluorophenyl)-2*H*-chromen-8-yl)piperidine-1-carboxylate (241 mg, yield: 60.0%).

MS m/z (ESI): 478.1[M+1].

### Step 4

### 2-((4-(4-Chloro-2-(4-chloro-2-fluorophenyl)-2H-chromen-8-yl)piperidin-1-yl)methyl)-1-(((S)-oxetan-2-yl)methyl)-1H-benzo[d]imidazole-6-carboxylic acid

Using tert-butyl 4-(4-chloro-2-(4-chloro-2-fluorophenyl)-2*H*-chromen-8-yl)piperidine-1-carboxylate and Im-1 as the starting materials, and referring to Step 6 to Step 8 of Example 1, the product 2-(4-(4-chloro-2-(4-chloro-2-fluorophenyl)-2*H-*chromen-8-yl)piperidin-1-yl)methyl)-1-(((*S*)-oxetan-2-yl)methyl)-1*H-*benzo[d]imidazole-6-carboxylic acid was obtained.

MS m/z (ESI): 622.1[M+1].

2-((4-(4-Chloro-2-(4-chloro-2-fluorophenyl)-2*H*-chromen-8-yl)piperidin-1-yl)methyl)-1-(((*S*)-oxetan-2-yl)methyl)-1*H*-benzo[d]imidazole-6-carboxylic acid was subjected to chiral separation to obtain the product

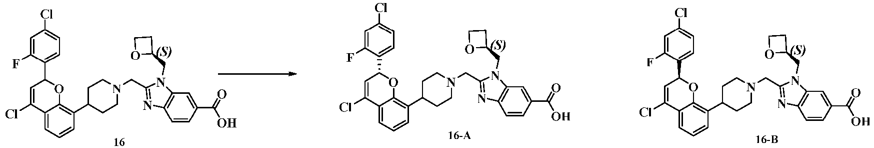

2-((4-((*R*)-4-chloro-2-(4-chloro-2-fluorophenyl)-2*H*-chromen-8-yl)piperidin-1-yl)methyl)-1-(((*S*)-oxetan-2-yl)methyl)-1*H*-benzo[d]imidazole-6-carboxylic acid

### (Example 16-A)

MS m/z (ESI): 622.1[M+1].

2-((4-((*S*)-4-chloro-2-(4-chloro-2-fluorophenyl)-2*H*-chromen-8-yl)piperidin-1-yl)methyl)-1-(((*S*)-oxetan-2-yl)methyl)-1*H*-benzo[d]imidazole-6-carboxylic acid (Example 16-B).

MS m/z (ESI): 622.1[M+1].

### Example 17

### 2-((4-(4-Chloro-2-(4-chloro-2-fluorophenyl)-2H-chromen-8-yl)piperidin-1-yl)methyl)-3-(((S)-oxetan-2-yl)methyl)-3H-imidazolo[4,5-b]pyridine-5-carboxylic acid

Using tert-butyl 4-(4-chloro-2-(4-chloro-2-fluorophenyl)-2*H*-chromen-8-yl)piperidine-1-carboxylate and Im-2 as the starting materials, and referring to Step 6 to Step 8 of Example 1, the product 2-(4-(4-chloro-2-(4-chloro-2-fluorophenyl)-2*H-*chromen-8-yl)piperidin-1-yl)methyl)-3-(((*S*)-oxetan-2-yl)methyl)-3*H*-imidazolo[4,5-b]pyridine-5-carboxylic acid was obtained.

MS m/z (ESI): 623.1[M+1].

2-((4-(4-Chloro-2-(4-chloro-2-fluorophenyl)-2*H*-chromen-8-yl)piperidin-1-yl)methyl)-3-(((*S*)-oxetan-2-yl)methyl)-3*H*-imidazolo[4,5-b]pyridine-5-carboxylic acid was obtained to obtain the product

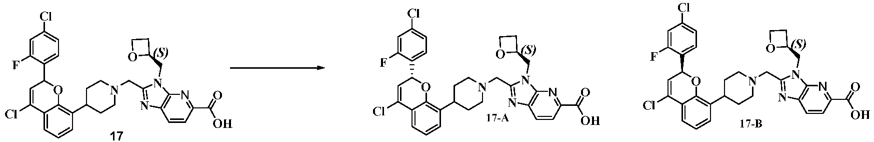

### 2-((4-((R)-4-chloro-2-(4-chloro-2-fluorophenyl)-2H-chromen-8-yl)piperidin-1-yl)methyl)-3-(((S)-oxetan-2-yl)methyl)-3H-imidazolo[4,5-b]pyridine-5-carboxylic acid (Example 17-A)

MS m/z (ESI): 623.1[M+1].

### 2-((4-(((S)-4-chloro-2-(4-chloro-2-fluorophenyl)-2H-chromen-8-yl)piperidin-1-yl)methyl)-3-(((S)-oxetan-2-yl)methyl)-3H-imidazolo[4,5-b]pyridine-5-carboxylic acid (Example 17-B).

MS m/z (ESI): 623.1[M+1].

### Example 18

### 2-((4-(2-(4-Chloro-2-fluorophenyl)-4-methyl-2H-chromen-8-yl)piperidin-1-yl)methyl)-1-(((S)-oxetan-2-yl)methyl)-1H-benzo[d]imidazole-6-carboxylic acid

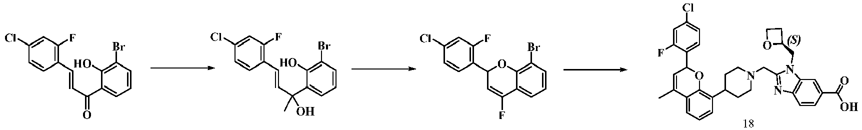

### Step 1

### (E)-2-Bromo-6-(4-(4-chloro-2-fluorophenyl)-2-hydroxybut-3-en-2-yl)phenol

(*E*)-1-(3-Bromo-2-hydroxyphenyl)-3-(4-chloro-2-fluorophenyl)prop-2-en-1-one (1 g, 2.81 mmol) was dissolved in 30 mL of THF, and methylmagnesium bromide (7 mL, 7 mmol) was added at 0°C. The reaction solution was slowly raised to room temperature and stirred for 3 hours. Ammonium chloride (10 mL) was added to quench the reaction, the reaction solution was extracted with ethyl acetate (20 mL×3). The combined organic phases were washed with saturated sodium chloride solution (20 mL×2), dried over anhydrous sodium sulfate and concentrated under reduced pressure to obtain the crude product. The resulting residue was purified by silica gel column chromatography with petroleum ether and ethyl acetate as eluent system to obtain (E)-2-bromo-6-(4-(4-chloro-2-fluorophenyl)-2-hydroxybut-3-en-2-yl)phenol (831 mg, yield: 79.6%).

MS m/z (ESI): 370.9 [M+1].

### Step 2

### 8-Bromo-2-(4-chloro-2-fluorophenyl)-4-fluoro-2H-chromene

(*E*)-2-Bromo-6-(4-(4-chloro-2-fluorophenyl)-2-hydroxybut-3-en-2-yl)phenol (0.5 g, 1.34 mmol) was dissolved in 15 mL of nitromethane, (2,3,4,5-tetrafluorophenyl)boric acid (52 mg, 0.26 mmol) was added, and stirred at 60°C for 16 hours. 10 mL of water was added to quench the reaction, and the reaction solution was extracted with ethyl acetate (20 mL×3), washed with saturated sodium chloride solution (20 mL×2), dried over anhydrous sodium sulfate and concentrated under reduced pressure to obtain the crude product. The resulting residue was purified by silica gel column chromatography with petroleum ether and ethyl acetate as eluent system to obtain 8-bromo-2-(4-chloro-2-fluorophenyl)-4-fluoro-2*H*-chromene (326 mg, yield: 68.0%).

MS m/z (ESI): 356.9 [M+1].

### Step 3

### 2-((4-(2-(4-Chloro-2-fluorophenyl)-4-methyl-2H-chromen-8-yl)piperidin-1-yl)methyl)-1-(((S)-oxetan-2-yl)methyl)-1H-benzo[d]imidazole-6-carboxylic acid

Using 8-bromo-2-(4-chloro-2-fluorophenyl)-4-fluoro-2*H*-chromene as the starting material, and referring to Step 4, Step 5, Step 7, and Step 8 of Example 1, the product 2-((4-(2-(4-chloro-2-fluorophenyl)-4-methyl-2*H*-chromen-8-yl)piperidin-1-yl)methyl)-1-(((*S*)-oxetan-2-yl)methyl)-1*H*-benzo[d]imidazole-6-carboxylic acid was obtained.

MS m/z (ESI): 602.2 [M+1].

2-((4-(2-(4-Chloro-2-fluorophenyl)-4-methyl-2*H*-chromen-8-yl)piperidin-1-yl)methyl)-1-(((*S*)-oxetan-2-yl)methyl)-1*H*-benzo[d]imidazole-6-carboxylic acid was subjected to chiral separation to obtain the product

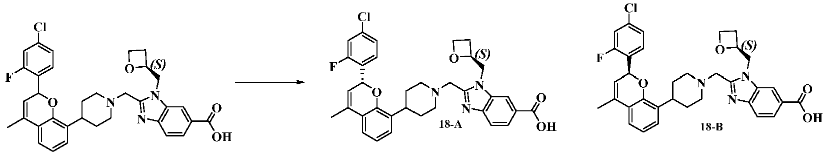

### 2-((4-((R)-2-(4-chloro-2-fluorophenyl)-4-methyl-2H-chromen-8-yl)piperidin-1-yl)methyl)-1-(((S)-oxetan-2-yl)methyl)-1H-benzo[d]imidazole-6-carboxylic acid (Example 18-A)

MS m/z (ESI): 602.2 [M+1].

2-((4-((*S*)-2-(4-chloro-2-fluorophenyl)-4-methyl-2*H*-chromen-8-yl)piperidin-1-yl)methyl)-1-(((*S*)-oxetan-2-yl)methyl)-1*H*-benzo[d]imidazole-6-carboxylic acid (Example 18-B).

MS m/z (ESI): 602.2 [M+1].

### Example 19

### 2-((4-(2-(4-Chloro-2-methoxyphenyl)-4-fluoro-2H-chromen-8-yl)piperidin-1-yl)methyl)-1-(1-(fluoromethyl)cyclopropyl)methyl)-1H-benzo[d]imidazole-6-carboxylic acid

Using 4-chloro-2-methoxybenzaldehyde as the starting material and referring to Example 3, the product 2-((4-(2-(4-chloro-2-methoxyphenyl)-4-fluoro-2*H*-chromen-8-yl)piperidin-1-yl)methyl)-1-(1-(fluoromethyl)cyclopropyl)methyl)-1*H-*benzo[d]imidazole-6-carboxylic acid was obtained.

MS m/z (ESI): 634.2 [M+1].

2-((4-(2-(4-Chloro-2-methoxyphenyl)-4-fluoro-2*H*-chromen-8-yl)piperidin-1-yl)methyl)-1-(1-(fluoromethyl)cyclopropyl)methyl)-1*H*-benzo[d]imidazole-6-carboxylic acid was subjected to chiral separation to obtain the product

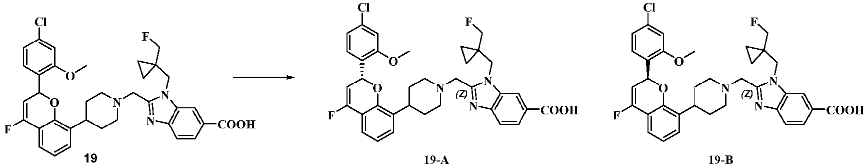

### (R)-2-((4-(2-(4-chloro-2-methoxyphenyl)-4-fluoro-2H-chromen-8-yl)piperidin-1-yl)methyl)-1-(1-(fluoromethyl)cyclopropyl)methyl)-1H-benzo[d]imidazole-6-carboxylic acid (Example 19-A)

MS m/z (ESI): 634.2 [M+1].

(*S*)-2-((4-(2-(4-chloro-2-methoxyphenyl)-4-fluoro-2*H*-chromen-8-yl)piperidin-1-yl)methyl)-1-(1-(fluoromethyl)cyclopropyl)methyl)-1*H*-benzo[d]imidazole-6-carboxylic acid (Example 19-B).

MS m/z (ESI): 634.2 [M+1].

### Example 20

### 2-((4-(2-(4-Chloro-2-methoxyphenyl)-4-fluoro-2H-chromen-8-yl)piperidin-1-yl)methyl)-3-((1-(fluoromethyl)cyclopropyl)methyl)-3H-imidazolo[4,5-b]pyridine-5-carboxylic acid

Using 4-chloro-2-methoxybenzaldehyde as the starting material and referring to Example 4, the product 2-((4-(2-(4-chloro-2-methoxyphenyl)-4-fluoro-2*H*-chromen-8-yl)piperidin-1-yl)methyl)-3-(1-(fluoromethyl)cyclopropyl)methyl)-3*H*-imidazolo[4,5-b]pyridine-5-carboxylic acid was obtained.

MS m/z (ESI): 635.2 [M+1].

2-((4-(2-(4-Chloro-2-methoxyphenyl)-4-fluoro-2*H*-chromen-8-yl)piperidin-1-yl)methyl)-3-(1-(fluoromethyl)cyclopropyl)methyl)-3*H*-imidazolo[4,5-b]pyridine-5-carboxylic acid was subjected to chiral separation to obtain the product

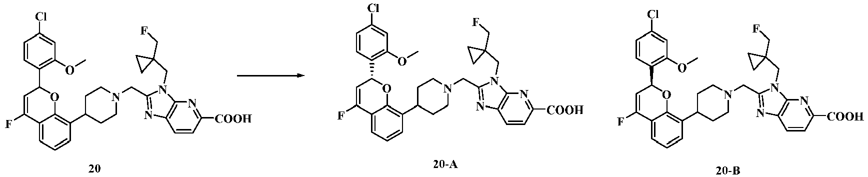

### (R)-2-((4-(2-(4-chloro-2-methoxyphenyl)-4-fluoro-2H-chromen-8-yl)piperidin-1-yl)methyl)-3-(1-(fluoromethyl)cyclopropyl)methyl)-3H-imidazolo[4,5-b]pyridine-5-carboxylic acid (Example 20-A)

MS m/z (ESI): 635.2 [M+1].

(*S*)-2-((4-(2-(4-chloro-2-methoxyphenyl)-4-fluoro-2*H*-chromen-8-yl)piperidin-1-yl)methyl)-3-(1-(fluoromethyl)cyclopropyl)methyl)-3H-imidazolo[4, 5-b]pyridine-5-carboxylic acid (Example 20-B).

MS m/z (ESI): 635.2 [M+1].

### Example 21

### 2-((4-(2-(4-Chloro-2-methoxyphenyl)-4-fluoro-2H-chromen-8-yl)piperidin-1-yl)methyl)-1-((1-(fluoromethyl)cyclopropyl)methyl)-1H-imidazolo[4,5-b]pyridine-6-carboxylic acid

Using 4-chloro-2-methoxybenzaldehyde as the starting material and referring to Example 6, the product 2-((4-(2-(4-chloro-2-methoxyphenyl)-4-fluoro-2*H*-chromen-8-yl)piperidin-1-yl)methyl)-1-((1-(fluoromethyl)cyclopropyl)methyl)-1H-imidazolo[4,5-b]pyridine-6-carboxylic acid was obtained.

MS m/z (ESI): 635.2 [M+1].

2-((4-(2-(4-Chloro-2-methoxyphenyl)-4-fluoro-2*H*-chromen-8-yl)piperidin-1-yl)methyl)-1-((1-(fluoromethyl)cyclopropyl)methyl)-1*H*-imidazolo[4,5-b]pyridine-6-carboxylic acid was subjected to chiral separation to obtain

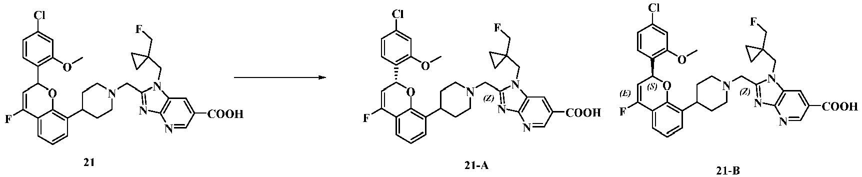

### (R)-2-((4-(2-(4-chloro-2-methoxyphenyl)-4-fluoro-2H-chromen-8-yl)piperidin-1-yl)methyl)-1-((1-(fluoromethyl)cyclopropyl)methyl)-1H-imidazolo[4,5-b]pyridine-6-carboxylic acid (Example 21-A)

MS m/z (ESI): 635.2 [M+1].

### (S)-2-((4-(2-(4-chloro-2-methoxyphenyl)-4-fluoro-2H-chromen-8-yl)piperidin-1-yl)methyl)-1-((1-(fluoromethyl)cyclopropyl)methyl)-1H-imidazolo[4,5-b]pyridine-6-carboxylic acid (Example 21-B)

MS m/z (ESI): 635.2 [M+1].

### Example 22

### 2-((4-(2-(4-Cyano-2-fluorophenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid

Using 3-fluoro-4-(4-fluoro-8-(piperidin-4-yl)-2*H*-chromen-2-yl)benzonitrile and Im-2 as the starting materials, and referring to Step 7 to Step 8 of Example 1, the product 2-((4-(2-(4-cyano-2-fluorophenyl)-4-fluoro-2*H*-chromen-8-yl)piperidin-1-yl)methyl)-3-(((*S*)-oxetan-2-yl)methyl)-3*H*-imidazolo[4,5-b]pyridine-5-carboxylic acid was obtained.

MS m/z (ESI): 598.2[M+1].

2-((4-(2-(4-Cyano-2-fluorophenyl)-4-fluoro-2*H*-chromen-8-yl)piperidin-1-yl)methyl)-3-(((*S*)-oxetan-2-yl)methyl)-3*H*-imidazolo[4,5-b]pyridine-5-carboxylic acid was subjected to chiral separation to obtain

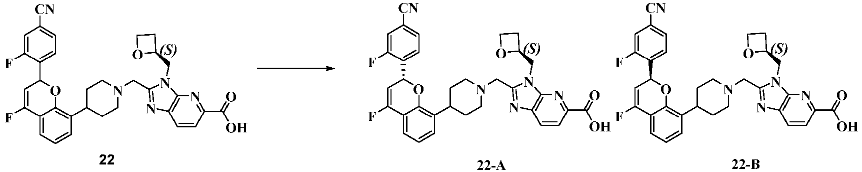

### 2-((4-((R)-2-(4-cyano-2-fluorophenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid (Example 22-A)

MS m/z (ESI): 598.2 [M+1].

### 2-((4-((S)-2-(4-cyano-2-fluorophenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid (Example 22-B)

MS m/z (ESI): 598.2 [M+1].

### Example 23

### 2-((4-(2-(4-Chloro-2-(methoxy-d3)phenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid

Using 4-chloro-2-(methoxy-d3)benzaldehyde as the starting material and referring to Example 2, the product 2-((4-(2-(4-chloro-2-(methoxy-d3)phenyl)-4-fluoro-2*H-*chromen-8-yl)piperidin-1-yl)methyl)-3-(((*S*)-oxetan-2-yl)methyl)-3*H*-imidazolo[4,5-b]pyridine-5-carboxylic acid was obtained.

MS m/z (ESI): 622.2 [M+1].

2-((4-(2-(4-Chloro-2-(methoxy-d3)phenyl)-4-fluoro-2*H*-chromen-8-yl)piperidin-1-yl)methyl)-3-(((*S*)-oxetan-2-yl)methyl)-3*H*-imidazolo[4,5-b]pyridine-5-carboxylic acid was subjected to chiral separation to obtain

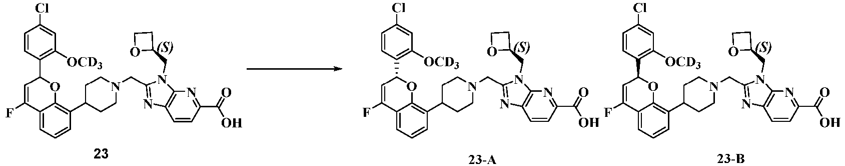

### 2-((4-((R)-2-(4-chloro-2-(methoxy-d3)phenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid (Example 23-A)

MS m/z (ESI): 622.2 [M+1].

### 2-((4-((S)-2-(4-chloro-2-(methoxy-d3)phenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid (Example 23-B)

MS m/z (ESI): 622.2 [M+1].

### Example 24

### 2-((4-(2-(4-Chloro-2-(methoxy-d3)phenyl)-4-fluoro-2H-chromen-8-yl)piperidin-1 -yl)methyl)-1-(((S)-oxetan-2-yl)methyl)-1H-benzo[d]imidazole-6-carboxylic acid

Using 4-chloro-2-(methoxy-d3)benzaldehyde as the starting material and referring to Example 1, the product 2-((4-(2-(4-chloro-2-(methoxy-d3)phenyl)-4-fluoro-2*H-*chromen-8-yl)piperidin-1-yl)methyl)-1-(((*S*)-oxetan-2-yl)methyl)-1*H-*benzo[d]imidazole-6-carboxylic acid was obtained.

MS m/z (ESI): 621.2[M+1].

2-((4-(2-(4-Chloro-2-(methoxy-d3)phenyl)-4-fluoro-2*H*-chromen-8-yl)piperidin-1 -yl)methyl)-1-(((*S*)-oxetan-2-yl)methyl)-1*H*-benzo[d]imidazole-6-carboxylic acid was subjected to chiral separation to obtain

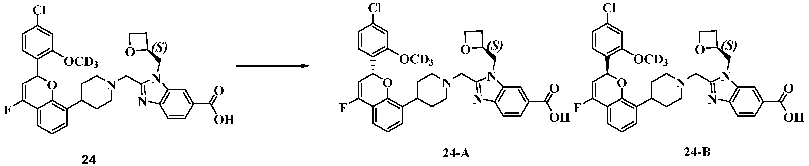

### 2-((4-((R)-2-(4-chloro-2-(methoxy-d3)phenyl)-4-fluoro-2H-chromen-8-yl)piperidin-1-yl)methyl)-1-(((S)-oxetan-2-yl)methyl)-1H-benzo[d]imidazole-6-carboxylic acid (Example 24-A)

MS m/z (ESI): 621.2 [M+1].

### 2-((4-((S)-2-(4-chloro-2-(methoxy-d3)phenyl)-4-fluoro-2H-chromen-8-yl)piperidin-1-yl)methyl)-1-(((S)-oxetan-2-yl)methyl)-1H-benzo[d]imidazole-6-carboxylic acid (Example 24-B)

MS m/z (ESI): 621.2 [M+1].

### Example 25

### 2-((4-(2-(4-Chloro-2-fluorophenyl)-4-fluoro-2H-chromen-8-yl)piperazin-1-yl)methyl)-1-(((S)-oxetan-2-yl)methyl)-1H-benzo[d]imidazole-6-carboxylic acid

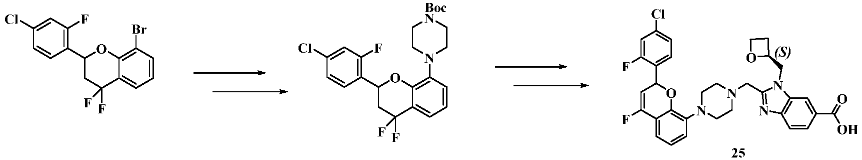

### Step 1

### Tert-butyl 4-(2-(4-chloro-2-fluorophenyl)-4,4-difluorochroman-8-yl)piperazine-1-carboxylate

To a mixture solution of 8-bromo-2-(4-chloro-2-fluorophenyl)-4,4-difluorochromane (1 g, 2.65 mmol), tert-butyl piperazine-1-carboxylate (740 mg, 3.97 mmol), cesium carbonate (2.59 g, 7.95 mmol), and toluene (30 mL) was added tris(dibenzylideneacetone)dipalladium (238 mg, 0.26 mmol) and 4,5-bis(diphenylphosphino)-9,9-dimethylxanthene (300 mg, 0.52 mmol). The reaction system was purged with nitrogen gas, and then stirred at 80°C for 12 hours. After the reaction was completed, the reaction solution was cooled and filtered. The filtrate was concentrated to dryness under reduced pressure. The resulting residue was purified by silica gel column chromatography with petroleum ether and ethyl acetate as eluent system to obtain tert-butyl 4-(2-(4-chloro-2-fluorophenyl)-4,4-difluorochroman-8-yl)piperazine-1-carboxylate (750 mg) with a yield of 58.6%.

MS m/z (ESI): 483.1 [M+1].

### Step 2

### 2-((4-(2-(4-Chloro-2-fluorophenyl)-4-fluoro-2H-chromen-8-yl)piperazin-1-yl)methyl)-1-(((S)-oxetan-2-yl)methyl)-1H-benzo[d]imidazole-6-carboxylic acid

Using tert-butyl 4-(2-(4-chloro-2-fluorophenyl)-4,4-difluorochroman-8-yl)piperazine-1-carboxylate as the starting material, and referring to Step 6 to Step 8 of Example, the product 2-((4-(2-(4-chloro-2-fluorophenyl)-4-fluoro-2*H*-chromen-8-yl)piperazine-1-yl)methyl)-1-(((*S*)-oxetan-2-yl)methyl)-1*H*-benzo[d]imidazole-6-carboxylic acid was obtained.

MS m/z (ESI): 607.1[M+1].

2-((4-(2-(4-Chloro-2-fluorophenyl)-4-fluoro-2*H*-chromen-8-yl)piperazin-1-yl)methyl)-1-(((*S*)-oxetan-2-yl)methyl)-1*H*-benzo[d]imidazole-6-carboxylic acid was subjected to chiral separation to obtain

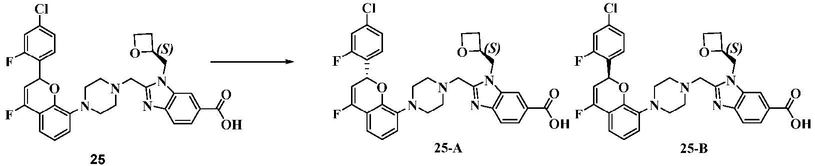

### 2-((4-((R)-2-(4-chloro-2-fluorophenyl)-4-fluoro-2H-chromen-8-yl)piperazin-1-yl)methyl)-1-(((S)-oxetan-2-yl)methyl)-1H-benzo[d]imidazole-6-carboxylic acid (Example 25-A)

MS m/z (ESI): 607.1 [M+1].

### 2-((4-((S)-2-(4-chloro-2-fluorophenyl)-4-fluoro-2H-chromen-8-yl)piperazin-1-yl)methyl)-1-(((S)-oxetan-2-yl)methyl)-1H-benzo[d]imidazole-6-carboxylic acid (Example 25-B)

MS m/z (ESI): 607.1 [M+1].

### Example 26

### 2-((4-(2-(4-Chloro-2-fluorophenyl)-2H-benzo[b][1,4]oxazin-8-yl)piperidin-1-yl)methyl)-1-(((S)-oxetan-2-yl)methyl)-1H-benzo[d]imidazole-6-carboxylic acid

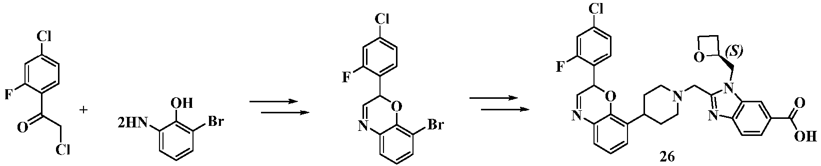

### Step 1

### 8-Bromo-2-(4-chloro-2-fluorophenyl)-2H-benzo[b][1,4]oxazine

2-Chloro-1-(4-chloro-2-fluorophenyl)ethan-1-one (1 g, 4.83 mmol) and 2-amino-6-bromophenol (908 mg, 4.83 mmol) were dissolved in THF (30 mL). The reaction system was purged with nitrogen gas, and then stirred under microwave at 60°C for 2 hours. After the reaction was completed, the reaction solution was cooled and filtered. The filtrate was concentrated to dryness under reduced pressure. The resulting residue was purified by silica gel column chromatography with petroleum ether and ethyl acetate as eluent system to obtain 8-bromo-2-(4-chloro-2-fluorophenyl)-2*H-*benzo[b][1,4]oxazine (810 mg) with a yield of 49.2%.

MS m/z (ESI): 340.1 [M+1].

### Step 2

### 2-((4-(2-(4-Chloro-2-fluorophenyl)-2H-benzo[b][1,4]oxazin-8-yl)piperidin-1-yl)methyl)-1-(((S)-oxetan-2-yl)methyl)-1H-benzo[d]imidazole-6-carboxylic acid

Using 8-bromo-2-(4-chloro-2-fluorophenyl)-2*H*-benzo[b][1,4]oxazine as the starting material, and referring to Step 4 to Step 8 of Example 1, the product 2-((4-(2-(4-chloro-2-fluorophenyl)-2*H*-benzo[b][1,4]oxazin-8-yl)piperidin-1-yl)methyl)-1-(((*S*)-oxetan-2-yl)methyl)-1*H*-benzo[d]imidazole-6-carboxylic acid was obtained..

MS m/z (ESI): 589.2[M+1].

2-((4-(2-(4-Chloro-2-fluorophenyl)-2*H*-benzo[b][1,4]oxazin-8-yl)piperidin-1-yl)methyl)-1-(((*S*)-oxetan-2-yl)methyl)-1*H*-benzo[d]imidazole-6-carboxylic acid was subjected to chiral separation to obtain

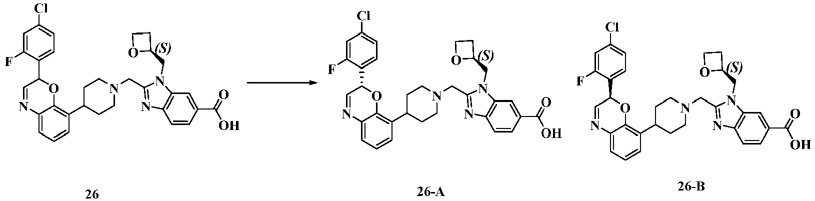

### 2-((4-((S)-2-(4-chloro-2-fluorophenyl)-2H-benzo[b][1,4]oxazin-8-yl)piperidin-1-yl)methyl)-1-(((S)-oxetan-2-yl)methyl)-1H-benzo[d]imidazole-6-carboxylic acid (Example 26-A)

MS m/z (ESI): 589.2 [M+1].

### 2-((4-((R)-2-(4-chloro-2-fluorophenyl)-2H-benzo[b][1,4]oxazin-8-yl)piperidin-1-yl)methyl)-1-(((S)-oxetan-2-yl)methyl)-1H-benzo[d]imidazole-6-carboxylic acid (Example 26-B)

MS m/z (ESI): 589.2 [M+1].

### Example 27

### 2-(((2S)-4-(2-(4-chloro-2-fluorophenyl)-4-fluoro-2H-chromen-8-yl)-2-methylpiperazin-1-yl)methyl)-1-(((S)-oxetan-2-yl)methyl)-1H-benzo[d]imidazole-6-carboxylic acid

Using 8-bromo-2-(4-chloro-2-fluorophenyl)-4,4-difluorochromane and tert-butyl (*S*)-2-methylpiperazine-1-carboxylate as the starting materials, and referring to Example 25, the product 2-(((2S)-4-(2-(4-chloro-2-fluorophenyl)-4-fluoro-2*H-*chromen-8-)-2-methylpiperazine-1-yl)methyl)-1-(((*S*)-oxetan-2-yl)methyl)-1*H-*benzo[d]imidazole-6-carboxylic acid was obtained.

MS m/z (ESI): 621.2 [M+1]

2-(((2S)-4-(2-(4-Chloro-2-fluorophenyl)-4-fluoro-2*H*-chromen-8-yl)-2-methylpiperazin-1-yl)methyl)-1-(((*S*)-oxetan-2-yl)methyl)-1*H*-benzo[d]imidazole-6-carboxylic acid was subjected to chiral separation to obtain

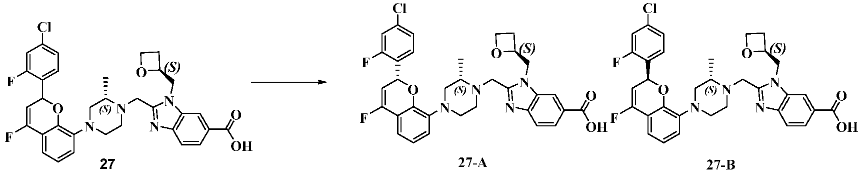

### 2-(((S)-4-((R)-2-(4-chloro-2-fluorophenyl)-4-fluoro-2H-chromen-8-yl)-2-methylpiperazin-1-yl)methyl)-1-(((S)-oxetan-2-yl)methyl)-1H-benzo[d]imidazole-6-carboxylic acid (Example 27-A)

MS m/z (ESI): 621.2 [M+1].

### 2-(((S)-4-((S)-2-(4-chloro-2-fluorophenyl)-4-fluoro-2H-chromen-8-yl)-2-methylpiperazin-1-yl)methyl)-1-(((S)-oxetan-2-yl)methyl)-1H-benzo[d]imidazole-6-carboxylic acid (Example 27-B)

MS m/z (ESI): 621.2 [M+1].

### Example 28

### 2-((4-(2-(4-Cyano-2-fluorophenyl)-4-fluoro-2H-chromen-8-yl)piperidin-1-yl)methyl)-3-((1-(fluoromethyl)cyclopropyl)methyl)-3H-imidazolo[4,5-b]pyridine-5-carboxylic acid

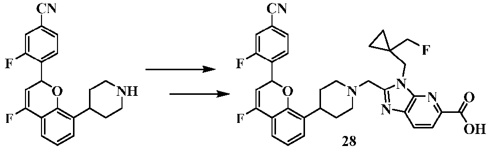

Using 3-fluoro-4-(4-fluoro-8-(piperidin-4-yl)-2*H*-chromen-2-yl)benzonitrile and Im-5 as the starting materials, and referring to Step 7 to Step 8 of Example 1, 2-((4-(2-(4-cyano-2-fluorophenyl)-4-fluoro-2*H*-chromen-8-yl)piperidin-1-yl)methyl)-3-((1-(fluoromethyl)cyclopropyl)methyl)-3*H*-imidazolo[4,5-b]pyridine-5-carboxylic acid was obtained.

MS m/z (ESI):614.2 [M+1].

2-((4-(2-(4-Cyano-2-fluorophenyl)-4-fluoro-2*H*-chromen-8-yl)piperidin-1-yl)methyl)-3-((1-(fluoromethyl)cyclopropyl)methyl)-3*H*-imidazolo[4,5-b]pyridine-5-carboxylic acid was subjected to chiral separation to obtain

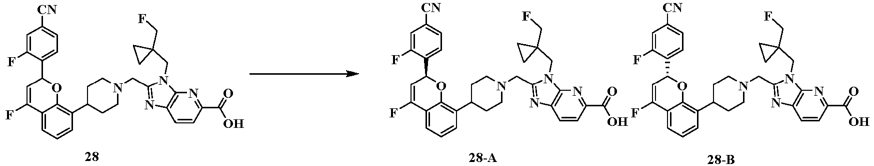

### (S)-2-((4-(2-(4-cyano-2-fluorophenyl)-4-fluoro-2H-chromen-8-yl)piperidin-1-yl)methyl)-3-((1-(fluoromethyl)cyclopropyl)methyl)-3H-imidazolo[4,5-b]pyridine-5-carboxylic acid (Example 28-A)

MS m/z (ESI):614.2 [M+1].

### (R)-2-((4-(2-(4-cyano-2-fluorophenyl)-4-fluoro-2H-chromen-8-yl)piperidin-1-yl)methyl)-3-((1-(fluoromethyl)cyclopropyl)methyl)-3H-imidazolo[4,5-b]pyridine-5-carboxylic acid (Example 28-B)

MS m/z (ESI):614.2 [M+1].

### Example 29

2-((4-(3-(4-Chloro-2-fluorophenyl)-4,4-difluorochroman-5-yl)piperidin-1-yl)methyl)-3-(((*S*)-oxetan-2-yl)methyl)-3*H*-imidazolo[4,5-b]pyridine-5-carboxylic acid

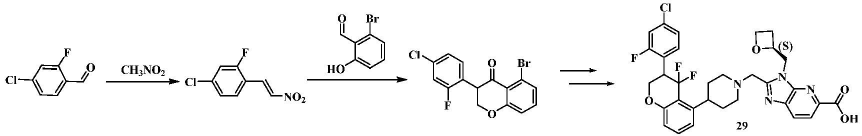

### Step 1

### (E)-4-Chloro-2-fluoro-1-(2-nitrovinyl)benzene

4-Chloro-2-fluorobenzaldehyde (4 g, 25.23 mmol) and nitromethane (14 mL) were dissolved in glacial acetic acid (50 mL), ammonium acetate (5.83 g, 75.68 mmol) was added, stirred at 110°C for 3 hours, and cooled. The reaction solution was added to ice water to precipitate a solid, and filtered. The filter cake was purified by silica gel column chromatography with petroleum ether and ethyl acetate as eluent system to obtain (E)-4-chloro-2-fluoro-1-(2-nitrovinyl)benzene (2.50 g, yield: 49.2%).

MS m/z (ESI):202.0 [M+1].

### Step 2

### 5-Bromo-3-(4-chloro-2-fluorophenyl)chroman-4-one

3-Ethyl-5-(2-hydroxyethyl)-4-methylthiazolium bromide (37.63 mg, 149.24 mmol) was added to a solution of (*E*)-4-chloro-2-fluoro-1-(2-nitrovinyl)benzene (451.26 mg, 2.24 mmol), 2-bromo-6-hydroxybenzaldehyde (300 mg, 1.49 mmol), and 4A molecular sieve (400 mg µmol) in dichloromethane (10 mL), then stirred at room temperature for 0.5 hours, and then 1,4-diazobicyclo[2.2.2]octane (33.48 mg, 298.48 µmol) was added dropwise. The reaction solution was stirred at room temperature for 12 hours under a nitrogen atmosphere, mixed with silica gel and concentrated to dryness under reduced pressure. The resulting residue was purified by silica gel column chromatography with petroleum ether and ethyl acetate as eluent system to obtain 5-bromo-3-(4-chloro-2-fluorophenyl) chromen-4-one (90 mg, yield: 16.9%)

MS m/z (ESI):355.0 [M+1].

### Step 3

### 2-((4-(3-(4-Chloro-2-fluorophenyl)-4,4-difluorochroman-5-yl)piperidin-1-yl)methyl)-3-(((S)-oxetan-2-yl)methyl)-3H-imidazolo[4,5-b]pyridine-5-carboxylic acid

Using 5-bromo-3-(4-chloro-2-fluorophenyl)chroman-4-one as the starting material, and referring to Step 3 to Step 8 of Example 1, the product 2-((4-(3-(4-chloro-2-fluorophenyl)-4,4-difluorochroman-5-yl)piperidin-1-yl)methyl)-3-(((*S*)-oxetan-2-yl)methyl)-3*H*-imidazolo[4,5-b]pyridine-5-carboxylic acid was obtained.

MS m/z (ESI): 627.2[M+1].

2-((4-(3-(4-Chloro-2-fluorophenyl)-4,4-difluorochroman-5-yl)piperidin-1-yl)methyl)-3-(((*S*)-oxetan-2-yl)methyl)-3*H*-imidazolo[4,5-b]pyridine-5-carboxylic acid was subjected to chiral separation to obtain

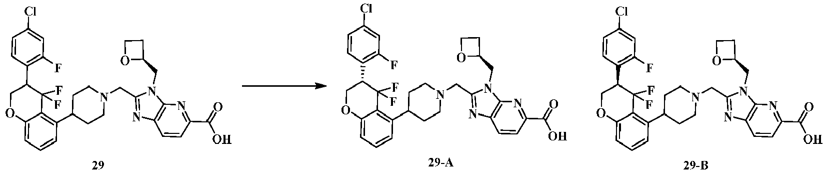

### 2-((4-((S)-3-(4-chloro-2-fluorophenyl)-4,4-difluorochroman-5-yl)piperidin-1-yl)methyl)-3-(((S)-oxetan-2-yl)methyl)-3H-imidazolo[4,5-b]pyridine-5-carboxylic acid (Example 29-A)

MS m/z (ESI): 627.2[M+1].

### 2-(4-(((R)-3-(4-chloro-2-fluorophenyl)-4,4-difluorochroman-5-yl)piperidin-1-yl)methyl)-3-(((S)-oxetan-2-yl)methyl)-3H-imidazolo[4,5-b]pyridine-5-carboxylic acid (Example 29-B)

MS m/z (ESI): 627.2[M+1].

### Example 30

### 2-((4-(3-(4-Chloro-2-fluorophenyl)chroman-5-yl)piperidin-1-yl)methyl)-3-(((S)-oxetan-2-yl)methyl)-3H-imidazolo[4,5-b]pyridine-5-carboxylic acid

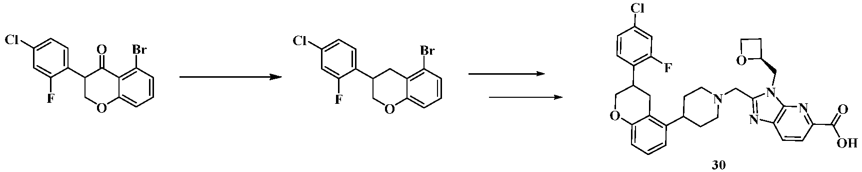

### Step 1

### 5-Bromo-3-(4-chloro-2-fluorophenyl)chromane

Triethylsilane (655 mg, 0.9 mL, 5.65 mmol) was added dropwise to a solution of 5-bromo-3-(4-chloro-2-fluorophenyl)chroman-4-one (500 mg, 1.41 mmol) in trifluoroacetic acid (5 mL), then stirred at 60°C for 8 hours, and cooled. Water was added to quench the reaction, and the reaction solution was extracted with dichloromethane (30 mL×3). The organic phase was washed with saturated sodium bicarbonate solution (50 mL×2) and saturated sodium chloride solution (50 mL×2), dried over anhydrous sodium sulfate, filtered, and the filtrate was concentrated under reduced pressure. The resulting residue was purified by silica gel column chromatography with petroleum ether and ethyl acetate as eluent system to obtain a light yellow solid 5-bromo-3-(4-chloro-2-fluorophenyl)chromane (230 mg, yield: 47.8%).

MS m/z (ESI): 340.9[M+1].

### Step 2

### 2-((4-(3-(4-Chloro-2-fluorophenyl)chroman-5-yl)piperidin-1-yl)methyl)-3-(((S)-oxetan-2-yl)methyl)-3H-imidazolo[4,5-b]pyridine-5-carboxylic acid

Using 5-bromo-3-(4-chloro-2-fluorophenyl)chromane as the starting material, and referring to Step 4 to Step 8 of Example 1, 2-((4-(3-(4-chloro-2-fluorophenyl)chroman-5-yl)piperidin-1-yl)methyl)-3-(((*S*)-oxetan-2-yl)methyl)-3*H*-imidazolo[4,5-b]pyridine-5-carboxylic acid was obtain.

MS m/z (ESI): 591.2[M+1].

2-((4-(3-(4-Chloro-2-fluorophenyl)chroman-5-yl)piperidin-1-yl)methyl)-3-(((*S*)-oxetan-2-yl)methyl)-3*H*-imidazolo[4,5-b]pyridine-5-carboxylic acid was subjected to chiral separation to obtain

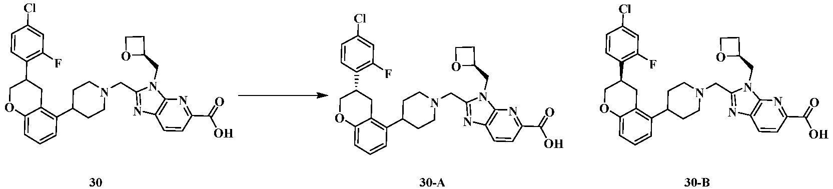

### 2-((4-((S)-3-(4-chloro-2-fluorophenyl)chroman-5-yl)piperidin-1-yl)methyl)-3-(((S)-oxetan-2-yl)methyl)-3H-imidazolo[4,5-b]pyridine-5-carboxylic acid (Example 30-A)

MS m/z (ESI): 591.2[M+1].

### 2-((4-((R)-3-(4-chloro-2-fluorophenyl)chroman-5-yl)piperidin-1-yl)methyl)-3-((((S)-oxetan-2-yl)methyl)-3H-imidazolo[4,5-b]pyridine-5-carboxylic acid (Examples 30-B)

MS m/z (ESI): 591.2[M+1].

### Example 31

### 2-((4-(3-(4-Chloro-2-fluorophenyl)-4-methyl-3,4-dihydro-2H-benzo[b][1,4]oxazin-5-yl)piperidin-1-yl)methyl)-3-(((S)-oxetan-2-yl)methyl)-3H-imidazolo[4,5-b]pyridine-5-carboxylic acid

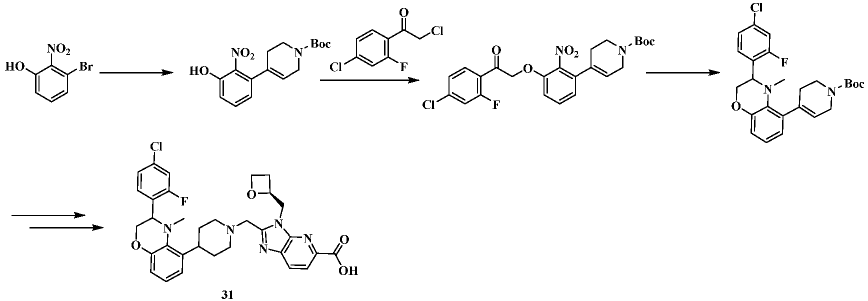

### Step 1

Tert-butyl 4-(3-hydroxy-2-nitrophenyl)-3,6-dihydropyridine-1(2H)-carboxyl ate 3-Bromo-2-nitrophenol (2 g, 9.17 mmol), tert-butyl 4-(4,4,5,5-tetramethyl-1,3,2-dioxaborolan-2-yl)-3,6-dihydro-2*H*-pyridin-1-carboxylate (2.84 g, 9.17 mmol), sodium carbonate (1.94 g, 18.34 mmol), and [1,1'-Bis(diphenylphosphino)ferrocene]palladium chloride (335.48 mg, 458.5 µmol) were dissolved in 1,4-dioxane (20 mL) and water (3 mL), and the reaction was stirred at 80°C for 6 hours. 20 mL of water was added, and the reaction solution was extracted with ethyl acetate (15 mL×3). The combined organic phases were washed with saturated sodium bicarbonate solution (5 mL×2), saturated ammonium chloride solution (5 mL×2) and saturated brine (5 mL×2) successively, dried over anhydrous sodium sulfate, filtered, and the filtrate was concentrated under reduced pressure. The resulting residue was purified by silica gel column chromatography with petroleum ether and ethyl acetate as eluent system to obtain the title product tert-butyl 4-(3-hydroxy-2-nitrophenyl)-3,6-dihydropyri dine-1(2H)-carboxylate (2.3 g, yellow solid) with a yield of 78.3%.

MS m/z (ESI):321.1 [M+1]

### Step 2

### Tert-butyl 4-(3-(2-(4-chloro-2-fluorophenyl)-2-oxoethoxy)-2-nitrophenyl)-3,6-dihydropyridine-1(2H)-carboxylate

Tert-butyl 4-(3-hydroxy-2-nitrophenyl)-3,6-dihydropyridine-1(2H)-carboxyl ate (2.30 g, 7.18 mmol), 2-chloro-1-(4-chloro-2-fluorophenyl)ethan-1-one (1.49 g, 7.18 mmol), and potassium carbonate (1.98 g, 14.36 mmol) were dissolved in toluene (20 mL), and stirred at 80°C for 2 hours. 20 mL of water was added, and the reaction solution was extracted with ethyl acetate (15 mL×3). The combined organic phases were washed with saturated sodium bicarbonate solution (5 mL×2), saturated ammonium chloride solution (5 mL×2) and saturated brine (5 mL×2) successively, dried over anhydrous sodium sulfate, filtered, and the filtrate was concentrated under reduced pressure. The resulting residue was purified by silica gel column chromatography with petroleum ether and ethyl acetate as eluent system to obtain the title product tert-butyl 4-(3-(2-(4-chloro-2-fluorophenyl)-2-oxoethoxy)-2-nitrophenyl)-3,6-dihydropyridine-1(2H)-carboxylate (3.1 g, yellow solid) with a yield of 87.9%.

MS m/z (ESI):491.1 [M+1]

### Step 3

### Tert-butyl 4-(3-(4-chloro-2-fluorophenyl)-4-methyl-3,4-dihydro-2H-benzo[b][1,4]oxazin-5-yl)-3,6-dihydropyridine-1(2H)-carboxylate

Tert-butyl 4-(3-(2-(4-chloro-2-fluorophenyl)-2-oxoethoxy)-2-nitrophenyl)-3,6-dihydropyridine-1(2H)-carboxylate (3.10 g, 6.31 mmol) and paraformaldehyde (1.13 g, 12.62 mmol) were dissolved in 30 mL of methanol and 10 mL of water, and palladium/carbon (310 mg, 10%) was added. The reaction system was purged with hydrogen gas three times, stirred for 12 hours, and filtered. The filtrate was concentrated under reduced pressure, and the resulting residue was purified by silica gel column chromatography with petroleum ether and ethyl acetate as eluent system to obtain the title product tert-butyl 4-(3-(4-chloro-2-fluorophenyl)-4-methyl-3,4-dihydro-2*H-*benzo[b][1,4]oxazin-5-yl)-3,6-dihydropyridine-1(2H)-carboxylate (1.30 g, yellow oil) with a yield of 44.9%.

MS m/z (ESI):459.1 [M+1]

### Step 4

### 2-((4-(3-(4-Chloro-2-fluorophenyl)-4-methyl-3,4-dihydro-2H-benzo[b][1,4]oxazin-5-yl)piperidin-1-yl)methyl)-3-(((S)-oxetan-2-yl)methyl)-3H-imidazolo[4,5-b]pyridine-5-carboxylic acid

Using tert-butyl 4-(3-(4-chloro-2-fluorophenyl)-4-methyl-3,4-dihydro-2*H-*benzo[b][1,4]oxazin-5-yl)-3,6-dihydropyridine-1(2H)carboxylate as the starting material, and referring to Step 5 to Step 8 of Example 1, the product 2-((4-(3-(4-chloro-2-fluorophenyl)-4-methyl-3,4-dihydro-2*H*-benzo[b][1,4]oxazin-5-yl)piperidin-1-yl)methyl)-3-(((*S*)-oxetan-2-yl)methyl)-3*H*-imidazolo[4,5-b]pyridine-5-carboxylic acid was obtain.

MS m/z (ESI):606.2 [M+1]

### 2-((4-(3-(4-Chloro-2-fluorophenyl)-4-methyl-3,4-dihydro-2H-benzo[b][1,4]oxazin-5-yl)piperidin-1-yl)methyl)-3-((S)-oxetan-2-yl)methyl)-3H-imidazolo[4,5-b]pyridine-5-carboxylic acid was subjected to chiral separation to obtain

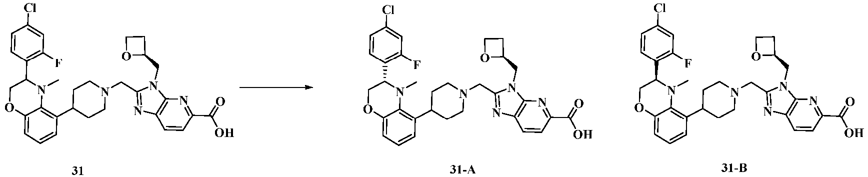

### 2-((4-((S)-3-(4-chloro-2-fluorophenyl)-4-methyl-3,4-dihydro-2H-benzo[b][1,4]oxazin-5-yl)piperidin-1-yl)methyl)-3-(((S)-oxetan-2-yl)methyl)-3H-imidazolo[4,5-b]pyridine-5-carboxylic acid (Example 31-A)

MS m/z (ESI):606.2 [M+1]

### 2-((4-((R)-3-(4-chloro-2-fluorophenyl)-4-methyl-3,4-dihydro-2H-benzo[b][1,4]oxazin-5-yl)piperidin-1-yl)methyl)-3-(((S)-oxetan-2-yl)methyl)-3H-imidazolo[4,5-b]pyridine-5-carboxylic acid (Example 31-B)

MS m/z (ESI):606.2 [M+1]

### Example 32

### 2-((4-(2-(4-Chloro-2-fluorophenyl)-4-fluoro-2H-chromen-8-yl)piperidin-1-yl)methyl)-3-(1-(cyanomethyl)cyclopropyl)methyl)-3H-imidazolo[4,5-b]pyridine-5-carboxylic acid

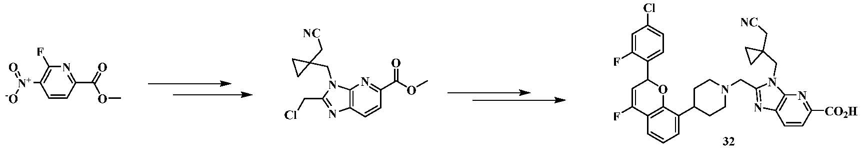

### Step 1

### Methyl 2-(chloromethyl)-3-((1-(cyanomethyl)cyclopropyl)methyl)-3H-imidazolo[4,5-b]pyridine-5-carboxylate

Using methyl 6-fluoro-5-nitropicolinate and 2-(1-(aminomethyl)cyclopropyl) acetonitrile as the starting materials, and referring to the synthesis of Im-2, the final product methyl 2-(chloromethyl)-3-((1-(cyanomethyl)cyclopropyl)methyl)-3*H-*imidazolo[4,5-b]pyridine-5-carboxylate was obtained

MS m/z (ESI): 319.0 [M+1]

### Step 2: 2-((4-(2-(4-Chloro-2-fluorophenyl)-4-fluoro-2H-chromen-8-yl)piperidin-1-yl)methyl)-3-((1-(cyanomethyl)cyclopropyl)methyl)-3H-imidazolo[4,5-b]pyridine-5-carboxylic acid

Using methyl 2-(chloromethyl)-3-((1-(cyanomethyl)cyclopropyl)methyl)-3*H-*imidazolo[4,5-b]pyridine-5-carboxylate and 4-[2-(4-chloro-2-fluorophenyl)-4-fluoro-2*H*-chromen-8-yl]piperidine as the starting materials, and referring to the synthesis of Step 7 to Step 8 of Example 1, the product 2-((4-(2-(4-chloro-2-fluorophenyl)-4-fluoro-2*H*-chromen-8-yl)piperidin-1-yl)methyl)-3-(1-(cyanomethyl)cyclopropyl)methyl)-3*H-*imidazolo[4,5-b]pyridine-5-carboxylic acid was obtained.

MS m/z (ESI): 630.2 [M+1]

2-((4-(2-(4-Chloro-2-fluorophenyl)-4-fluoro-2*H*-chromen-8-yl)piperidin-1-yl)methyl)-3-((1-(cyanomethyl)cyclopropyl)methyl)-3*H*-imidazolo[4,5-b]pyridine-5-carboxylic acid was subjected to chiral separation to obtain

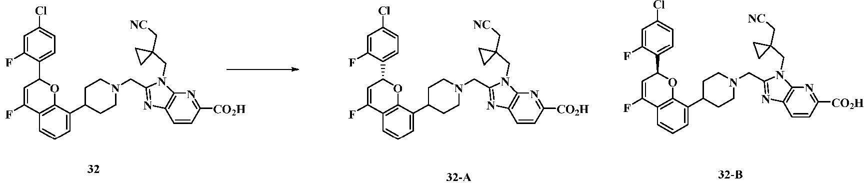

### (R)-2-((4-(2-(4-chloro-2-fluorophenyl)-4-fluoro-2H-chromen-8-yl)piperidin-1-yl)methyl)-3-((1-(cyanomethyl)cyclopropyl)methyl)-3H-imidazolo[4,5-b]pyridine-5-carboxylic acid (Example 32-A)

MS m/z (ESI): 630.2 [M+1]

### (S)-2-((4-(2-(-4-chloro-2-fluorophenyl)-4-fluoro-2H-chromen-8-yl)piperidin-1-yl)methyl)-3-((1-(cyanomethyl)cyclopropyl)methyl)-3H-imidazolo[4,5-b]pyridine-5-carboxylic acid (Example 32-B)

MS m/z (ESI): 630.2 [M+1]

### Example 33

### 2-((4-(2-(4-Chloro-2-fluorophenyl)-3-fluoro-2H-chromen-8-yl)piperidin-1-yl)methyl)-3-(((S)-oxetan-2-yl)methyl)-3H-imidazolo[4,5-b]pyridine-5-carboxylic acid

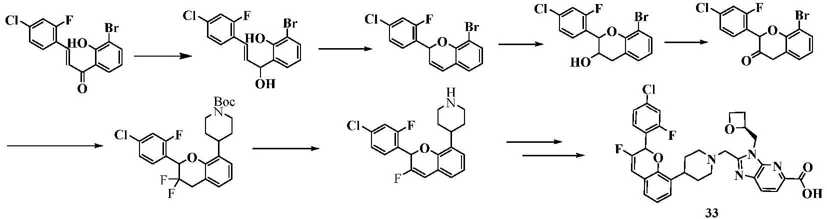

### Step 1

### (E)-2-bromo-6-(3-(4-chloro-2-fluorophenyl)-1-hydroxyallyl)phenol

(*E*)-1-(3-bromo-2-hydroxyphenyl)-3-(4-chloro-2-fluorophenyl)prop-2-en-1-one (500 mg, 1.41 mmol) was dissolved in methanol (15 mL), sodium borohydride (64 mg, 1.69 mmol) was added, and stirred at room temperature for 2 hours. Water was added to quench the reaction, and the reaction solution was extracted with dichloromethane (30 mL×3), washed with saturated sodium chloride solution (20 mL×2), dried over anhydrous sodium sulfate, filtered, and the filtrate concentrated under reduced pressure. The resulting residue was purified by silica gel column chromatography with petroleum ether and ethyl acetate as eluent system to obtain (*E*)-2-bromo-6-(3-(4-chloro-2-fluorophenyl)-1-hydroxyallyl)phenol (454 mg, yield: 90.0%).

MS m/z (ESI): 356.9 [M+1].

### Step 2

### 8-Bromo-2-(4-chloro-2-fluorophenyl)-2H-chromene

(*E*)-2-bromo-6-(3-(4-chloro-2-fluorophenyl)-1-hydroxyallyl)phenol (300 mg, 0.84 mmol) was dissolved in dichloromethane (10 mL), p-toluenesulfonic acid (29 mg, 0.17 mmol) was added, and stir at room temperature for 2 hours. Water was added to quench the reaction, and the reaction solution was extracted with dichloromethane (30 mL×3), washed with saturated sodium chloride solution (20 mL×2), dried over anhydrous sodium sulfate, filtered, and the filtrate concentrated under reduced pressure. The resulting residue was purified by silica gel column chromatography with petroleum ether and ethyl acetate as eluent system to obtain (8-bromo-2-(4-chloro-2-fluorophenyl)-2*H*-chromene (200 mg, yield: 70.1%)

MS m/z (ESI): 338.9[M+1].

### Step 3

### 8-Bromo-2-(4-chloro-2-fluorophenyl)chroman-3-ol

(8-Bromo-2-(4-chloro-2-fluorophenyl)-2*H*-chromene (200 mg, 0.59 mmol) was dissolved in THF (10 mL), a solution of borane (0.71 mL, 0.71 mmol) in THF was added, and stirred at 0°C for 1 hour. 2 mL of water was added, followed by the addition ofNaOH (71 mg, 1.77 mmol) and 0.1 mL of hydrogen peroxide solution, and stirred at 40°C for 2 hours. 10 mL of water was added to quench the reaction, and the reaction solution was extracted with dichloromethane (30 mL×3), washed with saturated sodium chloride solution (20 mL×2), dried over anhydrous sodium sulfate, filtered, and the filtrate concentrated under reduced pressure. The resulting residue was purified by silica gel column chromatography with petroleum ether and ethyl acetate as eluent system to obtain 8-bromo-2-(4-chloro-2-fluorophenyl)chroman-3-ol (63 mg, yield: 29.8%).

MS m/z (ESI): 356.9 [M+1].

### Step 4

### 8-Bromo-2-(4-chloro-2-fluorophenyl)chroman-3-one

8-Bromo-2-(4-chloro-2-fluorophenyl)chroman-3-ol (500 mg, 1.40 mmol) was dissolved in DCM (20 mL), Dess Martin oxidant (0.71 mL, 1.67 mmol) was added, and stirred at 0°C for 1 hour. 10 mL of water was added to quench the reaction, and the reaction solution was extracted with dichloromethane (30 mL×3), washed with saturated sodium chloride solution (20 mL×2), dried over anhydrous sodium sulfate, filtered, and the filtrate concentrated under reduced pressure. The resulting residue was purified by silica gel column chromatography with petroleum ether and ethyl acetate as eluent system to obtain 8-bromo-2-(4-chloro-2-fluorophenyl)chroman-3-one (448 mg, yield: 89.9%).

MS m/z (ESI): 354.9[M+1].

### Step 5

### Tert-butyl 4-(2-(4-chloro-2-fluorophenyl)-3,3-difluorochroman-8-yl)piperidine-1-carboxylate

Using 8-bromo-2-(4-chloro-2-fluorophenyl)chroman-3-one as the starting material, and referring to Step 3 to Step 5 of Example 1, the product tert-butyl 4-(2-(4-chloro-2-fluorophenyl)-3,3-difluorochroman-8-yl)piperidine-1-carboxylate was obtained.

MS m/z (ESI): 482.2 [M+1]

### Step 6

### 4-(2-(4-Chloro-2-fluorophenyl)-3-fluoro-2H-chromen-8-yl)piperidine

Using tert-butyl 4-(2-(4-chloro-2-fluorophenyl)-3,3-difluorochroman-8-yl)piperidine-1-carboxylate as the starting material, and referring to Step 6 of Example 1, the product 4-(2-(4-chloro-2-fluorophenyl)-3-fluoro-2*H*-chromen-8-yl)piperidine was obtained.

MS m/z (ESI): 362.1 [M+1]

### Step 7

### 2-((4-(2-(4-Chloro-2-fluorophenyl)-3-fluoro-2H-chromen-8-yl)piperidin-1-yl)methyl)-3-(((S)-oxetan-2-yl)methyl)-3H-imidazolo[4,5-b]pyridine-5-carboxylic acid

Using 4-(2-(4-chloro-2-fluorophenyl)-3-fluoro-2*H*-chromen-8-yl)piperidine as the starting material, and referring to Step 7 and Step 8 of Example 1, the product 2-((4-(2-(4-chloro-2-fluorophenyl)-3-fluoro-2*H*-chromen-8-yl)piperidine-1-l)methyl)-3-(((*S*)-oxetan-2-yl)methyl)-3*H*-imidazolo[4,5-b]pyridine-5-carboxylic acid was obtain.

MS m/z (ESI): 607.1 [M+1]

2-((4-(2-(4-chloro-2-fluorophenyl)-3-fluoro-2*H*-chromen-8-yl)piperidin-1-yl)methyl)-3-(((*S*)-oxetan-2-yl)methyl)-3*H*-imidazolo[4,5-b]pyridine-5-carboxylic acid was subjected to chiral separation to obtain

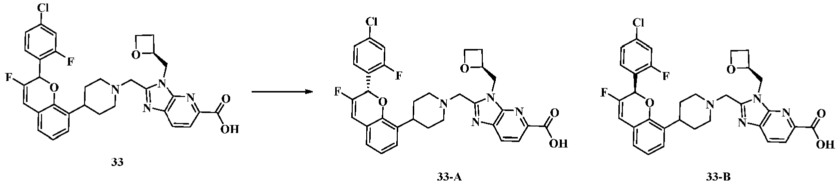

### 2-((4-((S)-2-(4-chloro-2-fluorophenyl)-3-fluoro-2H-chromen-8-yl)piperidin-1-yl)methyl)-3-(((S)-oxetan-2-yl)methyl)-3H-imidazolo[4,5-b]pyridine-5-carboxylic acid (Example 33-A)

MS m/z (ESI): 607.1 [M+1].

### 2-((4-((R)-2-(4-chloro-2-fluorophenyl)-3-fluoro-2H-chromen-8-yl)piperidin-1-yl)methyl)-3-(((S)-oxetan-2-yl)methyl)-3H-imidazolo[4,5-b]pyridine-5-carboxylic acid (Example 33-B)

MS m/z (ESI): 607.1 [M+1].

### Example 34

### 2-((4-(2-(4-Cyano-2-(methoxy-d3)phenyl)-4-fluoro-2H-chromen-8-yl)piperidin-1-yl)methyl)-1-(((S)-oxetan-2-yl)methyl)-1H-benzo[d]imidazole-6-carboxylic acid

Using 4-formyl-3-hydroxybenzonitrile as the starting material and referring to Example 24, the product 2-((4-(2-(4-cyano-2-(methoxy-d3)phenyl)-4-fluoro-2*H-*chromen-8-yl)piperidin-1-yl)methyl)-1-(((*S*)-oxetan-2-yl)methyl)-1*H-*benzo[d]imidazole-6-carboxylic acid was obtained.

MS m/z (ESI): 612.2[M+1].

### 2-((4-(2-(4-Cyano-2-(methoxy-d3)phenyl)-4-fluoro-2H-chromen-8-yl)piperidin-1-yl)methyl)-1-(((S)-oxetan-2-yl)methyl)-1H-benzo[d]imidazole-6-carboxylic acid was subjected to chiral separation to obtain

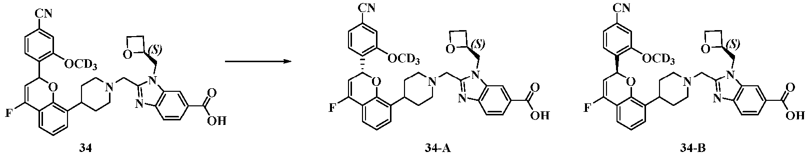

### 2-((4-((R)-2-(4-cyano-2-(methoxy-d3)phenyl)-4-fluoro-2H-chromen-8-yl)piperidin-1-yl)methyl)-1-(((S)-oxetan-2-yl)methyl)-1H-benzo[d]imidazole-6-carboxylic acid (Example 34-A)

MS m/z (ESI): 612.2 [M+1].

### 2-((4-((S)-2-(4-cyano-2-(methoxy-d3)phenyl)-4-fluoro-2H-chromen-8-yl)piperidin-1-yl)methyl)-1-(((S)-oxetan-2-yl)methyl)-1H-benzo[d]imidazole-6-carboxylic acid (Example 34-B)

MS m/z (ESI): 612.2 [M+1].

### Example 35

### 2-((4-(2-(4-Cyano-2-(methoxy-d3)phenyl)-4-fluoro-2H-chromen-8-yl)piperidin-1-yl)methyl)-3-((1-(fluoromethyl)cyclopropyl)methyl)-3H-imidazolo[4,5-b]pyridine-5-carboxylic acid

Using 4-formyl-3-hydroxybenzonitrile as the starting material and referring to Example 20, the product 2-((4-2-(4-cyano-2-(methoxy-d3)phenyl)-4-fluoro-2*H-*chromen-8-yl)piperidin-1-yl)methyl)-3-((1-(fluoromethyl)cyclopropyl)methyl)-3*H-*imidazolo[4,5-b]pyridine-5-carboxylic acid was obtained.

MS m/z (ESI): 629.2 [M+1]

2-((4-(2-(4-Cyano-2-(methoxy-d3)phenyl)-4-fluoro-2*H*-chromen-8-yl)piperidin-1-yl)methyl)-3-(1-(fluoromethyl)cyclopropyl)methyl)-3*H*-imidazolo[4,5-b]pyridine-5-carboxylic acid was subjected to chiral separation to obtain

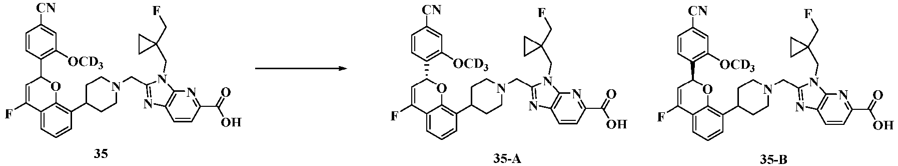

### (R)-2-((4-(2-(4-cyano-2-(methoxy-d3)phenyl)-4-fluoro-2H-chromen-8-yl)piperidin-1-yl)methyl)-3-((1-(fluoromethyl)cyclopropyl)methyl)-3H-imidazolo[4,5-b]pyridine-5-carboxylic acid (Example 35-A)

MS m/z (ESI): 629.2 [M+1].

### (S)-2-((4-(2-(4-cyano-2-(methoxy-d3)phenyl)-4-fluoro-2H-chromen-8-yl)piperidin-1-yl)methyl)-3-((1-(fluoromethyl)cyclopropyl)methyl)-3H-imidazolo[4,5-b]pyridine-5-carboxylic acid (Example 35-B)

MS m/z (ESI): 629.2 [M+1].

### Example 36

### 2-((4-((R)-2-(4-Chloro-2-(methoxy-d3)phenyl)-4-fluoro-2-methyl-2H-chromen-8-yl)piperidin-1-yl)methyl)-3-(((S)-oxetan-2-yl)methyl)-3H-imidazolor[4,5-b]pyridine-5-carboxylic acid

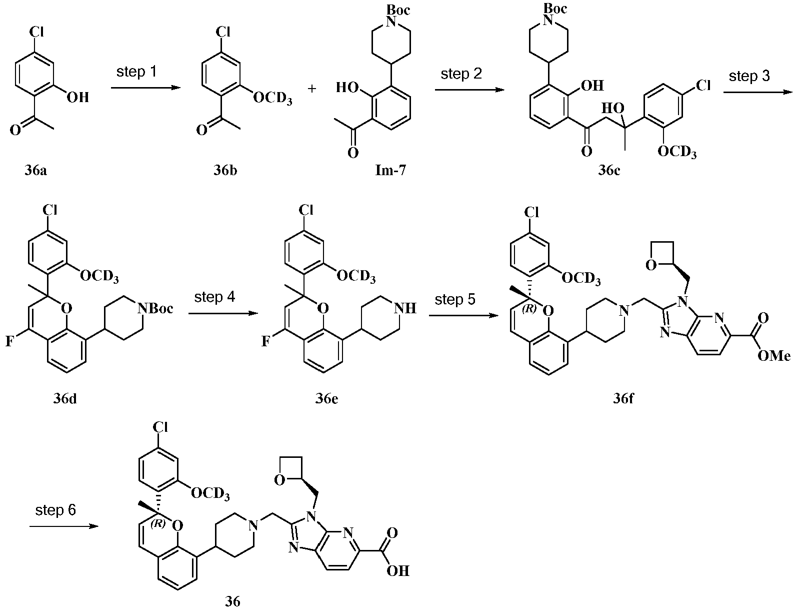

### Step 1

### 1-(4-Chloro-2-(methoxy-d3)phenyl)ethan-1-one

1-(4-Chloro-2-hydroxyphenyl)ethan-1-one **36a** (15 g, 87.93 mmol), deuterated iodomethane (16.57 g, 114.31 mmol), and anhydrous potassium carbonate (36.46 g, 0.26 mol) were dispersed in DMF (150 mL). The reaction solution was heated to 50 °C and stirred vigorously for 12 hours. The reaction was stopped, the reaction solution was filtered through diatomaceous earth, and the filtrate was concentrated under reduced pressure. The resulting residue was purified by silica gel column chromatography with petroleum ether and ethyl acetate as eluent system to obtain the title product 1-(4-chloro-2-(methoxy-d3)phenyl)ethan-1-one **36b** (15 g) with a yield of 90.9%.

MS m/z (ESI): 188.1 [M+1].

### Step 2

### Tert-butyl 4-(3-(3-(4-chloro-2-(methoxy-d3)phenyl)-3-hydroxybutanoyl)-2-hydroxyphenyl)piperidine-1-carboxylate

Tert-butyl 4-(3-acetyl-2-hydroxyphenyl)piperidine-1-carboxylate **Im-7** (25 g, 79.94 mmol) was dissolved in tetrahydrofuran (300 mL). The solution was cooled in a dry ice/ethanol bath, and LiHMDS (18.4 mL, 0.184 mol, 1 M in THF) was slowly added thereto. After the completion of addition, the mixture were stirred for 30 minutes. 1-(4-Chloro-2-(methoxy-d3)phenyl)ethan-1-one **36b** (15 g, 79.94 mmol) was dissolved in THF (30 mL), this solution was slowly added to the aforementioned reaction, and stirred for 40 minutes in the dry ice/ethanol bath. The reaction was stopped, the dry ice/ethanol bath was removed, saturated ammonium chloride solution was added to quench the reaction, and the reaction solution was extracted with ethyl acetate (300 mL×2). The combined organic phases were washed with saturated sodium chloride (500 mL), dried over anhydrous sodium sulfate, filtered, and the filtrate was concentrated under reduced pressure. The resulting residue was purified by silica gel column chromatography with petroleum ether and ethyl acetate as eluent system to obtain the title product tert-butyl 4-(3-(3-(4-chloro-2-(methoxy-d3)phenyl)-3-hydroxybutanoyl)-2-hydroxyphenyl)piperidine-1-carboxylate **36c** (31 g) with a yield of 76.5%.

MS m/z (ESI): 507.2 [M+1].

### Step 3

### Tert-butyl 4-(2-(4-chloro-2-(methoxy-d3)phenyl)-4-fluoro-2-methyl-2H-chromen-8-yl)pipendine-1-carboxylate

Tert-butyl 4-(3-(3-(4-chloro-2-(methoxy-d3)phenyl)-3-hydroxybutanoyl)-2-hydroxyphenyl)piperidine-1-carboxylate **36c** (2.00 g, 4.07 mmol) and BAST (10 mL) were dissolved in DMF (10 mL), then ethanol (20 µL) was added, and the reaction solution was stirred a for 4 hours. The reaction was stopped, the reaction solution was slowly added to ice water, and extracted with ethyl acetate (50 mL×2). The combined organic phases were washed with water (100 mL), washed with saturated sodium bicarbonate (100 mL), dried over anhydrous sodium sulfate, filtered, and the filtrate was concentrated under reduced pressure. The resulting residue was purified by silica gel column chromatography with petroleum ether and ethyl acetate as eluent system to obtain the title product tert-butyl 4-(2-(4-chloro-2-(methoxy-d3)phenyl)-4-fluoro-2-methyl-2*H*-chromen-8-yl)piperidine-1-carboxylate **36d** (750 mg) with a yield of 38.7%.

MS m/z (ESI): 491.2 [M+1].

### Step 4

### 4-(2-(4-Chloro-2-(methoxy-d3)phenyl)-4-fluoro-2-methyl-2H-chromen-8-yl)piperidine

Tert-butyl 4-(2-(4-chloro-2-(methoxy-d3)phenyl)-4-fluoro-2-methyl-2*H-*chromen-8-yl)piperidine-1-carboxylate **36d** (750 mg, 1.53 mmol), boron trifluoride etherate (543 mg, 3.83 mmol), and powdered 4 Å molecular sieve (750 mg) were dispersed in dichloromethane (10 mL), and stirred at 0 °C for 3 hours. The reaction was stopped, and saturated sodium bicarbonate solution was added to the reaction solution to quench the reaction. The mixture was filtered through diatomaceous earth, the filtrate was left to stand, the organic layer was separated, and the water layer was extracted with dichloromethane (20 mL×2). The combined organic phases were dried over anhydrous sodium sulfate, filtered, and the filtrate was concentrated under reduced pressure. The resulting residue was purified by silica gel column chromatography with dichloromethane and methanol as eluent system to obtain the title product 4-(2-(4-chloro-2-(methoxy-d3)phenyl)-4-fluoro-2-methyl-2*H*-chromen-8-yl)piperidine **36e** (490 mg) with a yield of 82.1%.

MS m/z (ESI): 391.2 [M+1].

### Step 5

### Methyl 2-((4-((R)-2-(4-chloro-2-(methoxy-d3)phenyl)-2-methyl-2H-chromen-8-vl)pipendin-1-yl)methyl)-3-(((S)-oxetan-2-yl)methyl)-3H-imidazolo[4,5-b]pyridine-5-carboxylate

4-(2-(4-Chloro-2-(methoxy-d3)phenyl)-4-fluoro-2-methyl-2*H*-chromen-8-yl)piperidine **36e** (490 mg, 1.25 mmol), methyl (*S*)-2-(chloromethyl)-3-(oxetan-2-ylmethyl)-3H-imidazolo[4,5-b]pyridine-5-carboxylate **Im-2** (371 mg, 1.25 mmol), and anhydrous potassium carbonate (518 mg, 3.75 mmol) were dispersed in acetonitrile (8 mL). The reaction solution was heated to 50°C and vigorously stirred for 3 hours. The reaction was stopped, the reaction solution was cooled to room temperature, filtered through diatomaceous earth, and the filtrate was concentrated under reduced pressure. The resulting residue was purified by silica gel column chromatography with ethyl acetate as eluent system, and then the purified resulting sample was subjected to chiral HPLC separation to obtain the title product methyl 2-((4-((*R*)-2-(4-chloro-2-(methoxy-d3)phenyl)-2-methyl-2*H*-chromen-8-yl)piperidin-1-yl)methyl)-3-(((*S*)-oxetan-2-yl)methyl)-3*H*-imidazolo[4,5-b]pyridine-5-carboxylate **36f** (210 mg) with a yield of 26.5%.

MS m/z (ESI): 632.2 [M+1].

### Step 6

### 2-((4-((R)-2-(4-Chloro-2-(methoxy-d3)phenyl)-2-methyl-2H-chromen-8-yl)piperidin-1-yl)methyl)-3-(((S)-oxetan-2-yl)methyl)-3H-imidazolo[4,5-b]pyridine-5-carboxylic acid

Methyl 2-((4-((*R*)-2-(4-chloro-2-(methoxy-d3)phenyl)-2-methyl-2*H*-chromen-8-yl)piperidin-1-yl)methyl)-3-(((*S*)-oxetan-2-yl)methyl)-3*H*-imidazolo[4,5-b]pyridine-5-carboxylate **36f** (210 mg, 332.19 µmol) and lithium hydroxide (133 mg, 3.32 mmol) were dissolved in a 9 mL mixed solvent of THF, water, and methanol (4:4:1), and stirred for 3 hours. The reaction was stopped, the pH was adjusted to 6 with formic acid, the reaction solution was concentrated under reduced pressure. The resulting residue was purified by preparative HPLC to obtain the title product 2-((4-(*R*)-2-(4-chloro-2-(methoxy-d3)phenyl)-2-methyl-2*H*-chromen-8-yl)piperidin-1-yl)methyl)-3-(((*S*)-oxetan-2-yl)methyl)-3*H*-imidazolo[4,5-b]pyridine-5-carboxylic acid **36** (170 mg), with a yield of 82.8%.

MS m/z (ESI): 618.2 [M+1].

¹H NMR (400 MHz, MeOD) δ 8.16 - 7.97 (m, 2H), 7.29 (d, J = 8.2 Hz, 1H), 7.08 - 6.98 (m, 2H), 6.94 - 6.85 (m, 2H), 6.81 (t, J = 7.5 Hz, 1H), 6.57 (d, J = 9.8 Hz, 1H), 5.78 (d, J = 9.8 Hz, 1H), 5.25 (dd, J = 7.5, 3.0 Hz, 1H), 4.96 (dd, J = 14.9, 6.7 Hz, 1H), 4.83 (dd, J = 14.9, 3.1 Hz, 1H), 4.66 - 4.53 (m, 1H), 4.48 - 4.35 (m, 1H), 4.25 (d, J = 14.2 Hz, 1H), 4.16 (d, J = 14.2 Hz, 1H), 3.25 (d, J = 11.5 Hz, 1H), 3.12 (d, J = 11.5 Hz, 1H), 2.91 (s, 1H), 2.83 - 2.70 (m, 1H), 2.61 - 2.41 (m, 3H), 1.85 (q, J = 3.9 Hz, 2H), 1.78 (s, 3H), 1.68 (d, J = 3.7 Hz, 1H), 1.59 (d, J = 14.9 Hz, 1H).

### Example 37

### 2-((4-((R)-2-(4-Chloro-2-fluorophenyl)-4-fluoro-2-methyl-2H-chromen-8-yl)piperidin-1-yl)methyl)-3-(((S)-oxetan-2-yl)methyl)-3H-imidazolo[4,5-b]pyridine-5-carboxylic acid

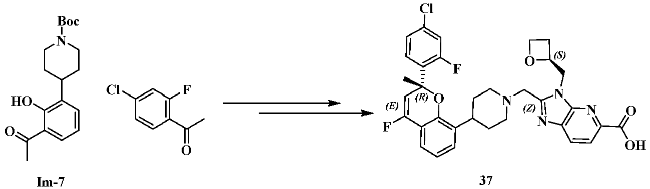

Using tert-butyl 4-(3-acetyl-2-hydroxyphenyl)piperidine-1-carboxylate **Im-7** and 1-(4-chloro-2-fluorophenyl)ethan-1-one as the starting materials, and referring to Step 2 to Step 6 of Example 36, 2-((4-(*R*)-2-(4-chloro-2-fluorophenyl)-4-fluoro-2-methyl-2*H*-chromen-8-yl)piperidin-1-yl)methyl)-3-(((*S*)-oxetan-2-yl)methyl)-3*H-*imidazolo[4,5-b]pyridine-5-carboxylic acid **37** was obtained.

MS m/z (ESI):621.2 [M+1].

### Example 38

### 2-((4-((R)-2-(4-Chloro-2-fluorophenyl)-2-methyl-2H-chromen-8-yl)piperidin-1-yl)methyl)-3-(((S)-oxetan-2-yl)methyl)-3H-imidazolo[4,5-b]pyridine-5-carboxylic acid

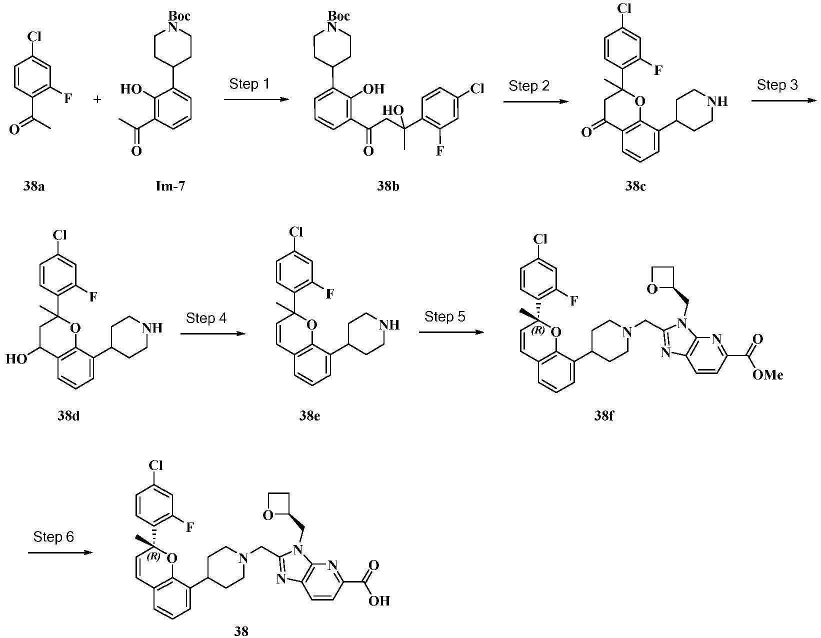

### Step 1

### Tert-butyl 4-(3-(3-4-chloro-2-fluorophenyl)-3-hydroxybutanoyl)-2-hydroxyphenyl)piperidine-1-carboxylate

Referring to the synthesis method of Example **37,** using 1-(4-chloro-2-fluorophenyl)ethan-1-one 38a (5 g, 28.97 mmol), Im-7 (9.25 g, 28.97 mmol), LiHMDS (66.6 mL, 66.63 mmol), and THF (100 mL) as the starting materials, the title product tert-butyl 4-(3 -(3 -4-chloro-2-fluorophenyl)-3 -hydroxybutanoyl)-2-hydroxyphenyl)piperidine-1-carboxylate **41b** (7.60 g) was obtained with a yield of 53.3%.

MS m/z (ESI): 492.2 [M+1].

### Step 2

### 2-(4-Chloro-2-fluorophenyl)-2-methyl-8-(piperidin-4-yl)chroman-4-one

Tert-butyl 4-(3-(3-(4-chloro-2-fluorophenyl)-3-hydroxybutanoyl)-2-hydroxyphenyl)piperidine-1-carboxylate **38b** (5 g, 10.16 mmol) and p-toluenesulfonic acid (5.25 g, 30.48 mmol) were dissolved in toluene (50 mL). The reaction solution was heated to 100 °C and stirred for 5 hours. The reaction was stopped, the reaction solution was cooled to room temperature, and 2 M sodium hydroxide solution was added thereto to quench the reaction. The organic layer was separated, and the water layer was extracted with dichloromethane (50 mL×3). The combined organic phases were dried over anhydrous sodium sulfate, filtered, and the filtrate was concentrated under reduced pressure. The resulting residue was purified by silica gel column chromatography with dichloromethane and methanol as eluent system to obtain the title product 2-(4-chloro-2-fluorophenyl)-2-methyl-8-(piperidin-4-yl)chroman-4-one **38c** (2.36 g) with a yield of 62.1%.

MS m/z (ESI): 374.1 [M+1].

### Step 3

### 2-(4-Chloro-2-fluorophenyl)-2-methyl-8-(piperidin-4-yl)chroman-4-ol

2-(4-Chloro-2-fluorophenyl)-2-methyl-8-(piperidin-4-yl)chroman-4-one **38c** (2 g, 5.35 mmol) was dissolved in methanol (30 mL). In an ice bath, sodium borohydride (396 mg, 10.70 mmol) was slowly added in batches to the solution. After the completion of addition, the ice bath was removed, and the reaction solution was naturally raised to ambient temperature for 2 hours. The reaction was stopped, saturated ammonium chloride solution was added to the reaction solution to quench the reaction, the pH= 10 with 1 M sodium hydroxide solution. The reaction solution was extracted with dichloromethane (50 mL×3). The combined organic phases were dried over anhydrous sodium sulfate, filtered, and the filtrate was concentrated under reduced pressure to obtain the title product 2-(4-chloro-2-fluorophenyl)-2-methyl-8-(piperidin-4-yl)chroman-4-ol **38d** (1.88 g) with a yield of 93.5%. The compound was not purified and was directly used for the next reaction.

MS m/z (ESI): 376.1 [M+1]

### Step 4

### 4-(2-(4-Chloro-2-fluorophenyl)-2-methyl-2H-chromen-8-yl)piperidine

2-(4-Chloro-2-fluorophenyl)-2-methyl-8-(piperidin-4-yl)chroman-4-ol **38d** (1.50 g, 3.99 mmol) and p-toluenesulfonic acid (2.06 g, 11.97 mmol) were dissolved in toluene (20 mL). The reaction solution was heated to 100 °C and stirred for 30 minutes. The reaction was stopped, the reaction solution was cooled to room temperature, 2 M sodium hydroxide solution was added thereto to quench the reaction. The organic layer was separated, and the water layer was extracted with dichloromethane (30 mL×3). The combined organic phases were dried over anhydrous sodium sulfate, filtered, and the filtrate was concentrated under reduced pressure to obtain the title product 4-(2-(4-chloro-2-fluorophenyl)-2-methyl-2*H*-chromen-8-yl)piperidine **38e** (1.02 g) with a yield of 71.4%. The compound is not purified and can be directly used for the next reaction.

MS m/z (ESI): 358.1 [M+1].

### Step 5

### Methyl 2-((4-((R)-2-(4-chloro-2-fluorophenyl)-2-methyl-2H-chromen-8-vl)piperidin-1-yl)methyl)-3-(((S)-oxetan-2-yl)methyl)-3H-imidazolo[4,5-b]pyridine-5-carboxylate

Referring to the synthesis method of Step 5 of Example **37,** using 4-(2-(4-chloro-2-fluorophenyl)-2-methyl-2*H*-chromen-8-yl)piperidine **38e** (500 mg, 1.40 mmol), methyl (*S*)-2-(chloromethyl)-3-(oxetan-2-ylmethyl)-3*H*-imidazolo[4,5-b]pyridine-5-carboxylate **Im-2** (413 mg, 1.40 mmol), anhydrous potassium carbonate (580 mg, 4.20 mmol), and acetonitrile (10 mL) as the starting materials, the title product methyl 2-((4-((*R*)-2-(4-chloro-2-fluorophenyl)-2-methyl-2*H*-chromen-8-yl)piperidin-1-yl)methyl)-3-(((*S*)-oxetan-2-yl)methyl)-3*H*-imidazolo[4,5-b]pyridine-5-carboxylate **41f** (263 mg) was obtained with a yield of 30.5%.

MS m/z (ESI): 617.2 [M+1]

### Step 6

### 2-((4-((R)-2-(4-Chloro-2-fluorophenyl)-2-methyl-2H-chromen-8-yl)piperidin-1-yl)methyl)-3-(((S)-oxetan-2-yl)methyl)-3H-imidazolo[4,5-b]pyridine-5-carboxylic acid

Referring to the synthesis method of Step 6 of Example **37,** using methyl 2-((4-((*R*)-2-(4-chloro-2-fluorophenyl)-2-methyl-2H-chromen-8-yl)piperidin-1-yl)methyl)-3-(((*S*)-oxetan-2-yl)methyl)-3*H*-imidazolo[4,5-b]pyridine-5-carboxylate **38f** (100 mg, 162 µmol) and lithium hydroxide (39 mg, 1.62 mmol) as the starting materials, the title product 2-((4-(*R*)-2-(4-chloro-2-fluorophenyl)-2-methyl-2*H*-chromen-8-yl)piperidin-1-yl)methyl)-3-(((*S*)-oxetan-2-yl)methyl)-3*H*-imidazolo[4,5-b]pyridine-5-carboxylic acid **38** (76 mg) was obtained with a yield of 77.8%.

MS m/z (ESI): 603.2 [M+1].

### Example 39

### 2-((4-((R)-2-(4-Chloro-2-(methoxy-d3)phenyl)-2-methyl-2H-chromen-8-yl)piperidin-1-yl)methyl)-3-(((S)-oxetan-2-yl)methyl)-1H-benzo[d]imidazole-6-carboxylic acid

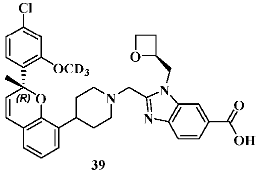

Referring to the synthesis method of Example **36,** using **Im-1** as the starting material, the title product 2-((4-(*R*)-2-(4-chloro-2-(methoxy-d3)phenyl)-2-methyl-2*H-*chromen-8-yl)piperidin-1-yl)methyl)-3-(((*S*)-oxetan-2-yl)methyl)-1*H-*benzo[d]imidazole-6-carboxylic acid was obtained.

MS m/z (ESI): 617.3 [M+1].

### Example 40

### 2-((4-((R)-2-(4-Chloro-2-fluorophenyl)-4-fluoro-2H-chromen-8-yl-2-d)piperidin-1-yl)methyl)-3-(((S)-oxetan-2-yl)methyl)-3H-imidazolo[4,5-b]pyridine-5-carboxylic acid

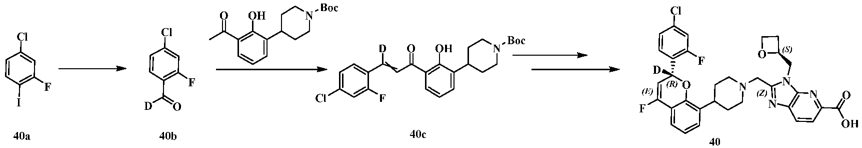

### Step 1

### 4-Chloro-2-fluoro<α-2H>benzaldehyde

4-Chloro-2-fluoro-1-iodobenzene (5 g, 19.50 mmol) and toluene (50 mL) were added to a 100 mL flask. Isopropanol magnesium chloride (12.9 mL, 3M, 38.99 mmol) was added at -30 °C, and the reaction solution was reacted at -20 °C for 2 hours. Then N,N-dimethylformamide-D7 (3.12 g, 38.99 mmol) was added to the reaction solution and reacted at 0 °C for 1 hour. The reaction was stopped, the reaction solution was quenched with saturated aqueous ammonium chloride solution, followed by the addition of water (50 mL), and extracted with ethyl acetate (20 mL×3). The organic phase was washed with saturated sodium chloride solution (20 mL), dried over anhydrous sodium sulfate, filtered, and concentrated to dryness by rotary evaporation. The resulting residue was purified by silica gel column chromatography with petroleum ether and ethyl acetate as eluent system to obtain the title product 4-chloro-2-fluoro<α-2H>benzaldehyde **40b** (2.7 g), yield: 86.79%.

MS m/z (ESI): 160.0 [M+1].

### Step 2

### Tert-butyl 4-(3-(3-(4-chloro-2-fluorophenyl)acryloyl-3-d-3-d)-2-hydroxyphenyl)piperidine-1-carboxylate

4-Chloro-2-fluoro<α-2H>benzaldehyde **40b** (1.5 g, 9.40 mmol), tert-butyl 4-(3-acetyl-2-hydroxyphenyl)piperidine-1-carboxylate **Im-7** (3.00 g, 9.40 mmol), and tetrahydrofuran (40 mL) were added to a 100 mL flask. Sodium hydride (1.13 g, 28.20 mmol, 60% purity) was added at 0 °C, and the reaction solution was reacted for 3 hours at 25 °C. The reaction was stopped, the reaction solution was quenched with saturated aqueous ammonium chloride solution, followed by the addition of water (20 mL), and extracted with ethyl acetate (20 mL×3). The organic phase was washed with saturated sodium chloride solution (20 mL), dried over anhydrous sodium sulfate, filtered, and concentrated to dryness by rotary evaporation. The resulting residue was purified by silica gel column chromatography with petroleum ether and ethyl acetate as eluent system to obtain the title product tert-butyl 4-(3-(3-(4-chloro-2-fluorophenyl)acryloyl-3-d-3-d)-2-hydroxyphenyl)piperidine-1-carboxylate **40c** (3 g) with a yield of 69.23%.

MS m/z (ESI): 461.1 [M+1].

### Step 3

### 2-((4-((R)-2-(4-Chloro-2-fluorophenyl)-4-fluoro-2H-chromen-8-yl-2-d)piperidin-1-yl)methyl)-3-(((S)-oxetan-2-yl)methyl)-3H-imidazolo[4,5-b]pyridine-5-carboxylic acid

Using tert-butyl 4-(3-(3-(4-chloro-2-fluorophenyl)acryloyl-3-d-3-d)-2-hydroxyphenyl)piperidine-1-carboxylate **40c** as the starting material, and referring to the synthesis method of Step 3 to Step 6 of Example 36, 2-((4-(*R*)-2-(4-chloro-2-fluorophenyl)-4-fluoro-2*H*-chromen-8-yl-2-d)piperidin-1-yl)methyl)-3-(((*S*)-oxetan-2-yl)methyl)-3*H*-imidazolo[4,5-b]pyridine-5-carboxylic acid 40 was obtained.

MS m/z (ESI):608.1 [M+1].

¹H NMR (400 MHz, DMSO-d6) δ 8.10 (d, 1H), 7.97 (d, 1H), 7.54 (dd, 1H), 7.41 (t, 1H), 7.29 (dd, 1H), 7.24 -7.15 (m, 2H), 6.96(t, 1H), 5.67 (d, 1H), 5.20 - 5.11 (m, 1H), 4.88 - 4.79 (m, 1H), 4.74 - 4.65 (m, 1H), 4.53 - 4.43 (m, 1H), 4.41 - 4.31 (m, 1H), 3.97 (d, 1H), 3.88 (d, 1H), 2.98 - 2.91 (m, 1H), 2.87 - 2.79 (m, 1H), 2.75 - 2.62 (m, 2H), 2.49 - 2.41 (m, 1H), 2.25 - 2.07 (m, 2H), 1.73 - 1.59 (m, 2H), 1.54 - 1.41 (m, 1H), 1.37 - 1.29 (m, 1H).

### Example 41

### 2-((4-((R)-2-(4-Chloro-2-(methoxy-d3)phenyl)-4-fluoro-2H-chromen-8-yl-2-d)piperidin-1-yl)methyl)-3-(((S)-oxetan-2-yl)methyl)-3H-imidazolo[4,5-b]pyridine-5-carboxylic acid

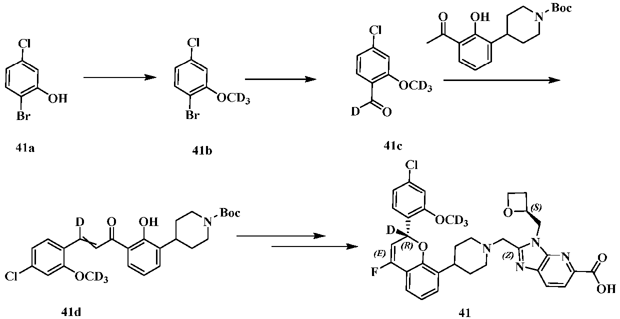

### Step 1

### 1-Bromo-4-chloro-2-(methoxy-d3)benzene

Using 2-bromo-5-chlorophenol **41a** as the starting material and referring to the synthesis method of Step 1 of Example 36, 1-bromo-4-chloro-2-(methoxy-d3)benzene **41b** was obtained.

MS m/z (ESI): 223.9 [M+1].

### Step 2

### 4-Chloro-2-(methoxy-d3)<α-2H>benzaldehyde

Using 1-bromo-4-chloro-2-(methoxy-d3)benzene **41b** as the starting material, and referring to the synthesis method of Step 1 of Example 40, 4-chloro-2-(methoxy-d3) <α-2H>benzaldehyde **41c** was obtained.

MS m/z (ESI): 175.0 [M+1].

### Step 3

### Tert-butyl 4-(3-(3-(4-chloro-2-(methoxy-d3)phenyl)acryloyl-3-d-3-d)-2-hydroxyphenyl)piperidine-1-carboxylate

Using 4-chloro-2-(methoxy-d3)<α-2H>benzaldehyde **41c** as the starting material, and referring to the synthesis method of Step 2 step of Example 40, tert-butyl 4-(3-(3-(4-chloro-2-(methoxy-d3)phenyl)acryloyl-3-d-3-d)-2-hydroxyphenyl)piperidine-1-carboxylate **41d** was obtained.

MS m/z (ESI): 476.2 [M+1].

### Step 4

### 2-((4-((R)-2-(4-Chloro-2-(methoxy-d3)phenyl)-4-fluoro-2H-chromen-8-yl-2-d)piperidin-1-yl)methyl)-3-(((S)-oxetan-2-yl)methyl)-3H-imidazolo[4,5-b]pyridine-5-carboxylic acid

Using tert-butyl 4-(3-(3-(4-chloro-2-(methoxy-d3)phenyl)acryloyl-3-d-3-d)-2-hydroxyphenyl)piperidine-1-carboxylate **41d** as the starting material, and referring to the synthesis method of Step 3 to Step 6 of Example 36, 2-((4-(*R*)-2-(4-chloro-2-(methoxy-d3)phenyl)-4-fluoro-2*H*-chromen-8-yl-2-d)piperidin-1-yl)methyl)-3-(((*S*)-oxetan-2-yl)methyl)-3*H*-imidazolo[4,5-b]pyridine-5-carboxylic acid **41** was obtained.

MS m/z (ESI): 623.2 [M+1].

¹H NMR (400 MHz, Methanol-d4) δ 8.14 - 8.04 (m, 2H), 7.31 (d, 1H), 7.20 - 7.13 (m, 2H), 7.05 (d, 1H), 6.96 - 6.87 (m, 2H), 5.36 (d, 1H), 5.29 - 5.24 (m, 1H), 5.03 - 4.93 (m, 1H), 4.82 - 4.75 (m, 1H), 4.65 - 4.55 (m, 1H), 4.45 - 4.36 (m, 1H), 4.20 (d, 1H), 4.10 (d, 1H), 3.23 - 3.15 (m, 1H), 3.09 - 3.02 (m, 1H), 2.91 - 2.86 (m, 1H), 2.81 - 2.72 (m, 1H), 2.57 - 2.37 (m, 3H), 1.88 - 1.78 (m, 2H), 1.71 - 1.63 (m, 1H), 1.59 - 1.52 (m, 1H).

### Example 42

2-((4-((*R*)-2-(4-Chloro-2-fluorophenyl)-2*H*-chromen-8-yl-2-d)piperidin-1-yl)methyl)-3-(((*S*)-oxetan-2-yl)methyl)-3*H*-imidazolo[4,5-b]pyridine-5-carboxylic acid

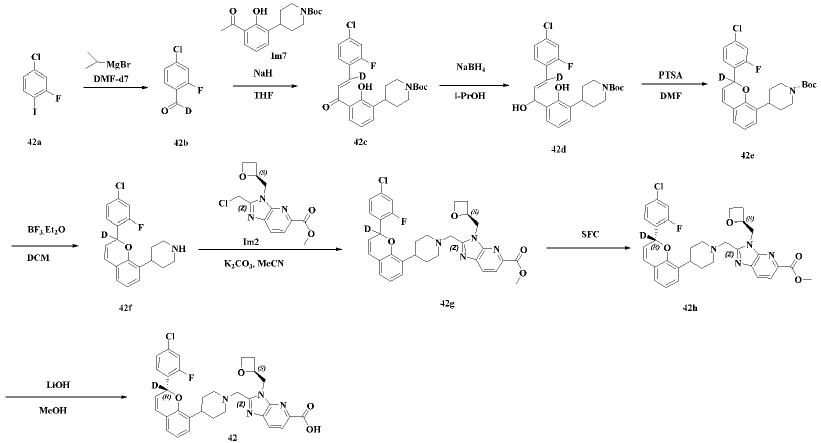

### Step 1

### 4-Chloro-2-fluoro<α-2H>benzaldehyde

4-Chloro-2-fluoro-1-iodobenzene **42a** (5 g, 19.50 mmol) and toluene (50 mL) were added to a 100 mL flask. Isopropanol magnesium chloride (12.9 mL, 3M, 38.99 mmol) was added at -30 °C, and the reaction solution was reacted at -20 °C for 2 hours. Then N,N-dimethylformamide-D7 (3.12 g, 38.99 mmol) was added to the reaction solution and reacted at 0 °C for 1 hour. The reaction was stopped, the reaction solution was quenched with saturated aqueous ammonium chloride solution, followed by the addition of water (50 mL), and extracted with ethyl acetate (20 mL×3). The organic phase was washed with saturated sodium chloride solution (20 mL), dried over anhydrous sodium sulfate, filtered, and concentrated to dryness by rotary evaporation. The resulting residue was purified by silica gel column chromatography with petroleum ether and ethyl acetate as eluent system to obtain the title product 4-chloro-2-fluoro<α-2H>benzaldehyde **42b** (2.7 g), yield: 86.79%.

MS m/z (ESI): 160.0 [M+1].

### Step 2

### Tert-butyl 4-(3-(3-(4-chloro-2-fluorophenyl)acryloyl-3-d-3-d)-2-hydroxyphenyl)piperidine-1-carboxylate

4-Chloro-2-fluoro<α-2H>benzaldehyde **42b** (1.5 g, 9.40 mmol), tert-butyl 4-(3-acetyl-2-hydroxyphenyl)piperidine-1-carboxylate **Im-7** (3.00 g, 9.40 mmol), and tetrahydrofuran (40 mL) were added to a 100 mL flask. Sodium hydride (1.13 g, 28.20 mmol, 60% purity) was added at 0 °C, and the reaction solution was reacted for 3 hours at 25 °C. The reaction was stopped, the reaction solution was quenched with saturated aqueous ammonium chloride solution, followed by the addition of water (20 mL), and extracted with ethyl acetate (20 mL×3). The organic phase was washed with saturated sodium chloride solution (20 mL), dried over anhydrous sodium sulfate, filtered, and concentrated to dryness by rotary evaporation. The resulting residue was purified by silica gel column chromatography with petroleum ether and ethyl acetate as eluent system to obtain the title product tert-butyl 4-(3-(3-(4-chloro-2-fluorophenyl)acryloyl-3-d-3-d)-2-hydroxyphenyl)piperidine-1-carboxylate **42c** (3 g) with a yield of 69.23%.

MS m/z (ESI): 461.1 [M+1].

### Step 3

### Tert-butyl 4-(3-(3-(4-chloro-2-fluorophenyl)-1-hydroxyallyl-3-d)-2-hydroxyphenyl)piperidine-1-carboxylate

Tert-butyl 4-(3-(3-(4-chloro-2-fluorophenyl)acryloyl-3-d-3-d)-2-hydroxyphenyl)piperidine-1-carboxylate **42c** (3 g, 6.51 mmol) and isopropanol (50 mL) were added to a 250 mL flask, and sodium borohydride (369.33 mg, 9.76 mmol) was added at 0 °C. Then the reaction solution was reacted at 20 °C for 3 hours. The reaction was stopped, the reaction solution was quenched with water (10 mL), and extracted with ethyl acetate (20 mL×3). The organic phase was washed with saturated sodium chloride solution (20 mL), dried over anhydrous sodium sulfate, filtered, and concentrated to dryness by rotary evaporation to obtain the title product tert-butyl 4-(3-(3-(4-chloro-2-fluorophenyl)-1-hydroxyallyl-3-d)-2-hydroxyphenyl)piperidine-1-carboxylate **42d** (3 g), with a yield of 99.56%.

MS m/z (ESI): 463.1 [M+1].

### Step 4

### Tert-butyl 4-(2-(4-chloro-2-fluorophenyl)-2H-chromen-8-yl-2-d)piperidine-1-carboxylate

Tert-butyl 4-(3-(3-(4-chloro-2-fluorophenyl)-1-hydroxyallyl-3-d)-2-hydroxyphenyl)piperidine-1-carboxylate **42d** (3 g, 6.48 mmol) and N,N-dimethylformamide (30 mL) were added to a 100 mL flask, and p-toluenesulfonic acid (1.67 g, 9.72 mmol) was added at 25 °C. Then the reaction solution was reacted at 50 °C for 4 hours. The reaction was stopped, the reaction solution was quenched with water (20 mL), and extracted with ethyl acetate (20 mL×3). The organic phase was washed with saturated sodium chloride solution (20 mL), dried over anhydrous sodium sulfate, filtered, and concentrated to dryness by rotary evaporation. The resulting residue was purified by silica gel column chromatography with petroleum ether and ethyl acetate as eluent system to obtain the title product tert-butyl 4-(2-(4-chloro-2-fluorophenyl)-2*H-*chromen-8-yl-2-d)piperidine-1-carboxylate **42e** (1.7 g) with a yield of 58.96%.

MS m/z (ESI): 445.1 [M+1].

### Step 5

### 4-(2-(4-Chloro-2-fluorophenyl)-2H-chromen-8-yl-2-d)piperidine

Tert-butyl 4-(2-(4-chloro-2-fluorophenyl)-2*H*-chromen-8-yl-2-d)piperidine-1-carboxylate **42e** (1.7 g, 3.82 mmol) and dichloromethane (25 mL) were added to a 100mL flask. 4A molecular sieve (1.7 g) and boron trifluoride etherate (1.36 g, 9.55 mmol) were added at 0 °C. Then the reaction solution was reacted at 0 °C for 2 hours. The reaction was stopped, the reaction solution was quenched with saturated sodium bicarbonate aqueous solution (10 mL), followed by the addition of water (10 mL), and extracted with dichloromethane (20 mL×3). The organic phase was washed with saturated sodium chloride solution (20 mL), dried over anhydrous sodium sulfate, filtered, and concentrated to dryness by rotary evaporation to obtain the title product 4-(2-(4-chloro-2-fluorophenyl)-2*H*-chromen-8-yl-2-d)piperidine **42f** (1.3 g) with a yield of 98.67%.

MS m/z (ESI): 345.1 [M+1]

### Step 6

### Methyl 2-((4-(-(-2-(4-chloro-2-fluorophenyl)-2H-chromen-8-yl-2-d)piperidin-1-yl)methyl)-3-(((S)-oxetan-2-yl)methyl)-3H-imidazolo[4,5-b]pyridine-5-carboxylate

4-(2-(4-chloro-2-fluorophenyl)-2*H*-chromen-8-yl-2-d)piperidine **42f** (1.3 g, 3.77 mmol) and acetonitrile (15 mL) were added to a 100 mL flask. Potassium carbonate (1.04 g, 7.54 mmol) and methyl (*S*)-2-(chloromethyl)-3-(oxetan-2-ylmethyl)-3*H-*imidazolo[4,5-b]pyridine-5-carboxylate **Im2** (1.11 g, 3.77 mmol) were added at 25 °C. Then the reaction solution was reacted at 25 °C for 10 hours. The reaction was stopped, the reaction solution was quenched with water (10 mL), and extracted with dichloromethane (10 mL×3). The organic phase was washed with saturated sodium chloride solution (10 mL), dried over anhydrous sodium sulfate, filtered, and concentrated to dryness by rotary evaporation. The resulting residue was purified by silica gel column chromatography with petroleum ether and ethyl acetate as eluent system to obtain the title product methyl 2-((4-(-(-2-(4-chloro-2-fluorophenyl)-2*H-*chromen-8-yl-2-d)piperidin-1-yl)methyl)-3-(((*S*)-oxetan-2-yl)methyl)-3*H-*imidazolo[4,5-b]pyridine-5-carboxylate **42g** (1.7 g,) with a yield of 74.65%.

MS m/z (ESI): 604.2 [M+1]

### Step 7

### Methyl 2-((4-((R)-2-(4-chloro-2-fluorophenyl)-2H-chromen-8-yl-2-d)piperidin-1-yl)methyl)-3-(((S)-oxetan-2-yl)methyl)-3H-imidazolo[4,5-b]pyridine-5-carboxylate

Methyl 2-((4-(-(-2-(4-chloro-2-fluorophenyl)-2*H*-chromen-8-2-yl-2-d)piperidin-1-yl)methyl)-3-(((*S*)-oxetan-2-yl)methyl)-3*H*-imidazolo[4,5-b] pyridin-5-carboxylate **42g** was subjected to chiral separation to obtain methyl 2-((4-((*R*)-2-(4-chloro-2-fluorophenyl)-2*H*-chromen-8-yl-2-d)piperidin-1-yl)methyl)-3-(((*S*)-oxetan-2-yl)methyl)-3*H*-imidazolo[4,5-b]pyridine-5-carboxylate **42h.**
Chiral separation condition: D-H 4.6 * 250
Hexane: EtOH: MeOH: DEA=70:15:15:0.1%
F=1mL T=35 °C

### Step 8

### 2-((4-((R)-2-(4-Chloro-2-fluorophenyl)-2H-chromen-8-yl-2-d)piperidin-1-yl)methyl)-3-(((S)-oxetan-2-yl)methyl)-3H-imidazolo[4,5-b]pyridine-5-carboxylic acid

Methyl 2-((4-((R)-2-(4-chloro-2-fluorophenyl)-2*H*-chromen-8-yl-2-d)piperidin-1-yl)methyl)-3-(((*S*)-oxetan-2-yl)methyl)-3*H*-imidazolo[4,5-b]pyridine-5-carboxylate **42h** (100 mg, 165.54 µmol) and methanol (2 mL) were added to a 25mL flask, and lithium hydroxide (39.64 mg, 1.66 mmol) was dissolved in water (1 mL) and added dropwise to the reaction solution at 25 °C. Then the reaction solution was reacted at 25 °C for 1 hour. The reaction was stopped, the reaction solution was quenched with formic acid (0.1 mL), followed by the addition of water (2 mL), and extracted with dichloromethane (2 mL×3). The organic phase was washed with saturated sodium chloride solution (2 mL), dried over anhydrous sodium sulfate, filtered, and concentrated to dryness by rotary evaporation. The resulting residue was purified by prep-HPLC to obtain the title product 2-((4-(*R*)-2-(4-chloro-2-fluorophenyl)-2*H-*chromen-8-2-yl-2-d)piperidin-1-yl)methyl)-3-(((*S*)-oxetan-2-yl)methyl)-3*H-*imidazolo[4,5-b]pyridine-5-carboxylic acid **42** (70 mg), with a yield of 71.66%.

MS m/z (ESI): 590.2 [M+1]

¹H NMR (400 MHz, DMSO) δ 8.08 (d, 1H), 7.96 (d, 1H), 7.52 (dd, 1H), 7.34 (t, 1H), 7.27 (dd, 1H), 7.05 (dd, 1H), 6.98 (dd, 1H), 6.85 (t, 1H), 6.75 (d, 1H), 5.91 (d, 1H), 5.18 - 5.11 (m, 1H), 4.87 - 4.80 (m, 1H), 4.72 - 4.65 (m, 1H), 4.53 - 4.43 (m, 1H), 4.39 - 4.31 (m, 1H), 3.96 (d, 1H), 3.87 (d, 1H), 3.01 - 2.90 (m, 1H), 2.89 - 2.79 (m, 1H), 2.77 - 2.61 (m, 2H), 2.48 - 2.45 (m, 1H), 2.27 - 2.08 (m, 2H), 1.73 - 1.61 (m, 2H), 1.54 - 1.42 (m, 1H), 1.42 - 1.34 (m, 1H).

### Example 43

### 2-((4-((R)-2-(4-Chloro-2-(methoxy-d3)phenyl)-2H-chromen-8-yl-2-d)piperidin-1-yl)methyl)-3-(((S)-oxetan-2-yl)methyl)-3H-imidazolo[4,5-b]pyridine-5-carboxylic acid

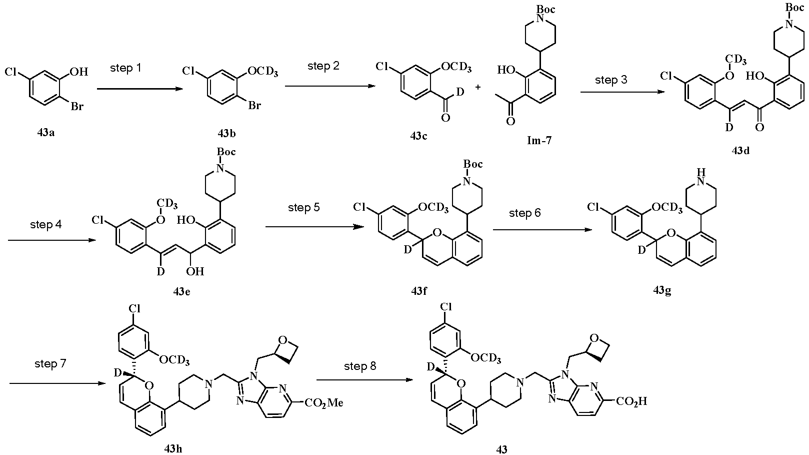

### Step 1

### 1-Bromo-4-chloro-2-(methoxy-d3)benzene

2-Bromo-5-chlorophenol **43a** (10 g, 48.20 mmol), deuterated iodomethane (10.48 g, 72.30 mmol), and anhydrous potassium carbonate (13.33 g, 96.41 mol) were dispersed in DMF (100 mL). The reaction solution was heated to 50 °C and stirred vigorously for 12 hours. The reaction was stopped, the reaction solution was filtered through diatomaceous earth, and the filtrate was concentrated under reduced pressure. The resulting residue was purified by silica gel column chromatography with petroleum ether and ethyl acetate as eluent system to obtain the title product 1-bromo-4-chloro-2-(methoxy-d3)benzene **43b** (10 g) with a yield of 92.5%.

MS m/z (ESI): 223.9 [M+1].

### Step 2

### 4-Chloro-2-(methoxy-d3)benzaldehyde-d

1-Bromo-4-chloro-2-(methoxy-d3)benzene **43b** (1.6 g, 7.13 mmol) was dissolved in tetrahydrofuran (30 mL), the reaction system was purged with nitrogen gas, and n-BuLi (2.5 M, 3.42 mL) was slowly added thereto at -78°C. After the completion of addition, the reaction solution was stirred at -78°C for 1 hour, and then N,N-dimethylformamide-D7 (856.71 mg, 10.69 mmol) was added dropwise thereto. The reaction system was stirred for 3 hours and naturally raised to room temperature. 20 mL of saturated ammonium chloride solution was slowly added thereto to quench the reaction, the reaction solution was extracted with ethyl acetate (30 mL × 3), washed with saturated brine (30 mL × 2), dried over anhydrous sodium sulfate, filtered, and concentrated to obtain a white solid 4-chloro-2-(methoxy-d3)-benzaldehyde-d **43c** (1.2 g, 6.87 mmol) with a yield of 96.42%.

MS m/z (ESI): 175.0 [M+1].

### Step 3

### Tert-butyl (E)-4-(3-(3-(4-chloro-2-(methoxy-d3)phenyl)acryloyl-3-d-3-d)-2-hydroxyphenyl)piperidine-1-carboxylate

4-Chloro-2-(methoxy-d3)-benzaldehyde-d **43c** (700 mg, 4.01 mmol) and tert-butyl 4-(3-acetyl-2-hydroxyphenyl)piperidine-1-carboxylate **Im-7** (1.28 g, 4.01 mmol) were dissolved in tetrahydrofuran (40 mL), and sodium hydride (481.05 mg, 12.03 mmol, 60% purity) was added in batches in an ice water bath. The reaction system was first stirred in an ice water bath for 0.5 hours, then placed at room temperature of 20 °C and stirred for 2.5 hours. After the reaction was completed, 20 mL of water was added dropwise to quench the reaction, the reaction solution was extracted with ethyl acetate (30 mL × 3), washed with saturated brine (30 mL × 2), dried over anhydrous sodium sulfate, filtered, and concentrated to obtain the crude product. The crude product was purified by silica gel column chromatography with petroleum ether and ethyl acetate as eluent system to obtain the title product tert-butyl (*E*)-4-(3-(3-(4-chloro-2-(methoxy-d3)phenyl)acryloyl-3-d-3-d)-2-hydroxyphenyl)piperidine-1-carboxylate **43d** (1.5 g), yield: 78.6%.

MS m/z (ESI): 476.2 [M+1].

### Step 4

### Tert-butyl (E)-4-(3-(3-(4-chloro-2-(methoxy-d3)phenyl)-1-hydroxyallyl-3-d)-2-hydroxyphenyl)piperidine-1-carboxylate

Tert-butyl (*E*)-4-(3-(3-(4-chloro-2-(methoxy-d3)phenyl)acryloyl-3-d-3-d)-2-hydroxyphenyl)piperidine-1-carboxylate **43d** (1.4 g, 2.94 mmol) was dissolved in tetrahydrofuran (30 mL), and sodium borohydride (333.82 mg, 8.82 mmol) was added thereto in batches at room temperature. The reaction system was stirred at room temperature for 2 hours. After the reaction was completed, 20 mL of water was added dropwise to quench the reaction, and the reaction solution was extracted with ethyl acetate (30 mL × 3), washed with saturated brine (30 mL × 2), dried over anhydrous sodium sulfate, filtered, and concentrated to obtain the title product tert-butyl (*E*)-4-(3-(3-(4-chloro-2-(methoxy-d3)phenyl)-1-hydroxyallyl-3-d)-2-hydroxyphenyl)piperidine-1-carboxylate **43e** (1.4 g).

MS m/z (ESI): 478.2 [M+1].

### Step 5

### Tert-butyl 4-(2-(4-chloro-2-(methoxy-d3)phenyl)-2H-chromen-8-yl-2-d)piperidine-1 -carboxylate

Tert-butyl (*E*)-4-(3-(3-(4-chloro-2-(methoxy-d3)phenyl)-1-hydroxyallyl-3-d)-2-hydroxyphenyl)piperidine-1-carboxylate **43e** (1.40 g, 2.93 mmol) was dissolved in dichloromethane (20 mL), and p-toluenesulfonic acid (151.62 mg, 880.49 µmol) was added thereto. The reaction system was stirred at room temperature for 3 hours. After the reaction was completed, the reaction solution was concentrated directly to remove the solvent to obtain the crude product. The crude product was purified by silica gel column chromatography with petroleum ether and ethyl acetate as eluent system to obtain the title product tert-butyl 4-(2-(4-chloro-2-(methoxy-d3)phenyl)-2H-chromen-8-yl-2-d)piperidine-1-carboxylate **43f** (600 mg, 1.30 mmol) with a yield of 44.4%.

MS m/z (ESI): 460.2 [M+1].

### Step 6

### 4-(2-(4-Chloro-2-(methoxy-d3)phenyl)-2H-chromen-8-yl-2-d)piperidine

Tert-butyl 4-(2-(4-chloro-2-(methoxy-d3)phenyl)-2*H*-chromen-8-yl-2-d)piperidine-1-carboxylate **43f** (260 mg, 565.22 µmol) was dissolved in dichloromethane (10 mL), and 4A° molecular sieve (260 mg) and boron trifluoride etherate (240.66 mg, 1.70 mmol) were added thereto in an ice water bath. The reaction system was stirred in an ice water bath for 1 hour. After the reaction was completed, saturated sodium bicarbonate solution (10 mL) was added dropwise to quench the reaction. The reaction solution was extracted with dichloromethane (20 mL × 3), washed with saturated brine (20 mL × 2), dried over anhydrous sodium sulfate, filtered, and concentrated to obtain the crude 4-(2-(4-chloro-2-(methoxy-d3)phenyl)-2*H-*chromen-8-yl-2-d)piperidine **43g** (200 mg).

MS m/z (ESI): 360.2 [M+1].

### Step 7

### Methyl 2-((4-((R)-2-(4-chloro-2-(methoxy-d3)phenyl)-2H-chromen-8-yl-2-d)piperidin-1-yl)methyl)-3-(((S)-oxetan-2-yl)methyl)-3H-imidazolo[4,5-b]pyridine-5-carboxylate

4-(2-(4-Chloro-2-(methoxy-d3)phenyl)-2*H*-chromen-8-yl-2-d)piperidine **43g** (200 mg, 555.74 µmol, crude), methyl (*S*)-2-(chloromethyl)-3-(oxetan-2-ylmethyl)-3*H-*imidazolo[4,5-b]pyridine-5-carboxylate **Im-2** (180.17 mg, 611.31 µmol) and potassium carbonate (230.42 mg, 1.67 mmol) were dissolved in acetonitrile (10 mL). The reaction system was stirred in an oil bath at 60 °C for 4 hours. After the reaction was completed, the reaction solution was diluted with water (10 mL), extracted with ethyl acetate (20 mL × 3), washed with saturated brine (20 mL × 2), dried over anhydrous sodium sulfate, filtered, and concentrated to obtain the crude product. The crude product was purified by silica gel column chromatography with petroleum ether and ethyl acetate as eluent system. The purified resulting sample was then subjected to chiral HPLC separation to obtain the title product methyl 2-((4-(*R*)-2-(4-chloro-2-(methoxy-d3)phenyl)-2*H-*chromen-8-yl-2-d)piperidin-1-yl)methyl)-3-(((*S*)-oxetan-2-yl)methyl)-3*H-*imidazolo[4,5-b]pyridine-5-carboxylate **43h** (100 mg, 161.5 µmol,), yield: 29.1%.

MS m/z (ESI): 619.2 [M+1].

### Step 8

### 2-((4-((R)-2-(4-Chloro-2-(methoxy-d3)phenyl)-2H-chromen-8-yl-2-d)piperidin-1-yl)methyl)-3-(((S)-oxetan-2-yl)methyl)-3H-imidazolo[4,5-b]pyridine-5-carboxylic acid

methyl 2-((4-(*R*)-2-(4-chloro-2-(methoxy-d3)phenyl)-2*H*-chromen-8-yl-2-d)piperidin-1-yl)methyl)-3-(((*S*)-oxetan-2-yl)methyl)-3*H*-imidazolo[4,5-b]pyridine-5-carboxylate **43h** (100 mg, 161.5 µmol) and lithium hydroxide (38.8 mg, 1.62 mmol) were dissolved in a 9 mL mixed solvent of THF, water, and methanol (4:4:1), and stirred for 3 hours. The reaction was stopped, the pH was adjusted to 6 with formic acid, the reaction solution was concentrated under reduced pressure. The resulting residue was purified by preparative HPLC to obtain the title product 2-((4-((*R*)-2-(4-chloro-2-(methoxy-d3)phenyl)-2*H*-chromen-8-yl-2-d)piperidin-1-yl)methyl)-3-(((*S*)-oxetan-2-yl)methyl)-3*H*-imidazolo[4,5-b]pyridine-5-carboxylic acid **43** (60 mg) , with a yield of 61.5%.

MS m/z (ESI): 605.2 [M+1].

¹H NMR (400 MHz, DMSO) δ 8.08 (d, 1H), 7.96 (d, 1H), 7.22 (d, 1H), 7.14 (d, 1H), 7.02 (d, 1H), 6.99 - 6.91 (m, 2H), 6.82 (t, 1H), 6.66 (d, 1H), 5.86 (d, 1H), 5.17 - 5.11 (m, 1H), 4.86 - 4.77 (m, 1H), 4.73 - 4.65 (m, 1H), 4.51 - 4.45 (m, 1H), 4.38 - 4.30 (m, 1H), 3.97 - 3.84 (m, 2H), 2.98 - 2.91 (m, 1H), 2.87 - 2.81 (m, 1H), 2.76 - 2.64 (m, 2H), 2.47 - 2.45 (m, 1H), 2.24 - 2.09 (m, 2H), 1.70 - 1.64 (m, 2H), 1.50 - 1.38 (m, 2H).

### Example 44

### 2-((4-((S)-2-(4-Chloro-2-fluorophenyl)-3-fluoro-2H-chromen-8-yl-2-d)piperidin-1-yl)methyl)-3-(((S)-oxetan-2-yl)methyl)-3H-imidazolo[4,5-b]pyridine-5-carboxylic acid

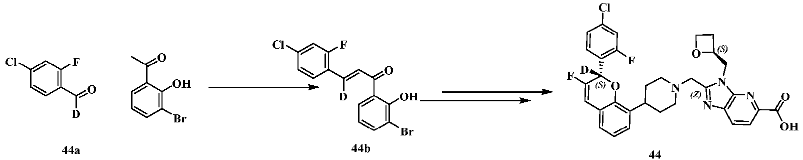

### Step 1

### 1-(3-Bromo-2-hydroxyphenyl)-3-(4-chloro-2-fluorophenyl)prop-2-en-1-one-3-d

Using 4-chloro-2-fluoro<α-2H>benzaldehyde **44a** and 1-(3-bromo-2-hydroxyphenyl)ethan-1-one as the starting materials and referring to the synthesis method of Step 1 of Example 1, 1-(3-bromo-2-hydroxyphenyl)-3-(4-chloro-2-fluorophenyl)prop-2-en-1-one-3-d **44b** was obtained.

MS m/z (ESI): 355.9 [M+1].

### Step 2

### 2-((4-((S)-2-(4-Chloro-2-fluorophenyl)-3-fluoro-2H-chromen-8-yl-2-d)piperidin-1-yl)methyl)-3-(((S)-oxetan-2-yl)methyl)-3H-imidazolo[4,5-b]pyridine-5-carboxylic acid

Using 1-(3-bromo-2-hydroxyphenyl)-3-(4-chloro-2-fluorophenyl)prop-2-en-1-one-3-d **44b** as the starting material, and referring to the synthesis method of Example 33, 2-((4-(*S*)-2-(4-chloro-2-fluorophenyl)-3-fluoro-2*H*-chromen-8-yl-2d)piperidin-1-yl)methyl)-3-(((*S*)-oxetan-2-yl)methyl)-3*H*-imidazolo[4,5-b]pyridine-5-carboxylic acid **44** was obtained.

MS m/z (ESI): 608.1 [M+1].

### Example 45

### 2-((4-((S)-2-(4-Chloro-2-(methoxy-d3)phenyl)-3-fluoro-2H-chromen-8-yl-2-d)piperidin-1-yl)methyl)-3-(((S)-oxetan-2-yl)methyl)-3H-imidazolo[4,5-b]pyridine-5-carboxylic acid

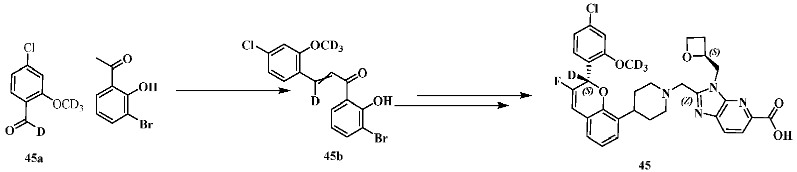

### Step 1

### 1-(3-Bromo-2-hydroxyphenyl)-3-(4-chloro-2-(methoxy-d3)phenyl)prop-2-en-1-one-3-d

Using 4-chloro-2-(methoxy-d3)<α-2H>benzaldehyde **45a** and 1-(3-bromo-2-hydroxyphenyl)ethan-1-one were used as the starting materials, and referring to the synthesis method of Step 1 of Example 1, 1-(3-bromo-2-hydroxyphenyl)-3-(4-chloro-2-(methoxy-d3)phenyl)prop-2-en-1-one-3-d **45b** was obtained.

MS m/z (ESI): 371.0 [M+1].

### Step 2

### 2-((4-((S)-2-(4-Chloro-2-(methoxy-d3)phenyl)-3-fluoro-2H-chromen-8-yl-2-d)piperidin-1-yl)methyl)-3-(((S)-oxetan-2-yl)methyl)-3H-imidazolo[4,5-b]pyridine-5-carboxylic acid

Using 1-(3-bromo-2-hydroxyphenyl)-3-(4-chloro-2-(methoxy-d3)phenyl)prop-2-en-1-one-3-d **45b** as the starting material, and referring to the synthesis method of Example 33, 2-(4-(*S*)-2-(4-chloro-2-(methoxy-d3)phenyl)-3-fluoro-2*H*-chromen-8-yl-2-d)piperidin-1-yl)methyl)-3-(((*S*)-oxetan-2-yl)methyl)-3*H*-imidazolo[4,5-b]pyridine-5-carboxylic acid **45** was obtained.

MS m/z (ESI): 623.2 [M+1].

### Example 46

### 2-((4-((R)-2-(4-Chloro-2-fluorophenyl)-4-fluoro-2H-chromen-8-yl-2-d)piperidin-1-yl)methyl)-1-(((S)-oxetan-2-yl)methyl)-1H-benzo[d]imidazole-6-carboxylic acid

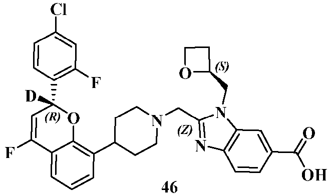

Using intermediate Im1 as the starting material, and referring to Example 40, the product 2-((4-((*R*)-2-(4-chloro-2-fluorophenyl)-4-fluoro-2*H*-chromen-8-yl-2-d)piperidin-1-yl)methyl)-1-(((*S*)-oxetan-2-yl)methyl)-1*H*-benzo[d]imidazole-6-carboxylic acid was obtained.

MS m/z (ESI): 607.2 [M+1]

### Example 47

### 2-((4-((R)-2-(4-Chloro-2-fluorophenyl)-2H-chromen-8-yl-2-d)piperidin-1-yl)methyl)-1-(((S)-oxetan-2-yl)methyl)-1H-benzo[d]imidazole-6-carboxylic acid

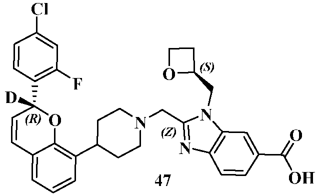

Using intermediate Im1 as the starting material, and referring to the sysntheis method of Example 42, the product 2-((4-((*R*)-2-(4-chloro-2-fluorophenyl)-2*H-*chromen-8-yl-2-d)piperidin-1-yl)methyl)-1-(((*S*)-oxetan-2-yl)methyl)-1*H-*benzo[d]imidazole-6-carboxylic acid was obtained.

MS m/z (ESI): 589.2 [M+1]

### Example 48

### 2-((4-((R)-2-(4-Chloro-2-fluorophenyl)-2H-chromen-8-yl)piperidin-1-yl)methyl)-3-(((S)-oxetan-2-yl)methyl)-3H-imidazolo[4,5-b]pyridine-5-carboxylic acid

### Method 1

Referring to the synthesis method of Example 42, the product 2-((4-((*R*)-2-(4-chloro-2-fluorophenyl)-2*H*-chromen-8-yl)piperidin-1-yl)methyl)-3-(((*S*)-oxetan-2-yl)methyl)-3*H*-imidazolo[4,5-b]pyridine-5-carboxylic acid was obtained.

### Method 2

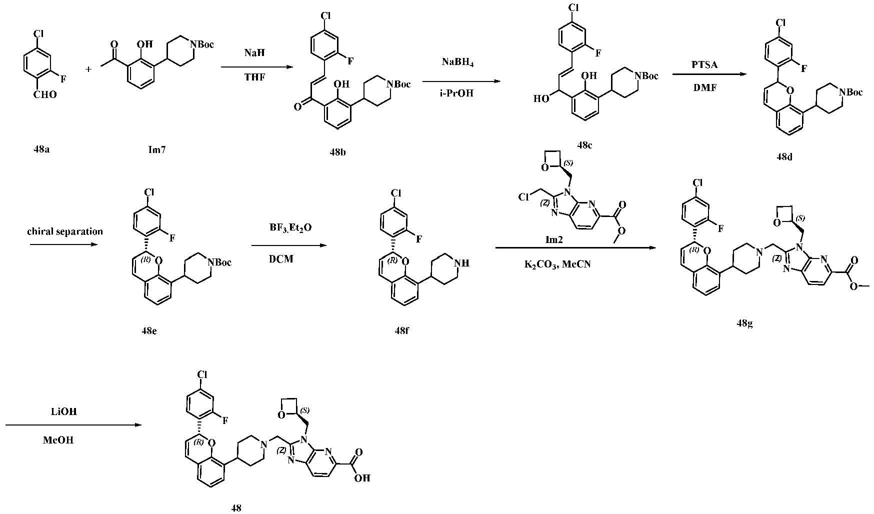

### Step 1

### Tert-butyl 4-(3-(3-(4-chloro-2-fluorophenyl)acryloyl)-2-hydroxyphenyl)piperidine-1-carboxylate

Tert-butyl 4-(3-acetyl-2-hydroxyphenyl)piperidine-1-carboxylate **Im7** (20.3 g, 63.56 mmol) and tetrahydrofuran (300 mL) were added to a 1000 mL flask, cooled at 0 °C for 10 minutes, and sodium hydride (7.63 g, 190.67 mmol, 60% purity) was added. Then, 4-chloro-2-fluorobenzaldehyde **48a** (10.08 g, 63.56 mmol) was dissolved in THF (100 mL) and slowly added dropwise into the reaction solution through a constant pressure dropping funnel for 20 minutes. After the completion of addition, the reaction solution was raised to 25 °C and reacted for 2 hours. The reaction was stopped, the reaction solution was quenched with saturated aqueous ammonium chloride solution (200 mL), followed by the addition of water (50 mL), and extracted with ethyl acetate (200 mL×3). The organic phase was washed with saturated sodium chloride solution (200 mL), dried over anhydrous sodium sulfate, filtered, concentrated to dryness by rotary evaporation, followed by the addition of ethyl acetate (60 mL), triturated (stirred for 15 minutes), and filtered. The filter cake was washed with petroleum ether (30 mL), and dried to obtain the title product tert-butyl 4-(3-(3-(4-chloro-2-fluorophenyl)acryloyl)-2-hydroxyphenyl)piperidine-1-carboxylate **48b** (15.2 g), with a yield of 52.00%.

MS m/z (ESI): 460.1 [M+1]

### Step 2

### Tert-butyl 4-(3-(3-(4-chloro-2-fluorophenyl)-1-hydroxyallyl)-2-hydroxyphenyl)piperidine-1-carboxylate

Tert-butyl 4-(3-(3-(4-chloro-2-fluorophenyl)acryloyl)-2-hydroxyphenyl)piperidine-1-carboxylate **48b** (25 g, 54.36 mmol) and isopropanol (500 mL) were added to a 1000 mL flask, and sodium borohydride (3.08 g, 81.53 mmol) was added at 0 °C. Then the reaction solution was reacted at 20 °C for 3 hours. The reaction was stopped, the reaction solution was quenched with water (100 mL), and extracted with ethyl acetate (200 mL×3). The organic phase was washed with saturated sodium chloride solution (200 mL), dried over anhydrous sodium sulfate, filtered, and concentrated to dryness by rotary evaporation to obtain the title product tert-butyl 4-(3-(3-(4-chloro-2-fluorophenyl)-1-hydroxyallyl)-2-hydroxyphenyl)piperidine-1-carboxylate **48c** (25 g) with a yield of 99.56%.

MS m/z (ESI): 462.1 [M+1].

### Step 3

### Tert-butyl 4-(2-(4-chloro-2-fluorophenyl)-2H-chromen-8-yl)piperidine-1-carboxylate

Tert-butyl 4-(3-(3-(4-chloro-2-fluorophenyl)-1-hydroxyallyl)-2-hydroxyphenyl)piperidine-1-carboxylate **48c** (25 g, 54.12 mmol) and N,N-dimethylformamide (300 mL) were added to a 500 mL flask, and p-toluenesulfonic acid (13.98 g, 81.18 mmol) was added at 25 °C. Then the reaction solution was reacted at 50 °C for 4 hours. The reaction was stopped, the reaction solution was quenched with water (200 mL), and extracted with ethyl acetate (200 mL×3). The organic phase was washed with saturated sodium chloride solution (200 mL), dried over anhydrous sodium sulfate, filtered, and concentrated to dryness by rotary evaporation. The resulting residue was purified by silica gel column chromatography with petroleum ether and ethyl acetate as eluent system to obtain the title product tert-butyl 4-(2-(4-chloro-2-fluorophenyl)-2*H*-chromen-8-yl)piperidine-1-carboxylate **48d** (13 g) with a yield of 54.11%.

MS m/z (ESI): 444.1 [M+1].

### Step 4

### Tert-butyl (R)-4-(2-(4-chloro-2-fluorophenyl)-2H-chromen-8-yl)piperidine-1-carboxylate

Tert-butyl 4 -(2-(4-chloro-2-fluorophenyl)-2*H*-chromen-8-yl)piperidine-1-carboxylate **48d** was subjected to chiral separation to obtain tert-butyl (*R*)-4-(2-(4-chloro-2-fluorophenyl)-2*H*-chromen-8-yl)piperidine-1-carboxylate **48e.**

### Chiral separation conditions

OD-H 4.6 * 150
Hexane: IPA: DEA=90: 10: 0.1%
F=1mL T=35 °C
MS m/z (ESI): 444.1 [M+1]

### Step 5

### (R)-4-(2-(4-Chloro-2-fluorophenyl)-2H-chromen-8-yl)piperidine

Tert-butyl (*R*)-4-(2-(4-chloro-2-fluorophenyl)-2*H*-chromen-8-yl)piperidine-1-carboxylate **48e** (2.55 g, 5.74 mmol) and dichloromethane (50 mL) were added to a 100mL flask. 4A molecular sieve (2.55 g) and boron trifluoride etherate (2.04 g, 14.36 mmol) were added at 0 °C. Then the reaction solution was reacted at 0 °C for 2 hours. The reaction was stopped, the reaction solution was quenched with saturated sodium bicarbonate aqueous solution (10 mL), followed by the addition of water (10 mL), and extracted with dichloromethane (20 mL×3). The organic phase was washed with saturated sodium chloride solution (20 mL), dried over anhydrous sodium sulfate, filtered, and concentrated to dryness by rotary evaporation to obtain the title product (R)-4-(2-(4-chloro-2-fluorophenyl)-2H-chromen-8-yl)piperidine **48f** (1.97 g,) with a yield of 100%.

MS m/z (ESI): 344.1 [M+1]

### Step 6

Methyl 2-((4-((*R*)-2-(4-chloro-2-fluorophenyl)-2*H*-chromen-8-yl)piperidin-1-yl)methyl)-3-(((*S*)-oxetan-2-yl)methyl)-3*H*-imidazolo[4,5-b]pyridine-5-carboxylate (*R*)-4-(2-(4-Chloro-2-fluorophenyl)-2*H*-chromen-8-yl)piperidine **48f** (1.97 g, 5.73 mmol) and acetonitrile (50 mL) were added to a 100mL flask. Potassium carbonate (1.58 g, 11.46 mmol) and methyl (*S*)-2-(chloromethyl)-3-(oxetan-2-ylmethyl)-3*H-*imidazolo[4,5-b]pyridine-5-carboxylate **Im2** (1.69 g, 5.73 mmol) were added at 25 °C. Then the reaction solution was reacted at 25 °C for 10 hours. The reaction was stopped, the reaction solution was quenched with water (20 mL), and extracted with dichloromethane (20 mL×3). The organic phase was washed with saturated sodium chloride solution (20 mL), dried over anhydrous sodium sulfate, filtered, and concentrated to dryness by rotary evaporation. The resulting residue was purified by silica gel column chromatography with petroleum ether and ethyl acetate as eluent system to obtain the title product methyl 2-((4-(*R*)-2-(4-chloro-2-fluorophenyl)-2*H-*chromen-8-yl)piperidin-1-yl)methyl)-3-(((*S*)-oxetan-2-yl)methyl)-3*H*-imidazolo[4,5-b]pyridine-5-carboxylate **48g** (3.1 g,) with a yield of 89.71%.

MS m/z (ESI): 603.2 [M+1]

### Step 7

### 2-((4-((R)-2-(4-Chloro-2-fluorophenyl)-2H-chromen-8-yl)piperidin-1-yl)methyl)-3-(((S)-oxetan-2-yl)methyl)-3H-imidazolo[4,5-b]pyridine-5-carboxylic acid

Methyl2-((4-((*R*)-2-(4-chloro-2-fluorophenyl)-2*H*-chromen-8-yl)piperidin-1-yl)methyl)-3-(((*S*)-oxetan-2-yl)methyl)-3*H*-imidazolo[4,5-b]pyridine-5-carboxylate **48g** (3.1 g, 5.14 mmol) and methanol (25 mL) were added to a 100mL flask. Lithium hydroxide (1.23 g, 51.40 mmol) was dissolved in water (10 mL) and added dropwise into the reaction solution at 25 °C. Then the reaction solution was reacted at 25 °C for 1 hour. The reaction was stopped, the reaction solution was quenched with formic acid (1 mL), followed by the addition of water (20 mL), and extracted with dichloromethane (20 mL×3). The organic phase was washed with saturated sodium chloride solution (20 mL), dried over anhydrous sodium sulfate, filtered, and concentrated to dryness by rotary evaporation. The resulting residue was purified by prep-HPLC to obtain the title product 2-((4-(*R*)-2-(4-chloro-2-fluorophenyl)-2*H*-chromen-8-yl)piperidin-1-yl)methyl)-3-(((*S*)-oxetan-2-yl)methyl)-3*H*-imidazolo[4,5-b]pyridine-5-carboxylic acid **48** (2.1 g,) with a yield of 69.35%.

MS m/z (ESI): 589.1 [M+1]

¹H NMR (400 MHz, MeOD) δ 8.14 - 8.03 (m, 2H), 7.40 (t, 1H), 7.25 (dd, 1H), 7.15 (dd, 1H), 7.05 (dd, 1H), 6.92 (dd, 1H), 6.84 (t, 1H), 6.68 (dd, 1H), 6.21 (dd, 1H), 5.82 (dd, 1H), 5.30 - 5.24 (m, 1H), 5.03 - 4.93 (m, 1H), 4.85 - 4.81 (m, 1H),4.64 - 4.54 (m, 1H), 4.45 - 4.35 (m, 1H), 4.23 (d, 1H), 4.13 (d, 1H), 3.25 - 3.18 (m, 1H), 3.12 - 3.05 (m, 1H),2.95 - 2.85 (m, 1H), 2.83 - 2.70 (m, 1H), 2.57 - 2.38 (m, 3H), 1.88 - 1.78 (m, 2H), 1.78 - 1.63 (m, 1H), 1.59 - 1.51 (m, 1H).

### Example 49

### 2-((4-((R)-2-(4-Chloro-2-(methoxy-d3)phenyl)-4-fluoro-2H-chromen-8-yl-2-d)piperidin-1-yl)methyl)-1-(((S)-oxetan-2-yl)methyl)-1H-benzo[d]imidazole-6-carboxylic acid

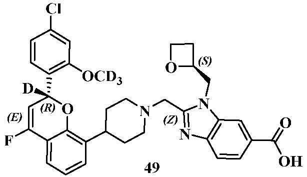

Referring to Example 41, the product 2-((4-((R)-2-(4-chloro-2-(methoxy-d3)phenyl)-4-fluoro-2*H*-chromen-8-yl-2-d)piperidin-1-yl)methyl)-1-(((*S*)-oxetan-2-yl)methyl)-1*H*-benzo[d]imidazole-6-carboxylic acid was obtained.

MS m/z (ESI): 622.2 [M+1].

### Example 50

### 2-((4-((R)-2-(4-Chloro-2-(methoxy-d3)phenyl)-2H-chromen-8-yl-2-d)piperidin-1-yl)methyl)-1-(((S)-oxetan-2-yl)methyl)-1H-benzo[d]imidazole-6-carboxylic acid

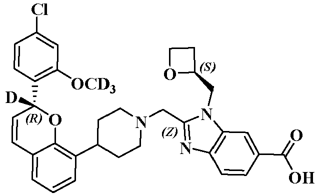

Referring to the synthsis method of Example 43, the product 2-((4-((*R*)-2-(4-chloro-2-(methoxy-d3)phenyl)-2*H*-chromen-8-yl-2-d)piperidin-1-yl)methyl)-1-(((*S*)-oxetan-2-yl)methyl)-1*H*-benzo[d]imidazole-6-carboxylic acid was obtained.

MS m/z (ESI): 604.2 [M+1].

### Example 51

### 2-((4-((R)-2-(4-Cyano-2-fluorophenyl)-2H-chromen-8-yl)piperidin-1-yl)methyl)-3-(((S)-oxetan-2-yl)methyl)-3H-imidazolo[4,5-b]pyridine-5-carboxylic acid

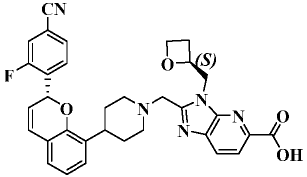

Using 4-cyano-2-fluorobenzaldehyde and intermediate Im-2 as the starting materials, and referring to Example 42, the product 2-((4-(*R*)-2-(4-cyano-2-fluorophenyl)-2*H*-chromen-8-yl)piperidin-1-yl)methyl)-3-(((*S*)-oxetan-2-yl)methyl)-3*H*-imidazolo[4,5-b]pyridine-5-carboxylic acid was obtained.

MS m/z (ESI): 580.2 [M+1].

¹H NMR (400 MHz, MeOD) δ 8.13 - 8.02 (m, 2H), 7.71 - 7.57 (m, 2H), 7.55 - 7.49 (m, 1H), 7.15 - 7.02 (m, 1H), 6.97 - 6.91 (m, 1H), 6.92 - 6.83 (m, 1H), 6.73 - 6.64 (m, 1H), 6.33 - 6.25 (m, 1H), 5.89 - 5.80 (m, 1H), 5.37 - 5.18 (m, 1H), 5.05 - 4.92 (m, 1H), 4.84 - 4.79 (m, 1H), 4.72 - 4.58 (m, 1H), 4.52 - 4.39 (m, 1H), 4.35 - 4.19 (m, 2H), 3.29 - 3.25 (m, 1H), 3.21 - 3.12 (m, 1H), 3.02 - 2.86 (m, 1H), 2.82 - 2.69 (m, 1H), 2.65 - 2.42 (m, 3H), 1.98 - 1.72 (m, 3H), 1.68 - 1.59 (m, 1H).

### Example 52

### 2-((4-((R)-2-(4-Cyano-2-fluorophenyl)-2H-chromen-8-yl)piperidin-1-yl)methyl)-1-(((S)-oxetan-2-yl)methyl)-1H-benzo[d]imidazole-6-carboxylic acid

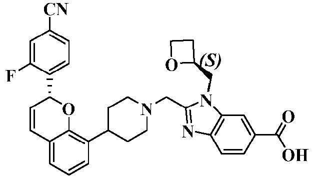

Using 4-cyano-2-fluorobenzaldehyde and intermediate Im-1 as the starting materials, and referring to Example 42, the product 2-((4-(*R*)-2-(4-cyano-2-fluorophenyl)-2*H*-chromen-8-yl)piperidin-1-yl)methyl)-1-(((*S*)-oxetan-2-yl)methyl)-1*H*-benzo[d]imidazole-6-carboxylic acid was obtained.

MS m/z (ESI): 579.2 [M+1].

¹H NMR (400 MHz, DMSO) δ 8.25 (d, 1H), 7.95 (dd, 1H), 7.79 (dd, 1H), 7.70 (dd, 1H), 7.62 (d, 1H), 7.52 (t, 1H), 7.07 (dd, 1H), 6.99 (dd, 1H), 6.87 (t, 1H), 6.75 (dd, 1H), 6.33 (dd, 1H), 5.93 (dd, 1H), 5.11 - 5.00 (m, 1H), 4.85 - 4.71 (m, 1H), 4.67 - 4.58 (m, 1H), 4.52 - 4.44 (m, 1H), 4.42 - 4.31 (m, 1H), 3.92 (d, 1H), 3.75 (d, 1H), 3.04 - 2.92 (m, 1H), 2.83 - 2.62 (m, 3H), 2.46 - 2.38 (m, 1H), 2.23 - 2.02 (m, 2H), 1.73 - 1.56 (m, 2H), 1.48 - 1.34 (m, 2H).

### Example 53

### 2-((4-((R)-2-(4-Chloro-2-fluorophenyl)-2H-chromen-8-yl)piperidin-1-yl)methyl)-3-(((S)-oxetan-2-yl)methyl)-1H-benzo[d]imidazole-6-carboxylic acid

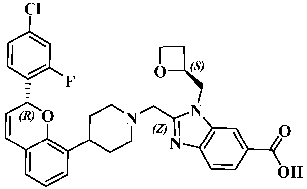

Using Im-1 as the starting material, and referring to Example 48, the product 2-((4-(*R*)-2-(4-chloro-2-fluorophenyl)-2*H*-chromen-8-yl)piperidin-1-yl)methyl)-3-(((*S*)-oxetan-2-yl)methyl)-1*H*-benzo[d]imidazole-6-carboxylic acid was obtained.

MS m/z (ESI): 588.2 [M+1].

¹H NMR (400 MHz, MeOD) δ 8.30 (s, 1H), 7.96 (dd, 1H), 7.66 (d, 1H), 7.40 (t, 1H), 7.25 (s, 1H), 7.16 (d, 1H), 7.04 (dd, 1H), 6.92 (dd, 1H), 6.83 (d, 1H), 6.68 (dd, 1H), 6.23 - 6.19 (m, 1H), 5.83 (dd, 1H), 5.27 - 5.20 (m, 1H), 4.85 - 4.80 (m, 1H), 4.74 - 4.62 (m, 2H), 4.48 - 4.41 (m, 1H), 4.05 (d, 1H), 3.95 (d, 1H), 3.12 - 3.05 (m, 1H), 2.94 - 2.77 (m, 3H), 2.55 - 2.46 (m, 1H), 2.39 - 2.25 (m, 2H), 1.82 - 1.73 (m, 2H), 1.66 - 1.50 (m, 2H).

### Example 54

### Methyl 2-((4-((R)-2-(4-chloro-2-fluorophenyl)-2H-chromen-8-yl)piperidin-1-yl)methyl)-3-(((S)-oxetan-2-yl)methyl)-3H-imidazolo[4,5-b]pyridine-5-carboxylate

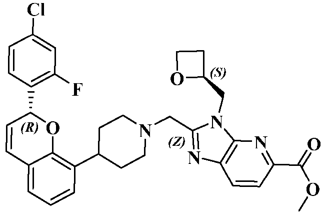

Referring to the synthesis method of Example 48, the product methyl 2-((4-((R)-2-(4-chloro-2-fluorophenyl)-2*H*-chromen-8-yl)piperidin-1-yl)methyl)-3-(((*S*)-oxetan-2-yl)methyl)-3*H*-imidazolo[4,5-b]pyridine-5-carboxylate was obtained.

MS m/z (ESI): 603.2 [M+1].

### Example 55

### 2-((4-((R)-2-(4-Chloro-2-fluorophenyl)-2H-chromen-8-yl)piperidin-1-yl)methyl)-3-(((S)-oxetan-2-yl)methyl)-3H-imidazolo[4,5-c]pyridin-6-yl)-4H-1,2,4-triazole-3-carbonitrile

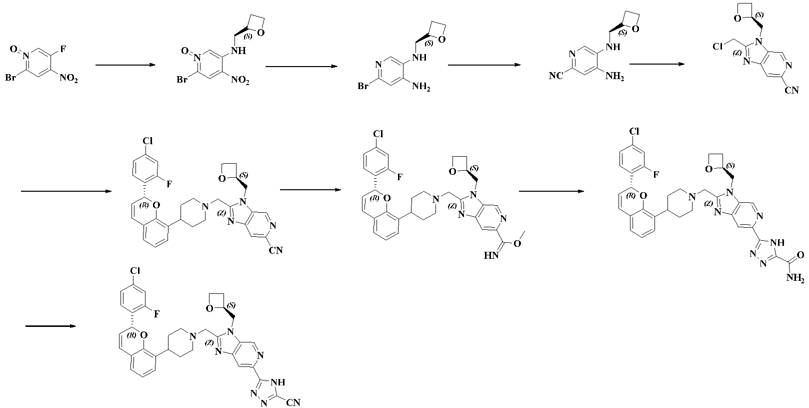

### Step 1

### (S)-2-Bromo-4-nitro-5-((oxetan-2-ylmethyl)amino)pyridine 1-oxide

Using 2-bromo-5-fluoro-4-nitropyridine N-oxide as the starting material and referring to Step 1 of intermediate Im-1, the product (*S*)-2-bromo-4-nitro-5-((oxetan-2-ylmethyl)amino)pyridine 1-oxide was obtained.

MS m/z (ESI): 303.9 [M+1]

### Step 2

### (S)-6-Bromo-N3-(oxetan-2-ylmethyl)pyridine-3,4-diamine

At room temperature, (*S*)-2-bromo-4-nitro-5-((oxetan-2-ylmethyl)amino)pyridine 1-oxide (1 g, 3.28 mmol) and Zn powder (2.1 g, 32.8 mmol) were dissolved in methanol (25 mL), then acetic acid (985 mg, 16.4 mmol) was added dropwise. The reaction solution was heated to 60 °C, stirred for 2 hours, cooled to room temperature, and filtered. The filtrate was adjusted to pH=7 with saturated sodium bicarbonate, and extracted with ethyl acetate (30 mL×3). The organic phase was dried over anhydrous sodium sulfate, filtered, and concentrated to dryness by rotary evaporation to obtain the title product (*S*)-6-bromo-N3-(oxetan-2-ylmethyl)pyridine-3,4-diamine (804 mg) with a yield of 95%.

MS m/z (ESI): 258.0 [M+1].

### Step 3

### (S)-4-Amino-5-((oxetan-2-ylmethyl)amino)picolinonitrile

At room temperature, (*S*)-6-bromo-N3-(oxetan-2-ylmethyl)pyridine-3,4-diamine (500 mg, 1.94 mmol), RuPhos Pd-G3 (324.5 mg, 0.39 mmol), X-Phos (186 mg, 0.39 mmol), and Zn(CN)₂ (458 mg, 3.9 mmol) were dissolved in NMP (15 mL), and the reaction system was purged with nitrogen gas three times. The reaction solution was heated to 130 °C, stirred for 0.5 hours, cooled to room temperature, and filtered. The filtrate was extracted with ethyl acetate (30 mL×3). The organic phase was dried over anhydrous sodium sulfate, filtered, concentrated to dryness by rotary evaporation. The resulting residue was purified by silica gel column chromatography with petroleum ether and ethyl acetate as eluent system to obtain the title product (5)-4-amino-5-((oxetan-2-ylmethyl)amino)picolinonitrile (345 mg) with a yield of 87.1%.

MS m/z (ESI): 205.1 [M+1]

### Step 4

### (S)-2-(Chloromethyl)-3-(oxetan-2-ylmethyl)-3H-imidazolo[4,5-c]pyridine-6-carbonitrile

Using (*S*)-4-amino-5-(oxetan-2-ylmethyl)amino)picolinonitrile as the starting material and referring to Step 3 of intermediate Im-2, the product (*S*)-2-(chloromethyl)-3-(oxetan-2-ylmethyl)-3*H*-imidazolo[4,5-c]pyridine-6-carbonitrile was obtained.

MS m/z (ESI): 263.0 [M+1]

### Step 5

### 2-((4-((R)-2-(4-Chloro-2-fluorophenyl)-2H-chromen-8-yl)piperidin-1-yl)methyl)-3-(((S)-oxetan-2-yl)methyl)-3H-imidazolo[4,5-c]pyridine-6-carbonitrile

Using (*S*)-2-(chloromethyl)-3-(oxetan-2-ylmethyl)-3*H*-imidazolo[4,5-c]pyridine-6-carbonitrile and (*R*)-4-(2-(4-chloro-2-fluorophenyl)-2*H*-chromen-8-yl)piperidine as the starting materials, and referring to Step 6 of Example 48, the product 2-((4-(*R*)-2-(4-chloro-2-fluorophenyl)-2*H*-chromen-8-yl)piperidin-1-yl)methyl)-3-(((S) oxetan-2-yl)methyl)-3*H*-imidazolo[4,5-c]pyridine-6-carbonitrile was obtain.

MS m/z (ESI): 570.2 [M+1].

### Step 6

### Methyl 2-((4-((R)-2-(4-chloro-2-fluorophenyl)-2H-chromen-8-yl)piperidin-1-yl)methyl)-3-(((S)-oxetan-2-yl)methyl)-3H-imidazolo[4,5-c]pyridine-6-carbimidate

At room temperature, 2-((4-((*R*)-2-(4-chloro-2-fluorophenyl)-2*H*-chromen-8-yl)piperidin-1-yl)methyl)-3-(((*S*)-oxetan-2-yl)methyl)-3*H*-imidazolo[4,5-c]pyridine-6-carbonitrile (500 mg, 0.88 mmol) was dissolved in methanol (15 mL), sodium methoxide (95 mg, 1.76 mmol) was added, and stirred at room temperature for 24 hours. 20 mL of water was added, and the reaction solution was extracted with ethyl acetate (30 mL×3). The organic phase was dried over anhydrous sodium sulfate, filtered, and concentrated to dryness by rotary evaporation to obtain the title product methyl 2-((4-((*R*)-2-(4-chloro-2-fluorophenyl)-2*H*-chromen-8-yl)piperidin-1-yl)methyl)-3-(((*S*)-oxetan-2-yl)methyl)-3*H*-imidazolo[4,5-c]pyridine-6-carbimidate (450 mg) with a yield of 85%.

MS m/z (ESI): 602.2 [M+1].

### Step 7

### 5-(2-((4-((R)-2-(4-Chloro-2-fluorophenyl)-2H-chromen-8-yl)piperidin-1-yl)methyl)-3-(((S)-oxetan-2-yl)methyl)-3H-imidazolo[4, 5-c]pyridin-6-yl)-4H-1,2,4-triazole-3-carboxamide

Methyl 2-((4-((*R*)-2-(4-chloro-2-fluorophenyl)-2*H*-chromen-8-yl)piperidin-1-yl)methyl)-3-(((*S*)-oxetan-2-yl)methyl)-3*H*-imidazolo[4,5-c]pyiridine-6-carbimidate (100 mg, 0.17 mmol) and 2-hydrazinyl-2-oxoacetamide (35 mg, 0.34 mmol) was dissolved in n-butanol (5 mL), and DIEA (66 mg, 0.51 mmol) was added. The reaction system was purged with nitrogen gas three times, and reacted at 120 °C under a nitrogen atmosphere for 16 hours. The reaction solution was cooled to room temperature, followed by the addition of 20 mL of water, and extracted with ethyl acetate (30 mL×3). The organic phase was dried over anhydrous sodium sulfate, filtered, concentrated to dryness by rotary evaporation. The resulting residue was purified by silica gel column chromatography with dichloromethane and methanol as eluent system to obtain the title product 5-(2-((4-((*R*)-2-(4-chloro-2-fluorophenyl)-2*H*-chromen-8-yl)piperidin-1-yl)methyl)-3-(((S)oxetan-2-yl)methyl)-3*H*-imidazolo[4,5-c]pyridin-6-yl)-4*H*-1,2,4-triazole-3-carboxamide (67 mg) with a yield of 60%.

MS m/z (ESI): 655.2 [M+1].

### Step 8

### 2-((4-((R)-2-(4-Chloro-2-fluorophenyl)-2H-chromen-8-yl)piperidin-1-yl)methyl)-3-(((S)-oxetan-2-yl)methyl)-3H-imidazo[4,5-c]pyridin-6-yl)-4H-1,2,4-triazole-3-carbonitrile

5-(2-((4-((*R*)-2-(4-Chloro-2-fluorophenyl)-2H-chromen-8-yl)piperidin-1-yl)methyl)-3-(((S)oxetan-2-yl)methyl)-3*H*-imidazolo[4,5-c]pyridin-6-yl)-4*H*-1,2,4-triazole-3-carboxamide (50 mg, 0.076 mmol) and DIEA (30 mg, 0.23 mmol) were dissolved in DCM, and TFAA (24mg, 0.11 mmol)was added. The reaction solution was stirred for 10 minutes, followed by the addition of 50 mL of water, and extracted with ethyl acetate (10 mL×3). The organic phase was dried over anhydrous sodium sulfate, filtered, and concentrated to dryness by rotary evaporation. The resulting residue was purified by prep-HPLC to obtain the title product 2-((4-((*R*)-2-(4-Chloro-2-fluorophenyl)-2*H*-chromen-8-yl)piperidin-1-yl)methyl)-3-(((S)-oxetan-2-yl)methyl)-3*H*-imidazo[4,5-c]pyridin-6-yl)-4*H*-1,2,4-triazole-3-carbonitrile (12 mg) with a yield of 25%.

MS m/z (ESI): 637.2 [M+1].

### Example 56

### 2-((4-((R)-2-(4-Chloro-2-fluorophenyl)-2H-chromen-8-yl)piperidin-1-yl)methyl)-3-(((S)-oxetan-2-yl)methyl)-6-(5-(trifluoromethyl)-4H-1,2,4-triazol-3-yl)-3H-imidazolo[4,5-c]pyridine

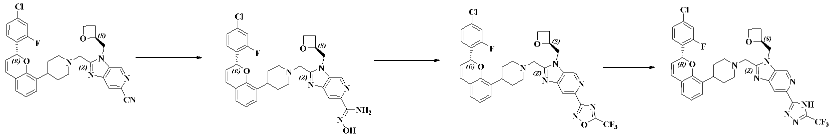

### Step 1

### (Z)-2-((4-((R)-2-(4-Chloro-2-fluorophenyl)-2H-chromen-8-yl)piperidin-1-yl)methyl)-N'-hydroxy-3-(((S)-oxetan-2-yl)methyl)-3H-imidazolo[4,5-c]pyridine-6-carboximidamide

2-((4-((*R*)-2-(4-Chloro-2-fluorophenyl)-2*H*-chromen-8-yl)piperidin-1-yl)methyl)-3-(((*S*)-oxetan-2-yl)methyl)-3*H*-imidazolo[4,5-c]pyridine-6-carbonitrile (50 mg, 0.088 mmol), DIEA (57 mg, 0.44 mmol), and hydroxylamine hydrochloride (12 mg, 0.18 mmol) were dissolved in ethanol (5 mL) and stirred at 90 °C for 2 hours. The reaction solution was filtered, and the solid was dried to obtain the title product (*Z*)-2-((4-(*R*)-2-(4-chloro-2-fluorophenyl)-2H-chromen-8-yl)piperidin-1-yl)methyl)-N'-hydroxy-3-(((*S*)-oxetan-2-yl)methyl)-3*H*-imidazolo[4,5-c]pyridine-6-carboximidamide (21.2 mg) with a yield of 40%.

MS m/z (ESI): 603.2 [M+1].

### Step 2

### 3-(2-((4-((R)-2-(4-Chloro-2-fluorophenyl)-2H-chromen-8-yl)piperidin-1-yl)methyl)-3-(((S)-oxetan-2-yl)methyl-3H-imidazolo[4,5-c]pylidin-6-yl)-5-(trifluoromethyl)-1,2,4-oxadiazole

(*Z*)-2-((4-((*R*)-2-(4-Chloro-2-fluorophenyl)-2*H*-chromen-8-yl)piperidin-1-yl)methyl)-N'-hydroxy-3-(((*S*)-oxetan-2-yl)methyl)-3*H*-imidazolo[4,5-c]pyridine-6-carboximidamide (50 mg, 0.083 mmol), and trifluoroacetic anhydride TFAA (87 mg, 0.42 mmol) were dissolved in THF (5 mL) and stirred at 60 °C for 2 hours. 10 mL of saturated sodium bicarbonate solution was added, and the reaction solution was extracted with ethyl acetate (10 mL×3). The organic phase was dried over anhydrous sodium sulfate, filtered, concentrated to dryness by rotary evaporation. The resulting residue was purified by silica gel column chromatography with dichloromethane and methanol as eluent system to obtain the title product (3-(2-((4-((*R*)-2-(4-chloro-2-fluorophenyl)-2*H*-chromen-8-yl)piperidin-1-yl)methyl)-3-(((*S*)-oxetan-2-yl)methyl-3*H*-imidazolo[4,5-c]pyridin-6-yl)-5-(trifluoromethyl)-1,2,4-oxadiazole (41.3 mg) with a yield of 73%.

MS m/z (ESI): 681.2 [M+1].

### Step 3

### 2-((4-((R)-2-(4-Chloro-2-fluorophenyl)-2H-chromen-8-yl)piperidin-1-yl)methyl)-3-(((S)-oxetan-2-yl)methyl)-6-(5-(trifluoromethyl)-4H-1,2,4-triazol-3-yl)-3H-imidazolo[4,5-c]pyridine

(3-(2-((4-(((*R*)-2-(4-Chloro-2-fluorophenyl)-2*H*-chromen-8-yl)piperidin-1-yl)methyl)-3-(((*S*)-oxetan-2-yl)methyl-3*H*-imidazolo[4,5-c]pyridin-6-yl)-5-(trifluoromethyl)-1,2,4-oxadiazole (50 mg, 0.073 mmol), and 2 drops of hydrazine hydrate were dissolved in DMF (2 mL) and stirred at room temperature for 2 hours. The reaction solution was filtered, and concentrated to dryness by rotary evaporation. The resulting residue was purified by prep-HPLC to obtain the title product 2-((4-((*R*)-2-(4-chloro-2-fluorophenyl)-2*H*-chromen-8-yl)piperidin-1-yl)methyl)-3-(((*S*)-oxetan-2-yl)methyl)-6-(5-(trifluoromethyl)-4*H*-1,2,4-triazole-3-yl)-3*H*-imidazolo[4,5-c]pyridine (21 mg) with a yield of 42%.

MS m/z (ESI): 680.2 [M+1].

### Example 57

### 3-(2-((4-((R)-2-(4-Chloro-2-fluorophenyl)-2H-chromen-8-yl)piperidin-1-yl)methyl)-3-(((S)oxetan-2-yl)methyl)-3H-imidazolo[4,5-c]pyridin-6-yl)-1,2,4-oxadiazol-5 (4H)-one

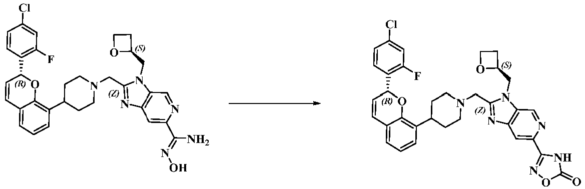

(*Z*)-2-((4-((*R*)-2-(4-Chloro-2-fluorophenyl)-2*H*-chromen-8-yl)piperidin-1-yl)methyl)-N'-hydroxy-3-(((*S*)-oxetan-2-yl)methyl)-3*H*-imidazolo[4,5-c]pyridine-6-carboximidamide (50 mg, 0.083 mmol), and CDI (27 mg, 0.166 mmol) were dissolved in DMF (2 mL), and stirred at 80 °C for 2 hours. The reaction solution was filtered, and concentrated to dryness by rotary evaporation. The resulting residue was purified by prep-HPLC to obtain the title product 3-(2-((4-(*R*)-2-(4-chloro-2-fluorophenyl)-2*H-*chromen-8-yl)piperidin-1-yl)methyl)-3-(((*S*)oxetan-2-yl)methyl)-3*H*-imidazolo[4, 5-c]pyridin-6-yl)-1,2,4-oxadiazol-5(4*H*)-one (20.9 mg) with a yield of 40%.

MS m/z (ESI): 629.2 [M+1].

### Example 58

### 2-((4-((R)-2-(4-Carbamoyl-2-fluorophenyl)-2H-chromen-8-yl)piperidin-1-yl)methyl)-3-(((S)-oxetan-2-yl)methyl)-3H-imidazolo[4,5-b]pyridine-5-carboxylic acid

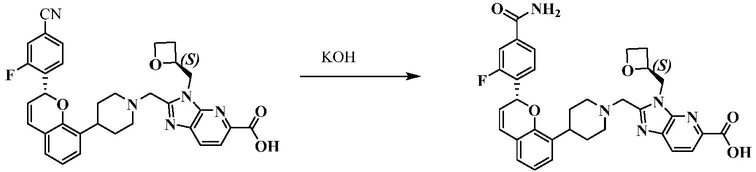

2-((4-((*R*)-2-(4-Cyano-2-fluorophenyl)-2*H*-chromen-8-yl)piperidin-1-yl)methyl)-3-(((*S*)-oxetan-2-yl)methyl)-3*H*-imidazolo[4,5-b]pyridine-5-carboxylic acid (100 mg, 172.52 µmol) and ethanol (5 mL) were added to a 25mL flask, and potassium hydroxide (96.79 mg, 1.73 mmol) was dissolved in water (1 mL) and added dropwise to the reaction solution at 25 °C. Then the reaction solution was reacted at 80 °C for 2 hours. The reaction was stopped, cooled to 25 °C, the reaction solution was quenched with formic acid (0.1 mL), followed by the addition of water (2 mL), and extracted with dichloromethane (2 mL×3). The organic phase was washed with saturated sodium chloride solution (2 mL), dried over anhydrous sodium sulfate, filtered, and concentrated to dryness by rotary evaporation. The resulting residue was purifed by prep-HPLC to obtain the title product 2-((4-((*R*)-2-(4-carbamoyl-2-fluorophenyl)-2*H-*chromen-8-yl)piperidin-1-yl)methyl)-3-(((*S*)-oxetan-2-yl)methyl)-3*H*-imidazolo[4,5-b]pyridine-5-carboxylic acid (40 mg), with a yield of 38.79%.

MS m/z (ESI): 598.2 [M+1].

### Example 59

### 2-((4-((R)-2-(4-Carbamoyl-2-fluorophenyl)-2H-chromen-8-yl)piperidin-1-yl)methyl)-1-(((S)-oxetan-2-yl)methyl)-1H-benzo[d]imidazole-6-carboxylic acid

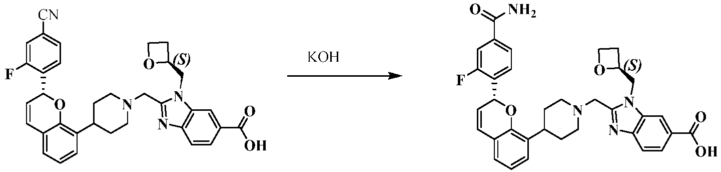

2-((4-((*R*)-2-(4-Cyano-2-fluorophenyl)-2*H*-chromen-8-yl)piperidin-1-yl)methyl)-1-(((*S*)-oxetan-2-yl)methyl)-1*H*-benzo[d]imidazole-6-carboxylic acid (100 mg, 172.82 µmol) and ethanol (5 mL) were added to a 25mL flask, and potassium hydroxide (96.96 mg, 1.73 mmol) was dissolved in water (1 mL) and added dropwise to the reaction solution at 25 °C. Then the reaction solution was reacted at 80 °C for 2 hours. The reaction was stopped, cooled to 25 °C, the reaction solution was quenched with formic acid (0.1 mL), followed by the addition of water (2 mL), and extracted with dichloromethane (2 mL×3). The organic phase was washed with saturated sodium chloride solution (2 mL), dried over anhydrous sodium sulfate, filtered, and concentrated to dryness by rotary evaporation. The resulting residue was purifed by prep-HPLC to obtain the title product 2-((4-((*R*)-2-(4-carbamoyl-2-fluorophenyl)-2*H-*chromen-8-yl)piperidin-1-yl)methyl)-1-(((*S*)-oxetan-2-yl)methyl)-1*H-*benzo[d]imidazole-6-carboxylic acid (35 mg) with a yield of 33.94%.

MS m/z (ESI): 597.2 [M+1].

### Example 60

### 2-((4-((R)-2-(4-(Dimethylcarbamoyl)-2-fluorophenyl)-2H-chromen-8-yl)piperidin-1-yl)methyl)-1-(((S)-oxetan-2-yl)methyl)-1H-benzo[d]imidazole-6-carboxylic acid

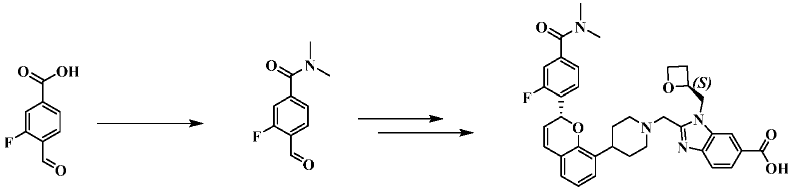

### Step 1

### 3-Fluoro-4-formyl-N,N-dimethylbenzamide

3-Fluoro-4-formylbenzoic acid (5 g, 29.74 mmol) was dissolved in N,N-dimethylformamide (50 mL), then dimethanamine (1.34 g, 29.74 mmol), HATU (11.30 g, 29.74 mmol), and DIEA (3.84 g, 29.74 mmol) were added to the reaction. The reaction was stirred at 25 °C for 8 hours. The reaction was stopped, water (50 mL) was added, and the reaction solution was extracted with dichloromethane (30 mL×3). The organic phase was washed with saturated sodium chloride solution (30 mL), dried over anhydrous sodium sulfate, filtered, and concentrated to dryness by rotary evaporation. The resulting residue was purified by silica gel column chromatography with petroleum ether and ethyl acetate as eluent system to obtain the title product 3-fluoro-4-formyl-N,N-dimethylbenzamide (4.5 g) with a yield of 77.52%.

MS m/z (ESI): 196.0 [M+1]

### Step 2

### 2-((4-((R)-2-(4-(Dimethylcarbamoyl)-2-fluorophenyl)-2H-chromen-8-yl)piperidin-1-yl)methyl)-1-(((S)-oxetan-2-yl)methyl)-1H-benzo[d]imidazole-6-carboxylic acid

Using 3-fluoro-4-formyl-N,N-dimethylbenzamide as the starting material and referring to Example 48, the target product 2-((4-((*R*)-2-(4-(dimethylcarbamoyl)-2-fluorophenyl)-2*H*-chromen-8-yl)piperidin-1-yl)methyl)-1-(((S)-oxetan-2-yl)methyl)-1*H*-benzo[d]imidazole-6-carboxylic acid was obtained.

MS m/z (ESI): 625.2 [M+1].

### Example 61

### 2-((4-((R)-2-(4-(Cyclopropylcarbonyl)-2-fluorophenyl)-2H-chromen-8-yl)piperidin-1-yl)methyl)-1-(((S)-oxetan-2-yl)methyl)-1H-benzo[d]imidazole-6-carboxylic acid

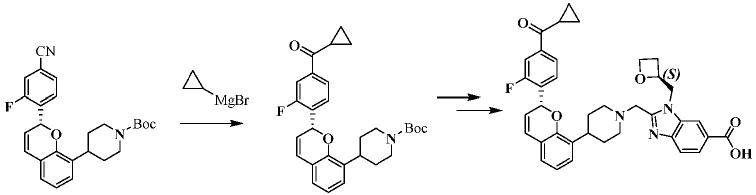

### Step 1

### Tert-butyl (R)-4-(2-(4-(cyclopropylcarbonyl)-2-fluorophenyl)-2H-chromen-8-yl)piperidine-1 -carboxylate

Tert-butyl (*R*)-4-(2-(4-cyano-2-fluorophenyl)-2*H*-chromen-8-yl)piperidine-1-carboxylate (1 g, 2.30 mmol) and tetrahydrofuran (20 mL) were added to a 100mL flask. Cyclopropyl magnesium bromide (2.3 mL, 1M, 2.30 mmol) was added at 0 °C, and the reaction solution was reacted at 80 °C for 12 hours, cooled to 0 °C, then 2N hydrochloric acid (2 mL) was added, the reaction solution was reacted at 0 °C for 2 hours. Then the reaction solution was neutralized with a saturated sodium bicarbonate solution, and extracted with ethyl acetate (20 mL×3). The organic phase was washed with saturated sodium chloride solution (20 mL), dried over anhydrous sodium sulfate, filtered, and concentrated to dryness by rotary evaporation. The resulting residue was purified by silica gel column chromatography with petroleum ether and ethyl acetate as eluent system to obtain the title product tert-butyl (*R*)-4-(2-(4-(cyclopropylcarbonyl)-2-fluorophenyl)-2*H*-chromen-8-yl)piperidine-1-carboxylate (0.6 g) with a yield of 54.59%.

MS m/z (ESI): 478.2 [M+1]

### Step 2

### 2-((4-((R)-2-(4-(Cyclopropylcarbonyl)-2-fluorophenyl)-2H-chromen-8-yl)piperidin-1-yl)methyl)-1-(((S)-oxetan-2-yl)methyl)-1H-benzo[d]imidazole-6-carboxylic acid

Using tert-butyl (R)-4-(2-(4-(cyclopropylcarbonyl)-2-fluorophenyl)-2*H*-chromen-8-yl)piperidine-1-carboxylate as the starting material, and referring to Step 5 to Step 7 of Example 48, the target product 2-((4-((*R*)-2-(4-(cyclopropylcarbonyl)-2-fluorophenyl)-2*H*-chromen-8-yl)piperidin-1-yl)methyl)-1-(((*S*)-oxetan-2-yl)methyl)-1*H*-benzo[d]imidazole-6-carboxylic acid was obtain.

MS m/z (ESI): 622.2 [M+1].

### Biological Assay and Evaluation

The present invention is further illustrated below in combination with the following test examples, which are not intended to limit the scope of the present invention.

### I. Determination of the ability of the compound of the present invention to stimulate human GLP1 receptor stable transgenic cell lines to produce cAMP

1. Experimental purpose:
The purpose of this test example is to determine the ability of the compound to activate the human GLP-1 receptor on the cell surface. The EC50 that stimulates AMP-production after agonism characterizes the compound's ability to activate the human GLP-1 receptor.
2. Experimental reagents and instruments:
2.1 Experimental instruments:
   Microplate reader (BioTek Synergy H1);
   Pipette (Eppendorf & Rainin).
2.2 Experimental reagents:
   DMEM/F12 medium was purchased from Gibco, and the article number was 11330032;
   Casein was purchased from Sigma, and the article number was C3400;
   384-well plate was purchased from Sigma, and the article number was CLS4514;
   IBMX was purchased from Sigma, and the article number was I7018;
   Cisbio cAMP - Gs Dynamic kit was purchased from Cisbio, and the article number was 62AM4PEC.

3. Experimental methods:
The frozen human GLP1 receptor stable transgenic cell line CHO-K1/GLP-1R/CRE-luc was taken from the liquid nitrogen tank, quickly thawed in a 37°C water bath, resuspended with DMEM/F12 medium, centrifuged, and washed once. The cells were resuspended with the experimental buffer, namely DMEM/F12 medium containing 0.1% casein, and the experimental buffer was used to adjust the cell density. The cells were spread on spread on a 384-well plate at a density of 2500 cells / 5 µL/ well, and then to each well was added 2.5 µL of buffer-prepared IBMX working solution with a final concentration of IBMX of 0.5 mM, and 2.5 µL of the compound sample with gradient dilution (starting at 1000 nM, 3-fold dilution, 11 concentrations). The plate was centrifuged at 1000 rpm for 1 minute, shaken for 30 seconds to mix well, and incubated at room temperature for 30 minutes. Detection was performed using the Cisbio cAMP - Gs Dynamic kit. cAMP-d2 and Anti-cAMP-Eu³⁺-Cryptate were diluted 20-fold with cAMP Lysis & Detection Buffer, respectively, and mixed well. To each well was added 5 µL diluted cAMP-d2 solution, and then added 5 µL diluted Anti-cAMP-Eu3⁺-Cryptate solution. The plate was shaken for 30 seconds, and incubated at room temperature for 1 hour in the dark. The signal reading of HTRF was performed using the Biotek Synergy H1 microplate reader with an excitation wavelength of 320 nm and emission wavelengths of 620 nm and 665 nm.
4. Experimental data processing methods:
The signal ratio (665nm/620nm* 10,000) was calculated, and the signal ratio and sample concentration were nonlinearly fitted using a four-parameter equation in GraphPad Prism 6 to obtain the EC50 value.
5. Experimental results:

**Table 1**

| Example | EC₅₀ (nM) |
|---|---|
| 1 | 0.13 |
| 2-A | 0.07 |
| 3 | 0.06 |
| 23-A | 0.07 |
| 24-A | 0.05 |
| 40 | 0.1 |
| 41 | 0.04 |
| 42 | 0.07 |
| 43 | 0.05 |
| 47 | 0.08 |
| 48 | 0.03 |
| 49 | 0.08 |
| 52 | 0.05 |
| 53 | 0.06 |

6. Experimental conclusion:
Through the above protocol, it is concluded that the example compound of the present invention shows good biological activity in the experiment of stimulating the human GLP1 receptor stable transgenic cell lines to produce cAMP.

### II. The effect of single administration of the compound of the present invention on the intraperitoneal glucose tolerance in GLP-1R humanized mice

1. Experimental purpose:
To evaluate the effect of single administration of the compound of the present invention on blood glucose changes in the intraperitoneal glucose tolerance (*ip*GTT) experiment in GLP-1R humanized C57BL/6 mice.
2. Experimental materials:
C57BL/6*_hGLP-1R*, male, 5-8 weeks; Ultra-clean workbench; Electronic balance; Vitality type blood glucose meter; Glucose.
3. Experimental operations and data processing:
3.1 On the day before the experiment, the animals were randomly divided according to their weight, with 5 animals per group. All animals were deprived of food overnight and fasted for more than 16 hours from the time of administration.
3.2 0.2 g/mL glucose solution was prepared with pure water and filtered by 0.22 µm filter membrane for use.
3.3 On the testing day, the blood glucose value of each animal was measured successively by tail clipping method before administration, which was recorded as Baseline value;
Blood glucose test method: the mouse was placed in the immobilizer, the tail tip was disinfected with an alcohol cotton ball, then a little tail tip was cut off with scissors, the first drop of blood was discarded, and the second drop of blood was dropped onto the prepared blood glucose test strip to detect the blood glucose value;
3.4 The animals were administered according to animal weight, and the administration time was record for each animal. After 1 hour of administration, the blood glucose value of each animal was measured sequentially, and recorded as the 0 minute blood glucose value;
3.5 Subsequently, according to the weight of the day, the aminanls were immediately injected with pure water or glucose solution into the abdominal cavity, with a volume of 10 mL/kg and a glucose dose of 2 g/kg;
3.6 The blood glucose values of each mouse were measured, and the time and data were recorded after 15, 30, 60, 90, and 120 minutes of pure water or glucose solution injection;
3.7 After the test, all animals resumed feeding.
3.8 Data processing:
   The blood glucose (BG) -time curve was drawn, and the area under the blood glucose -time curve was calculated. The calculation formula is as follows: AUC (mmol/L.hr) = (BG0+BG15)×0.25/2 + (BG15+BG30)×0.25/2 + (BG30+BG60)×0.5/2 + (BG60+BG90)×0.5/2+ (BG90+BG120)×0.5/2.
   Note: BG0, BG15, BG30, BG60, BG90, and BG120 represent blood glucose values before glucose administration (0 min) and 15, 30, 60, 90, and 120 min after glucose administration, respectively.
   The blood glucose decline rate of AUC at each time point was calculated according to the average blood glucose value and AUC of blood glucose at each time point. The calculation formula is: Blood glucose decline rate= (blood glucose in the administration group /AUC - blood glucose in the model control group/ AUC)/blood glucose in the model control group /AUC × 100%.

4. Experimental results:

| Compound | Dosage (mg/kg) | Number of animals | Blood glucose decline rate of AUC% |
|---|---|---|---|
| Vehicle | - | 5 | - |
| Example 41 | 0.3 | 5 | 53.09 |
| Example 42 | 0.3 | 5 | 56.79 |
| Example 43 | 0.3 | 5 | 46.57 |
| Example 48 | 0.3 | 5 | 62.89 |

5. Experimental conclusion:
According to the above experimental results, it can be seen that the example compounds of the present invention can effectively reduce blood sugar in mice.

### III. The effect of long-term administration of the compound of the present invention on the body weight and ingestion of GLP-1R humanized mice fed with high-fat diet

1. Experimental purpose:
The purpose of this test is to evaluate the effects of long-term administration of the compound on the body weight and ingestion of GLP-1R humanized C57BL/6 mice fed with high-fat diet.
2. Experimental reagents and instruments:
C57BL/6*_hGLP-1R,* male, 5-8 weeks; 60% high-fat diet (HFD); Ultra-clean workbench; Electronic balance.
3. Experimental method:
3.1 On the day of feeding with high-fat diet, C57BL/6 mice were randomly divided into two groups according to their weight. The first group was Blank, with 7 mice fed with normal control diet, while the remaining animals were in the modeling group, fed with high-fat diet, and continued until the end of the experiment.
3.2 At the 8^{th} week of HFD feeding, the modeling group animals were randomly divided with 7 animals per group according to their weight. The first group was the Vehicle group (Vehicle: 0.5% CMC Na+1% Tween 80), which was given vehicle; The remaining group was the administration group; Administration regimen: Oral administration of the corresponding compound, administration cycle was 14 days, once a day for 14 days, with an administration dose of 10 mg/kg and an administration volume of 10 mL/kg. The Blank group continued to be fed with normal diet without any administration operation.
3.3 The day of administration was defined as Day 0.
3.4 During each administration, animals were weighed and data was recorded. Oral administration is performed according to body weight, with an administration volume of 10 mL/kg.
3.5 Starting from Day 0 of the experiment, the food intake of each group of mice was measured once every three days. Specifically, the feed was changed after each weighing and administration, and the added and remaining amounts were recorded.
3.6 On the end day of Day 14, all mice were euthanized sequentially according to grouping order, and the livers were dissected and weighed.

4. Experimental data processing and statistical analysis
The weight and weight change rate of mice after administration were summarize and statistically analyzed. Weight change rate calculation: (BWt - BW0)/BW0 × 100%. BWt represents the weight of the mice on Day t of the experiment, and BW0 represents the weight of the mice on Day 0 of the experiment.
Calculation of food intake: (added amount (g) - remaining amount (g))/number of animals per cage, cumulative food intake is the sum of daily food intake of each animal during the administration period.
Experimental data were analyzed using GraphPad Prism software. The t-test method was used for comparison between the two groups. The one-way ANOVA method was used for comparisons between three or more groups.

5. Experimental results:

| **Compound** | **Dosage (mg/kg)** | **Weight (g)-D0** | | | **Weight (g)-D10** | | | **Body weight change(%)-D10** |
|---|---|---|---|---|---|---|---|---|
| | | **Mean** | **±** | **SEM** | **Mean** | **±** | **SEM** | **Mean** |
| Vehicle | - | 28.52 | ± | 0.54 | 28.28 | ± | 0.58 | -0.84% |
| Example 41 | 10 | 28.54 | ± | 0.37 | 26.20 | ± | 0.36 | -8.19% |
| Example 40 | 10 | 28.40 | ± | 0.67 | 26.01 | ± | 0.85 | -8.49% |
| Example 42 | 10 | 28.54 | ± | 0.46 | 26.04 | ± | 0.42 | -8.75% |

| **Compound** | **Dosage (mg/kg)** | **Weight (g)-D0** | | | **Eeight (g)-D10** | | | **Body weight change(%)-D10** |
|---|---|---|---|---|---|---|---|---|
| | | **Mea n** | **±** | **SEM** | **Mean** | **±** | **SEM** | **Mean** |
| Vehicle | - | 28.09 | ± | 0.81 | 28.23 | ± | 0.75 | 0.55% |
| Example 43 | 10 | 28.37 | ± | 1.06 | 26.57 | ± | 1.14 | -6.37% |
| Example 48 | 10 | 28.23 | ± | 0.37 | 25.85 | ± | 0.44 | -8.44% |
| Example 53 | 10 | 28.34 | ± | 0.46 | 25.65 | ± | 0.77 | -9.53% |

6. Experimental conclusion:
According to the above experimental results, it can be seen that long-term administration of the example compounds of the present invention has a good weight reduction effect on GLP-1R humanized C57BL/6 mice fed with high-fat diet.

### IV. Pharmacokinetic assay of SD rats

1. Research purpose:
   SD rats were used as test animals. The pharmacokinetic behavior of the following example compounds was studied in the plasma of rats administered orally at a dose of 50 mg/kg.
2. Test protocol:
   2.1 Test drugs:
      Vehicle formula: 0.5% CMC-Na (1% Tween 80);
      Compounds of the examples of the present invention are self-made.
   2.2 Test animals:
      Three SD rats per group, male.
   2.3 Administration:

Three SD rats per group, male; p.o. after fasting overnight, respectively, with a dose of 5 mg/kg, an administration volume of 10 mL/kg.

### 2.4 Sample collection:

Before and after administration, 0.2 mL of blood was collected from the jugular vein at 0, 0.25, 0.5, 1, 2, 4, 6, 8 and 24 hours, placed in EDTA-K₂ tubes, centrifuged at 6000 rpm at 4°C for 6 min to separate the plasma. The plasma samples were stored at - 80°C. The mouse was fed 4 hours after the administration.

### 2.5 Sample process:

1) 160 µL of acetonitrile was added to 40 µL of the plasma sample for precipitation, and then the mixture was centrifuged at 3500 × g for 5-20 min.
2) After the above process, 100 µL of the supernatant was taken to analyze the concentration of the test compound by LC/MS/MS.

### 2.6 Liquid chromatography analysis

• Liquid chromatography condition: Shimadzu LC-20AD pump
• Mass spectrometry condition: AB Sciex API 4000 mass spectrometer
• Chromatographic column: phenomenex Gemiu 5 µm C18 50 × 4.6 mm
• Mobile phase: Eluent A was 0.1% formic acid in water, and Eluent B was methanol
• Flow rate: 1.0 mL/min
• Elution time: 0-4.0 minutes, the eluent is as follows:

| Time/minute | Liquid A | Liquid B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

### 3. Experimental results and analysis

The main pharmacokinetic parameters were calculated using WinNonlin 8.2.

### 4. Experimental results

| Compound | tₘₐₓ(h) | Cₘₐₓ(ng/mL) | AUC₀₋ₜ(ng/mL*h) | AUC0-∞ (ng/ml*h) | t_{1/2}(h) |
|---|---|---|---|---|---|
| Example 2-A | 2.0 | 7157 | 35755 | 35838 | 3.1 |
| Example 40 | 1.0 | 2303 | 10311 | 10831 | 2.0 |
| Example 48 | 2.0 | 6403 | 19084 | 19114 | 3.8 |
| Example 52 | 1.0 | 9397 | 12550 | 12555 | 1.6 |
| Example 53 | 0.5 | 7980 | 21532 | 21556 | 2.0 |

### 5. Experimental conclusion:

It can be seen from the results of rat pharmacokinetic experiments in the table that the compounds of the examples of the present invention show good metabolic properties at a dose of 50 mg/kg, and both the exposure AUC and the maximum plasma concentration Cₘₐₓ perform well.

## Claims

1. A compound of formula (I'), a stereoisomer thereof or a pharmaceutically acceptable salt thereof: 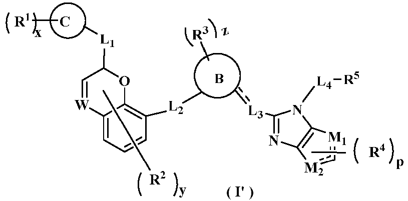 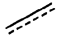 is a single bond or a double bond;
ring B is selected from the group consisting of cycloalkyl, heterocyclyl, aryl and heteroaryl, wherein the cycloalkyl, heterocyclyl, aryl, and heteroaryl can be each optionally further substituted; or, ring B is absent;
ring C is selected from aryl or heteroaryl, preferably phenyl or pyridinyl;
L₁ is selected from the group consisting of a bond, alkenylene, alkynylene, - (CH₂)ₙ₁-, -(CH₂)ₙ₁(CRₐₐR_{bb})ₙ₂-, -(CRₐₐR_{bb})ₙ₁O(CH₂)ₙ₂-, -(CH₂)ₙ₁O(CRₐₐR_{bb})ₙ₂-, - (CRₐₐR_{bb})ₙ₁S(CH₂)ₙ₂-, -(CH₂)ₙ₁S(CRₐₐR_{bb})ₙ₂-, -(CRₐₐR_{bb})ₙ₁(CH₂)ₙ₂NR_{cc}-, - (CH₂)ₙ₁NRₐₐ(CR_{bb}R_{cc})ₙ₂-, -(CH₂)ₙ₁C(O)(CRₐₐR_{bb})ₙ₂-, -(CH₂)ₙ₁NRₐₐC(O)(CRₐₐR_{bb})ₙ₂-, - (CH₂)ₙ₁P(O)Rₐₐ-, -(CH₂)ₙ₁S(O)ₘ₁-, -(CH₂)ₙ₁S(O)ₘ₁NRₐₐ-, -(CH₂)ₙ₁NRₐₐS(O)ₘ₁- and - (CH₂)ₙ₁C(O)NRₐₐ-;
Rₐₐ, R_{bb}, and R_{cc} are each independently selected from the group consisting of hydrogen, deuterium, halogen, amino, nitro, hydroxy, cyano, carboxy, alkyl, deuterated alkyl, haloalkyl, hydroxyalkyl, alkoxy, haloalkoxy, deuterated alkoxy, alkenyl, alkynyl, heterocyclylalkyl, cycloalkyl, heterocyclyl, aryl and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, any two of Rₐₐ, R_{bb}, and R_{cc} are bonded to form a cycloalkyl, heterocyclyl, aryl or heteroaryl, wherein the cycloalkyl, heterocyclyl, aryl and heteroaryl can be each optionally further substituted;
L₂ is selected from the group consisting of a bond, alkenylene, alkynylene, - (CH₂)ₙ₃-, -(CH₂)ₙ₃(CRₐ₁R_{b1})ₙ₄-, -(CRₐ₁R_{b1})ₙ₃O(CH₂)ₙ₄-, -(CH₂)ₙ₃O(CRₐ₁R_{b1})ₙ₄-, - (CRₐ₁R_{b1})ₙ₃S(CH₂)ₙ₄-, -(CH₂)ₙ₃S(CRₐ₁R_{b1})ₙ₄-, -(CRₐ₁R_{b1})ₙ₃(CH₂)ₙ₄NR_{c1}-, - (CH₂)ₙ₃NRₐ₁(CR_{b1}R_{c1})ₙ₄-, -(CH₂)ₙ₃C(O)(CRₐ₁R_{b1})ₙ₄-, -(CH₂)ₙ₃NRₐ₁C(O)(CRₐ₁R_{b1})ₙ₄-, - (CH₂)ₙ₃P(O)Rₐ₁-, -(CH₂)ₙ₃S(O)ₘ₂-, -(CH₂)ₙ₃S(O)ₘ₂NRₐ₁-, -(CH₂)ₙ₃NRₐ₁S(O)ₘ₂- and - (CH₂)ₙ₃C(O)NRₐ₁-;
Rₐ₁, R_{b1} and R_{c1} are each independently selected from the group consisting of hydrogen, deuterium, halogen, amino, nitro, hydroxy, cyano, carboxy, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, Rₐ₁ is absent;
or, any two of Rₐ₁, R_{b1} and R_{c1} are bonded to form a cycloalkyl, heterocyclyl, aryl or heteroaryl, wherein the cycloalkyl, heterocyclyl, aryl and heteroaryl can be each optionally further substituted;
L₃ is selected from the group consisting of a bond, alkenylene, alkynylene, - (CH₂)ₙ₅-, -(CH₂)ₙ₅(CRₐ₂R_{b2})ₙ₆-, -(CRₐ₂R_{b2})ₙ₅O(CH₂)ₙ₆-, -(CH₂)ₙ₅O(CRₐ₂R_{b2})ₙ₆-, - (CRₐ₂R_{b2})ₙ₅S(CH₂)ₙ₆-, -(CH₂)ₙ₅S(CRₐ₂R_{b2})ₙ₆-, -(CRₐ₂R_{b2})ₙ₅(CH₂)ₙ₆NR_{c2}-, - (CH₂)ₙ₅NRₐ₂(CR_{b2}R_{c2})ₙ₆-, -(CH₂)ₙ₅C(O)(CRₐ₂R_{b2})ₙ₆-, -(CH₂)ₙ₅NRₐ₂C(O)(CRₐ₂R_{b2})ₙ₆-, - (CH₂)ₙ₅P(O)Rₐ₂-, -(CH)ₙ(CH₂)ₙ₅S(O)ₘ₃-, -(CH₂)ₙ₅S(O)ₘ₃NRₐ₂-, -(CH₂)ₙ₅NRₐ₂S(O)ₘ₃- and -(CH₂)ₙ₅C(O)NRₐ₂-;
Rₐ₂, R_{b2} and R_{c2} are each independently selected from the group consisting of hydrogen, deuterium, halogen, amino, nitro, hydroxy, cyano, oxo, thioxo, carboxy, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, Rₐ₂ is absent;
W is selected from CRₘ or N;
Rₘ is selected from the group consisting of hydrogen, deuterium, halogen, amino, nitro, hydroxy, cyano, oxo, thioxo, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
L₄ is selected from the group consisting of a bond, -(CH₂)ₙ₇-, -(CH₂)ₙ₇(CRₐ₃R_{b3})ₙ₈-, -(CRₐ₃R_{b3})ₙ₇O(CH₂)ₙ₈-, -(CH₂)ₙ₇O(CRₐ₃R_{b3})ₙ₈-, -(CRₐ₃R_{b3})ₙ₇S(CH₂)ₙ₈-, - (CH₂)ₙ₇S(CRₐ₃R_{b3})ₙ₈-, -(CRₐ₃R_{b3})ₙ₇(CH₂)ₙ₈NRc₃-, -(CH₂)ₙ₇NRₐ₃(CR_{b3}R_{c3})ₙ₈-, - (CH₂)ₙ₇C(O)(CRₐ₃R_{b3})ₙ₈-, -(CH₂)ₙ₇NRₐ₃C(O)(CRₐ₃R_{b3})ₙ₈-, -(CH₂)ₙ₇P(O)Rₐ₃-, - (CH₂)ₙ₇S(O)ₘ₄-, -(CH₂)ₙ₇S(O)ₘ₄NRₐ₃-, -(CH₂)ₙ₇NRₐ₃S(O)ₘ₄- and -(CH₂)ₙ₇C(O)NRₐ₃-;
Rₐ₃, R_{b3} and R_{c3} are each independently selected from the group consisting of hydrogen, deuterium, halogen, amino, nitro, hydroxy, cyano, oxo, thioxo, carboxy, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
or, any two of Rₐ₃, R_{b3} and R_{c3} are bonded to form a cycloalkyl, heterocyclyl, aryl or heteroaryl, optionally can be further substituted;
R¹ is each independently selected from the group consisting of hydrogen, deuterium, halogen, amino, nitro, hydroxy, cyano, alkyl, deuterated alkyl, haloalkyl, hydroxyalkyl, alkoxy, haloalkoxy, deuterated alkoxy, alkenyl, alkynyl, heterocyclylalkyl, cycloalkyl, heterocyclyl, aryl, heteroaryl, -(CH₂)ₙ₁C(O)Rₐₐ and - (CH₂)ₙ₁C(O)NRₐₐR_{bb}, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, any two of R¹ are bonded to form a cycloalkyl, heterocyclyl, aryl or heteroaryl, wherein the cycloalkyl, heterocyclyl, aryl and heteroaryl can be each optionally further substituted;
or, R¹ and L₁ are bonded to form a cycloalkyl, heterocyclyl, aryl or heteroaryl, wherein the cycloalkyl, heterocyclyl, aryl and heteroaryl can be each optionally further substituted;
R² is each independently selected from the group consisting of hydrogen, deuterium, halogen, amino, nitro, hydroxy, cyano, oxo, thioxo, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, any two of R² are bonded to form a cycloalkyl, heterocyclyl, aryl or heteroaryl, wherein the cycloalkyl, heterocyclyl, aryl and heteroaryl can be each optionally further substituted;
or, R² and L₁ are bonded to form a cycloalkyl, heterocyclyl, aryl or heteroaryl, wherein the cycloalkyl, heterocyclyl, aryl and heteroaryl can be each optionally further substituted;
or, R² and L₂ are bonded to form a cycloalkyl, heterocyclyl, aryl or heteroaryl, wherein the cycloalkyl, heterocyclyl, aryl and heteroaryl can be each optionally further substituted;
or, R² and R³ are bonded to form a cycloalkyl, heterocyclyl, aryl or heteroaryl, wherein the cycloalkyl, heterocyclyl, aryl and heteroaryl can be each optionally further substituted;
R³ is each independently selected from the group consisting of hydrogen, deuterium, halogen, amino, nitro, hydroxy, cyano, thiol, oxo, thioxo, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, wherein the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl can be each optionally further substituted;
or, any two of R³ are bonded to form a cycloalkyl, heterocyclyl, aryl or heteroaryl, wherein the cycloalkyl, heterocyclyl, aryl and heteroaryl can be each optionally further substituted;
or, R³ and L₂ are bonded to form a cycloalkyl, heterocyclyl, aryl or heteroaryl, wherein the cycloalkyl, heterocyclyl, aryl and heteroaryl can be each optionally further substituted;
or, R³ and L₃ are bonded to form a cycloalkyl, heterocyclyl, aryl or heteroaryl, wherein the cycloalkyl, heterocyclyl, aryl and heteroaryl can be each optionally further substituted;
R⁴ is each independently selected from the group consisting of hydrogen, deuterium, halogen, oxo, amino, nitro, hydroxy, C₁₋₆ carboxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
R⁵ is selected from the group consisting of hydrogen, deuterium, halogen, amino, nitro, hydroxy, cyano, oxo, thioxo, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
Mi is selected from the group consisting of O, S, N, C, (CH)ₙ₉, (CH₂)ₙ₉O, (CH₂)ₙ₉S, (CH₂)ₙ₉NH and (CH₂)ₙ₉;
M₂ is selected from the group consisting of O, S, N, C, (CH)ₙ₁₀, (CH₂)ₙ₁₀O, (CH₂)ₙ₁₀S, (CH₂)ₙ₁₀NH and (CH₂)ₙ₁₀;
x is an integer from 0 to 12;
y is an integer from 0 to 12;
z is an integer from 0 to 12;
p is an integer from 0 to 4;
n1~n6 are each independently integer from 0 to 3;
n7~n10 are each independently integer from 0 to 3;
m1~m3 are each independently integer from 0 to 2; and
m4 is an integer from 0 to 2.

2. The compound, the stereoisomer thereof or the pharmaceutically acceptable salt thereof according to claim 1, **characterized in that** the compound is further shown as formula (I): 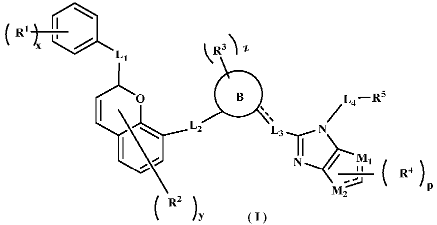
L₄ is selected from the group consisting of a bond, -(CH₂)ₙ₇-, -(CH₂)ₙ₇(CRₐ₃R_{b3})ₙ₈-, -(CRₐ₃R_{b3})ₙ₇O(CH₂)ₙ₈-, -(CH₂)ₙ₇O(CRₐ₃R_{b3})ₙ₈-, -(CRₐ₃R_{b3})ₙ₇S(CH₂)ₙ₈-, - (CH₂)ₙ₇S(CRₐ₃R_{b3})ₙ₈-, -(CRₐ₃R_{b3})ₙ₇(CH₂)ₙ₈NR_{c3}-, -(CH₂)ₙ₇NRa₃(CR_{b3}R_{c3})ₙ₈-, - (CH₂)ₙ₇C(O)(CRₐ₃R_{b3})ₙ₈-, -(CH₂)ₙ₇NRa₃C(O)(CRₐ₃R_{b3})ₙ₈-, -(CH₂)ₙ₇P(O)Rₐ₃-, - (CH₂)ₙ₇S(O)ₘ₄-, -(CH₂)ₙ₇S(O)ₘ₄NRₐ₃-, -(CH₂)ₙ₇NRₐ₃S(O)ₘ₄- and -(CH₂)ₙ₇C(O)NRₐ₃-;
Rₐ₃, R_{b3} and R_{c3} are each independently selected from the group consisting of hydrogen, deuterium, halogen, amino, nitro, hydroxy, cyano, oxo, thioxo, carboxy, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
or, any two of Rₐ₃, R_{b3} and R_{c3} are bonded to form a cycloalkyl, heterocyclyl, aryl or heteroaryl, optionally can be further substituted;
R⁴ is each independently selected from the group consisting of hydrogen, deuterium, halogen, oxo, amino, nitro, hydroxy, carboxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
R⁵ is selected from the group consisting of hydrogen, deuterium, halogen, amino, nitro, hydroxy, cyano, oxo, thioxo, alkyl, deuterated alkyl, haloalkyl, hydroxyalkyl, alkoxy, haloalkoxy, alkenyl, alkynyl, heterocyclylalkyl, cycloalkyl, heterocyclyl, aryl and heteroaryl, optionally can be further substituted;
Mi is selected from the group consisting of O, S, N, C, (CH)ₙ₉, (CH₂)ₙ₉O, (CH₂)ₙ₉S, (CH₂)ₙ₉NH and (CH₂)ₙ₉;
M₂ is selected from the group consisting of O, S, N, C, (CH)ₙ₁₀, (CH₂)ₙ₁₀O, (CH₂)ₙ₁₀S, (CH₂)ₙ₁₀NH and (CH₂)ₙ₁₀;
p is an integer from 0 to 4;
n7~n10 are each independently integer from 0 to 3;
m4 is an integer from 0 to 2.

3. The compound, the stereoisomer thereof or the pharmaceutically acceptable salt thereof according to claim 1 or 2, **characterized in that**,
ring B is selected from the group consisting of C₃₋₁₂ cycloalkyl, 3 to 12 membered heterocyclyl, C₆₋₁₄ aryl and 5 to 14 membered heteroaryl;
preferably C₃₋₈ cycloalkyl, 4 to 12 membered heterocyclyl, C₆₋₁₀ aryl and 5 to 10 membered heteroaryl;
more preferably C₅₋₈ cycloalkyl, 5 to 10 membered heterocyclyl comprising 1 to 3 nitrogen atoms, oxygen atoms, or sulfur atoms, C₆₋₈ aryl and 5 to 10 membered heteroaryl comprising 1 to 3 nitrogen atoms, oxygen atoms, or sulfur atoms;
further preferably piperidinyl and piperazinyl, and more further preferably piperidinyl.

4. The compound, the stereoisomer thereof or the pharmaceutically acceptable salt thereof according to claim 1, **characterized in that** the compound is further shown as formula (II'): 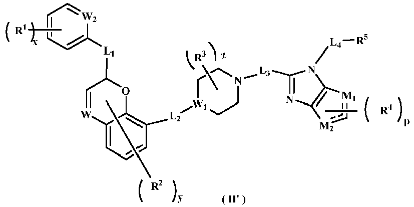
W₁ is selected from CH or N;
W₂ is selected from CRₘ₅ or N;
R¹ is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, -C(O)Rₐₐ and -C(O)NRₐₐR_{bb};
Rₐₐ, and R_{bb} are each independently selected from the group consisting of hydrogen, deuterium, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3 to 8 membered heterocyclyl, C₆₋₁₀ aryl and 5 to 10 membered heteroaryl;
R² is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, and C₁₋₆ haloalkoxy;
R³ is each independently selected from the group consisting of hydrogen, deuterium, fluorine, methyl, deuterated methyl, and halomethyl;
Rₘ₅ is selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, C₁₋₆ deuterated alkoxy, and C₁₋₆ haloalkoxy, preferably hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy, more preferably hydrogen, and deuterium;
R⁴ is each independently selected from the group consisting of hydrogen, deuterium, fluorine, chlorine, cyano, oxo, C₁₋₆ carboxy, C₁₋₆ alkyl, C₁₋₆ deuterated alkyl, C₁₋₆ haloalkyl, and substituted or unsubstituted 5 to 6 membered heteroaryl, wherein the C₁₋₆ carboxy is optionally further substituted by one or more substituents selected from halogen or C₁₋₆ alkyl, preferably carboxy and -C(O)OCH₃;
R⁵ is selected from the group consisting of C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ cyanoalkyl, C₃₋₈ cycloalkyl, and 3 to 8 membered heterocyclyl, preferably C₁₋₆ alkyl, C₁₋₆ deuterated alkyl, C₁₋₆ haloalkyl, C₃₋₈ cycloalkyl, and 3 to 8 membered heterocyclyl, optionally further substituted by one or more substituents selected from the group consisting of halogen, C₁₋₆ alkyl, C₁₋₆ deuterated alkyl, C₁₋₆ haloalkyl, C₁₋₆ cyanoalkyl, C₃₋₈ cycloalkyl, and 3 to 8 membered heterocyclyl;
L₁ and L₂ are each independently selected from a bond or -(CH₂)ₙ₁-, preferably bond;
L₃ is selected from a bond or -(CH₂)ₙ₁-, preferably -CH₂-;
L₄ is selected from a bond or -(CH₂)ₙ₇-, preferably bond or -CH₂-;
Mi is selected from the group consisting of N, NH, O, S, C and (CH)ₙ₉;
M₂ is selected from the group consisting of N, NH, O, S, C and (CH)ₙ₁₀;
n9 or n10 is each independently integer from 0 to 2.

5. The compound, the stereoisomer thereof or the pharmaceutically acceptable salt thereof according to claim 1, **characterized in that** the compound is further shown as formula (II'-a) or (II'-b): 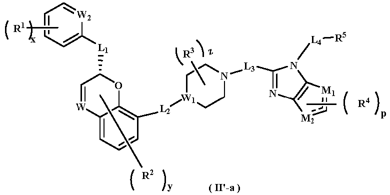 or

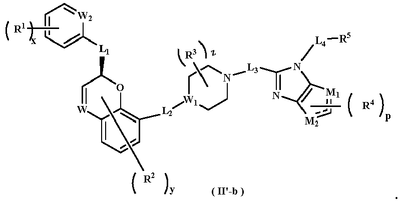

6. The compound, the stereoisomer thereof or the pharmaceutically acceptable salt thereof according to claim 3, **characterized in that** the compound is further shown as formula (II): 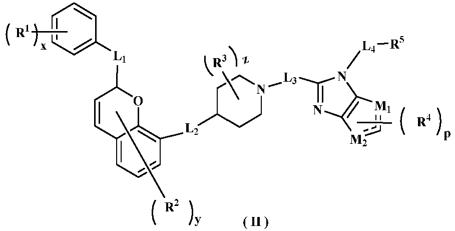
R¹ is each independently selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ deuterated alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
or, any two of R¹ are bonded to form a C₃₋₈ cycloalkyl, 3 to 8 membered heterocyclyl, C₆₋₁₀ aryl or 5 to 10 membered heteroaryl, wherein the C₃₋₈ cycloalkyl, 3 to 8 membered heterocyclyl, C₆₋₁₀ aryl and 5 to 10 membered heteroaryl are each optionally further substituted by one or more substituents selected from the group consisting of halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
R² is each independently selected from the group consisting of hydrogen, deuterium, halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
or, any two of R² are bonded to form a C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
R³ is each independently selected from the group consisting of hydrogen, deuterium, halogen, amino, nitro, hydroxy, cyano, carboxy, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, carboxy, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
R⁴ is each independently selected from the group consisting of hydrogen, deuterium, halogen, amino, nitro, hydroxy, cyano, carboxy, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, carboxy, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
or, any two of R⁴ are bonded to form a C₃₋₈ cycloalkyl, 3 to 8 membered heterocyclyl, C₆₋₁₀ aryl and 5 to 10 membered heteroaryl, wherein the C₃₋₈ cycloalkyl, 3 to 8 membered heterocyclyl, C₆₋₁₀ aryl and 5 to 10 membered heteroaryl are each optionally further substituted by one or more substituents selected from the group consisting of halogen, amino, nitro, hydroxy, cyano, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
R⁵ is selected from the group consisting of hydrogen, deuterium, halogen, amino, nitro, hydroxy, cyano, carboxy, oxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, amino, nitro, hydroxy, cyano, oxo, carboxy, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ cyanoalkyl, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
Mi is selected from the group consisting of O, S, N, NH, C, CH and (CH)₂;
M₂ is selected from the group consisting of O, S, N, NH, C, CH and (CH)₂;
x is 0, 1, 2, 3 ,4 or 5;
y is 0, 1, 2, 3 or 4;
z is 0, 1, 2, 3 or 4;
p is 0, 1, 2, or 3.

7. The compound, the stereoisomer thereof or the pharmaceutically acceptable salt thereof according to any one of claims 1 to 6, **characterized in that**,
R¹ is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, and C₁₋₆ haloalkoxy, preferably hydrogen, deuterium, fluorine, chlorine, cyano, C₁₋₃ alkyl, C₁₋₃ deuterated alkyl, C₁₋₃ haloalkyl, C₁₋₃ alkoxy, and C₁₋₃ haloalkoxy, more preferably hydrogen, deuterium, fluorine, chlorine, cyano and methoxy;
R² is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, and C₁₋₆ haloalkoxy, preferably hydrogen, deuterium, fluorine, chlorine, cyano, C₁₋₃ alkyl, C₁₋₃ deuterated alkyl, C₁₋₃ haloalkyl, C₁₋₃ alkoxy, and C₁₋₃ haloalkoxy, more preferably hydrogen, deuterium, fluorine, chlorine, and methyl;
R³ is each independently selected from the group consisting of hydrogen, deuterium, and fluorine, preferably hydrogen;
R⁴ is each independently selected from the group consisting of hydrogen, deuterium, fluorine, chlorine, cyano, carboxy, C₁₋₆ alkyl, C₁₋₆ deuterated alkyl, and C₁₋₆ haloalkyl, preferably carboxy;
R⁵ is selected from the group consisting of C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3 to 8 membered heterocyclyl, C₆₋₁₀ aryl and 5 to 10 membered heteroaryl, optionally further substituted by one or more substituents selected from the group consisting of halogen, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl and 3 to 8 membered heterocyclyl, preferably C₁₋₆ alkyl, C₁₋₆ deuterated alkyl, C₁₋₆ haloalkyl, C₃₋₈ cycloalkyl and 3 to 8 membered heterocyclyl, optionally further substituted by one or more substituents selected from the group consisting of halogen, C₁₋₆ alkyl, C₁₋₆ deuterated alkyl, C₁₋₆ haloalkyl, C₃₋₈ cycloalkyl and 3 to 8 membered heterocyclyl, more preferably 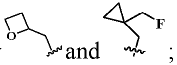
L₁ and L₂ are each independently selected from a bond or -(CH₂)ₙ₁-, preferably bond;
L₃ is selected from a bond or -(CH₂)ₙ₁-, preferably -CH₂-;
L₄ is selected from a bond or -(CH₂)ₙ₇-, preferably bond or -CH₂-.

8. The compound, the stereoisomer thereof or the pharmaceutically acceptable salt thereof according to any one of claims 1 to 6, **characterized in that**,
R¹ is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, C₁₋₆ deuterated alkoxy, and C₁₋₆ haloalkoxy, preferably hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy, more preferably hydrogen, deuterium, fluorine, chlorine, cyano, -OCD₃ and methoxy;
R² is each independently selected from the group consisting of hydrogen, deuterium, fluorine, chlorine, cyano, C₁₋₆ alkyl, C₁₋₆ deuterated alkyl, C₁₋₆ haloalkyl, C₁₋₆ alkoxy, C₁₋₆ deuterated alkoxy, and C₁₋₆ haloalkoxy, preferably hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy, more preferably hydrogen, deuterium, fluorine, chlorine, and methyl;
R³ is each independently selected from the group consisting of hydrogen, deuterium, and fluorine, preferably hydrogen;
R⁴ is each independently selected from the group consisting of hydrogen, deuterium, fluorine, chlorine, cyano, oxo, C₁₋₆ carboxy, C₁₋₆ alkyl, C₁₋₆ deuterated alkyl, C₁₋₆ haloalkyl and 5 to 6 membered heteroaryl comprising 1 to 4 nitrogen atoms, oxygen atoms, or sulfur atoms, wherein the C₁₋₆ carboxy and 5 to 6 membered heteroaryl comprising 1 to 4 nitrogen atoms, oxygen atoms are each optionally further substituted by one or more substituents selected from the group consisting of oxo, halogen, cyano, C₁₋₃ deuterated alkyl, C₁₋₃ haloalkyl, C₁₋₃ alkoxy, C₁₋₃ deuterated alkoxy, and C₁₋₃ haloalkoxy, preferably carboxy and -C(O)OCH₃;
R⁵ is selected from the group consisting of C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3 to 8 membered heterocyclyl, C₆₋₁₀ aryl and 5 to 10 membered heteroaryl, optionally further substituted by one or more substituents selected from halogen, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ cyanoalkyl, C₃₋₈ cycloalkyl, and 3 to 8 membered heterocyclyl, preferably C₁₋₃ alkyl, C₁₋₃ deuterated alkyl, C₁₋₃ haloalkyl, C₃₋₆ cycloalkyl, and 3 to 6 membered heterocyclyl, optionally further substituted by one or more substituents selected from the group consisting of halogen, C₁₋₃ alkyl, C₁₋₃ deuterated alkyl, C₁₋₃ haloalkyl, C₁₋₃ cyanoalkyl, C₃₋₆ cycloalkyl, and 3 to 6 membered heterocyclyl, more preferably C₃₋₆ cycloalkyl, and 3 to 6 membered heterocyclyl comprising 1 to 2 nitrogen atoms, oxygen atoms, or sulfur atoms, optionally further substituted by one or more substituents selected from the group consisting of halogen, C₁₋₃ alkyl, C₁₋₃ deuterated alkyl, C₁₋₃ cyanoalkyl, and C₁₋₃ haloalkyl, further preferably 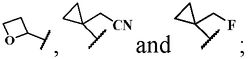
L₁ and L₂ are each independently selected from a bond or -(CH₂)ₙ₁-, preferably bond;
L₃ is selected from a bond or -(CH₂)ₙ₁-, preferably -CH₂-;
L₄ is selected from a bond or -(CH₂)ₙ₇-, preferably bond or -CH₂-.

9. The compound, the stereoisomer thereof or the pharmaceutically acceptable salt thereof according to claim 4, 7 or 8, **characterized in that** the compound is further shown as formula (III)-(III-1): 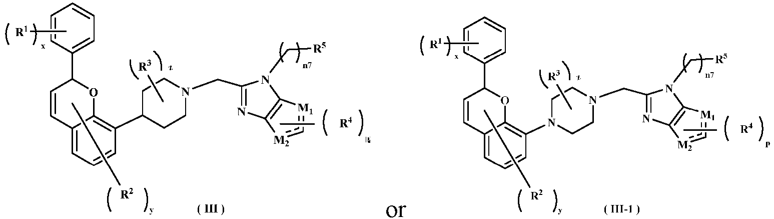 wherein:
R⁵ is selected from C₃₋₈ cycloalkyl, or 3 to 8 membered heterocyclyl, optionally further substituted by one or more substituents selected from the group consisting of halogen, C₁₋₆ alkyl, C₁₋₆ deuterated alkyl, C₁₋₆ cyanoalkyl, and C₁₋₆ haloalkyl;
Mi is selected from the group consisting of O, S, N, C, CH and (CH)₂;
M₂ is selected from the group consisting of O, S, N, C, CH and (CH)₂;
n7 is 0, 1, 2, or 3;
x is 0, 1, 2, 3 ,4 or 5;
y is 0, 1, 2, 3 or 4;
z is 0, 1, 2, 3 or 4;
p is 0, 1, 2, or 3.

10. The compound, the stereoisomer thereof or the pharmaceutically acceptable salt thereof according to claim 4, **characterized in that**,
R¹ is each independently selected from the group consisting of hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy;
R² is each independently selected from the group consisting of hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy;
R³ is each independently selected from the group consisting of hydrogen, deuterium, and fluorine;
R⁴ is each independently selected from 5 to 6 membered heteroaryl comprising 1 to 4 nitrogen atoms, oxygen atoms, or sulfur atoms, optionally further substituted by one or more substituents selected from the group consisting of oxo, halogen, cyano, C₁₋₃ alkyl, and C₁₋₃ haloalkyl, -(CH₂)ₙCOOH and -(CH₂)ₙC(O)OCH₃;
R⁵ is selected from C₃₋₆ cycloalkyl or 3 to 6 membered heterocyclyl comprising 1 to 2 nitrogen atoms, oxygen atoms, or sulfur atoms, optionally further substituted by one or more substituents selected from the group consisting of halogen, C₁₋₃ alkyl, C₁₋₃ deuterated alkyl, C₁₋₃ cyanoalkyl, and C₁₋₃ haloalkyl;
M₁ and M₂ are each independently selected from the group consisting of N, CH and (CH)₂;
n is 0, 1, 2, or 3.

11. The compound, the stereoisomer thereof or the pharmaceutically acceptable salt thereof according to claim 4, **characterized in that** the compound is further shown as formula (V-1) and (V-2): 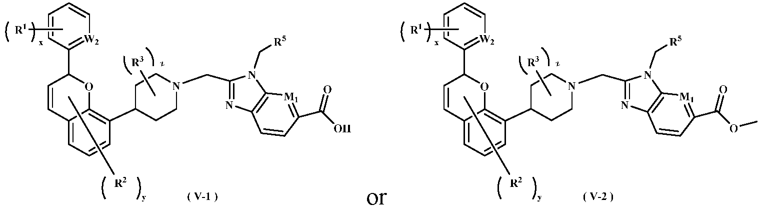 wherein:
R¹ is each independently selected from the group consisting of hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy, preferably hydrogen, deuterium, fluorine, chlorine, methoxy and -OCD₃;
R² is each independently selected from the group consisting of hydrogen, deuterium, fluorine, chlorine, cyano, C₁₋₃ alkyl, C₁₋₃ deuterated alkyl, C₁₋₃ haloalkyl, C₁₋₃ alkoxy, C₁₋₃ deuterated alkoxy, and C₁₋₃ haloalkoxy, preferably hydrogen, deuterium, fluorine, chlorine, and methyl;
R³ is each independently selected from the group consisting of hydrogen, deuterium, and fluorine, preferably hydrogen;
Mi is selected from N or CH, preferably N;
W₂ is selected from N or CH, preferably CH;
x, y and z are each independently 0, 1, or 2;
R⁵ is selected from the group consisting of 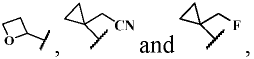 preferably

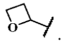

12. A compound as shown below, the stereoisomer thereof or the pharmaceutically acceptable salt thereof, wherein, the compound structure is as follows:

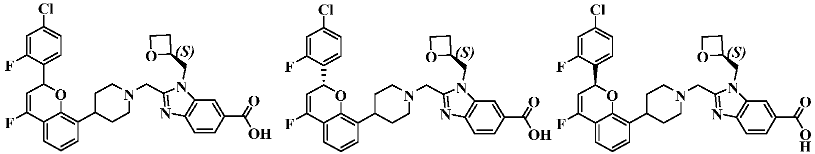

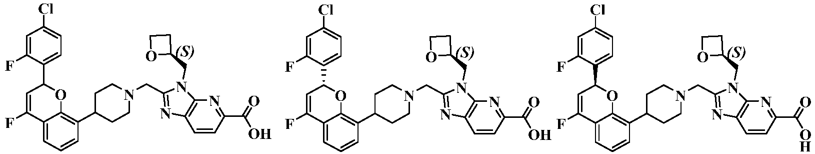

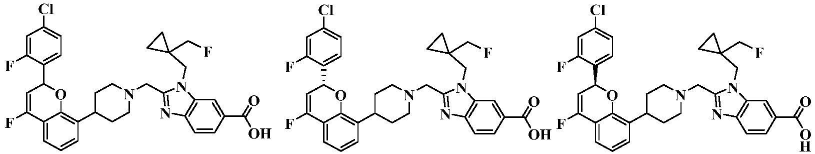

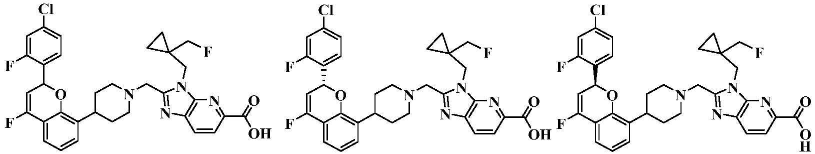

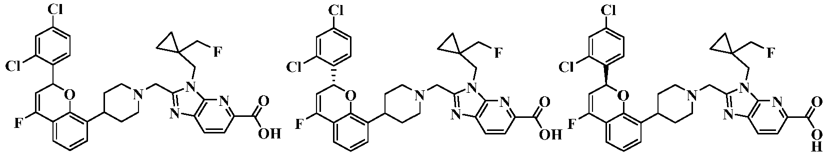

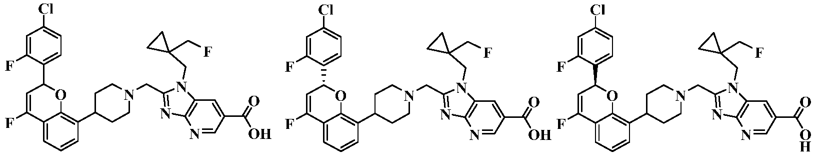

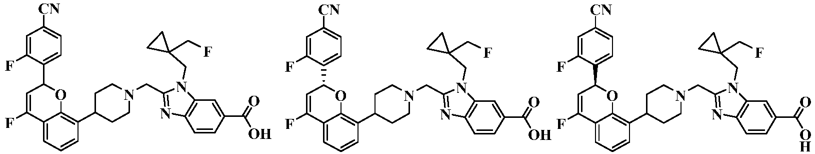

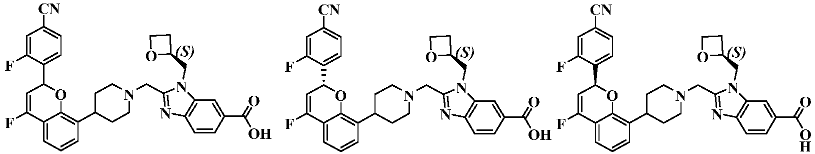

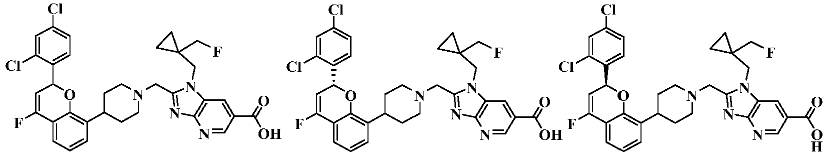

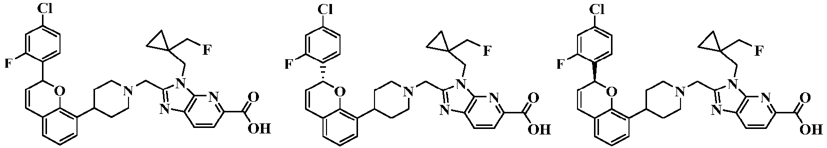

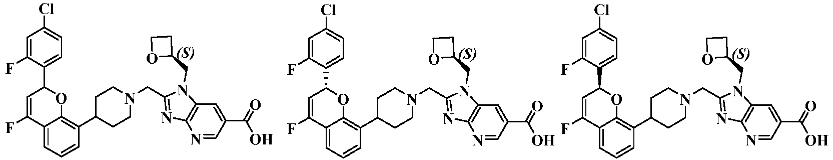

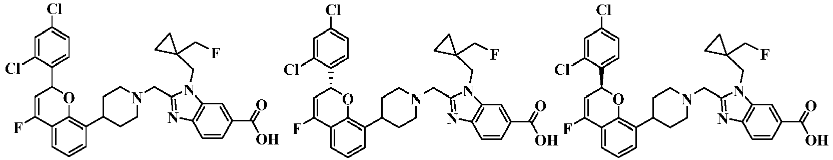

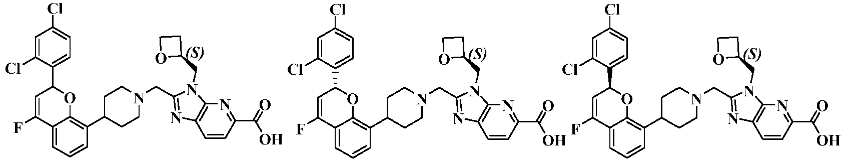

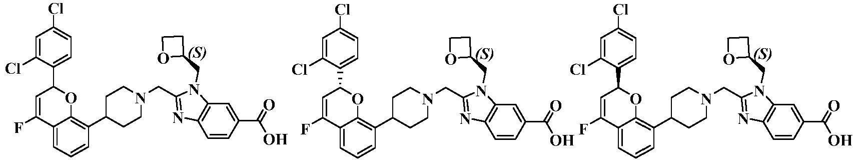

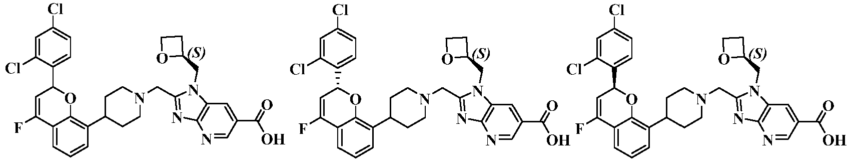

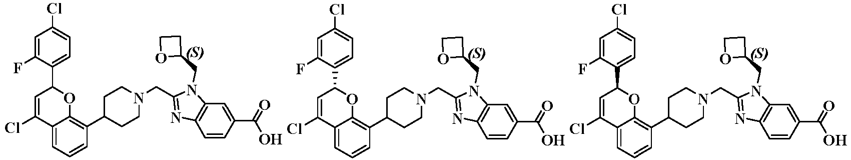

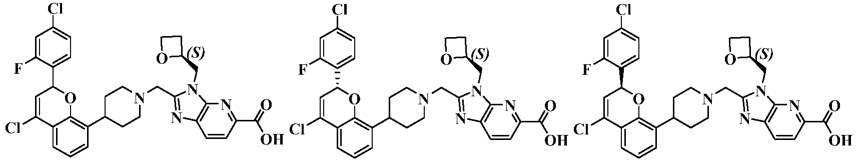

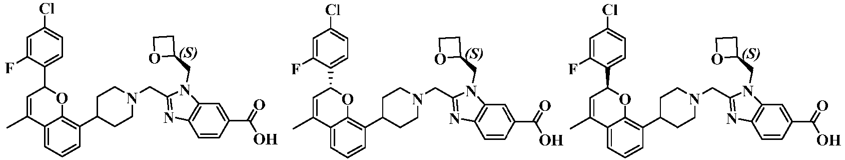

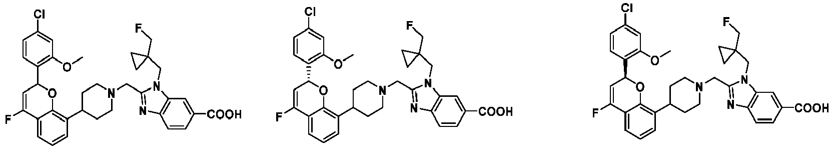

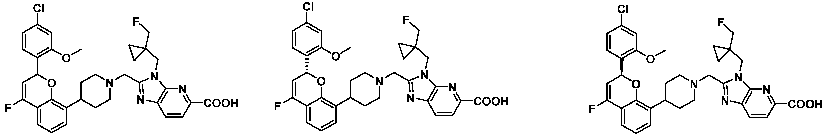

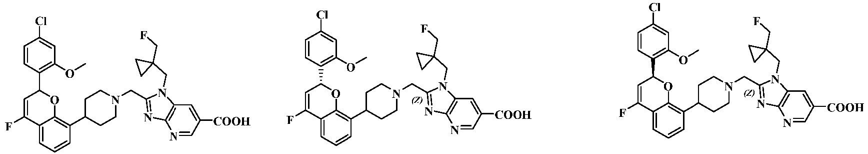

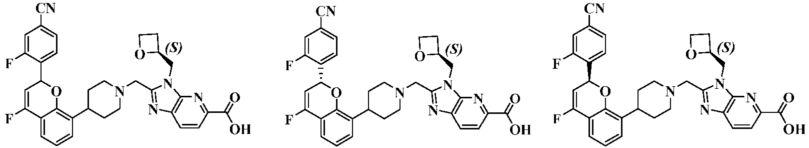

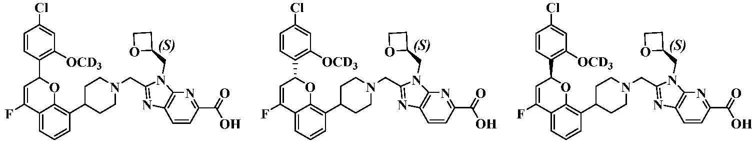

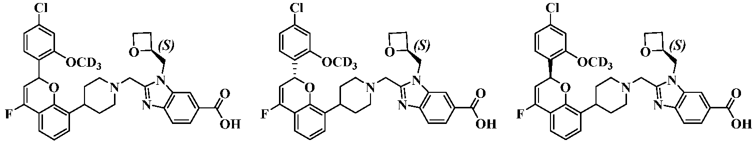

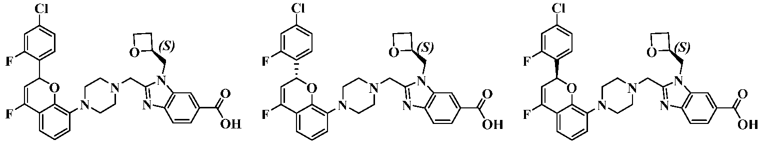

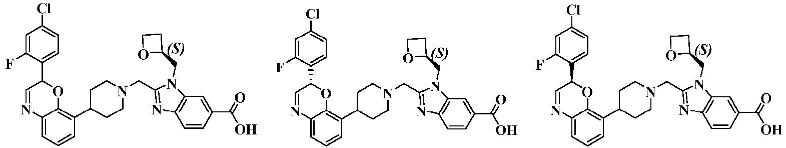

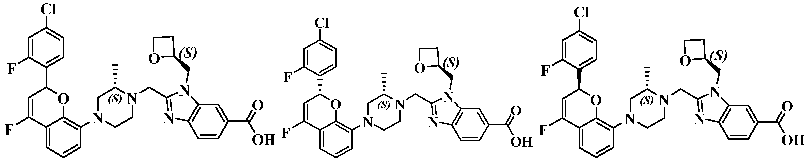

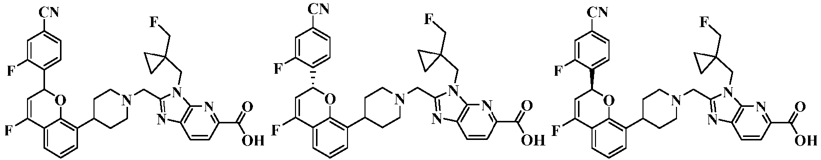

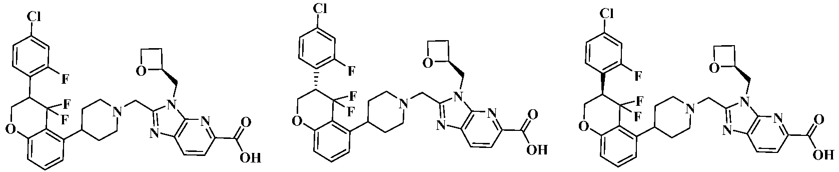

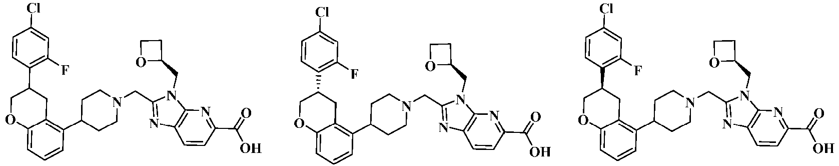

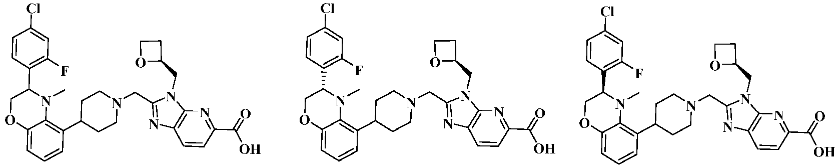

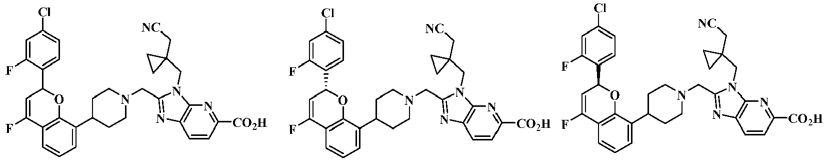

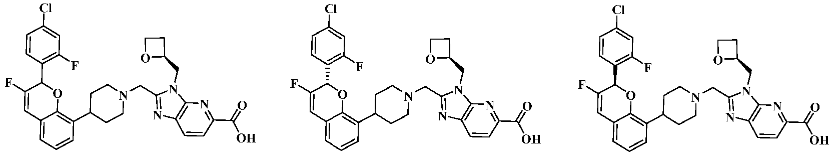

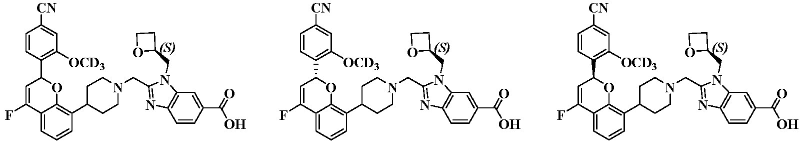

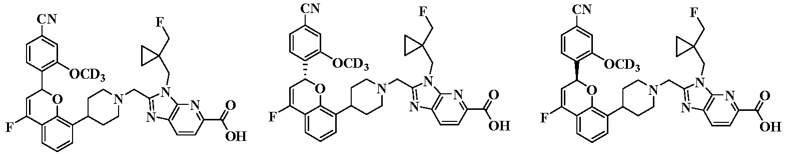

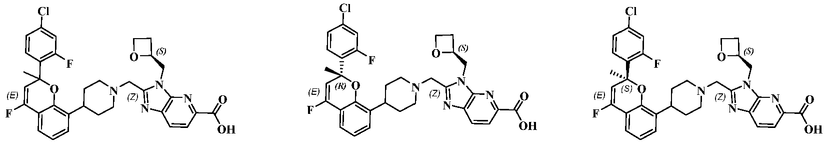

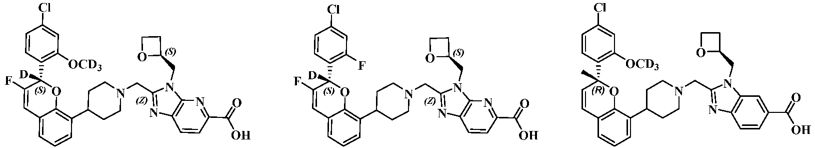

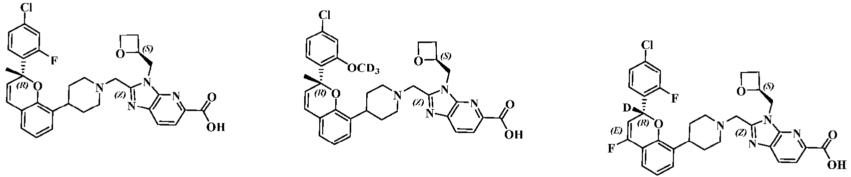

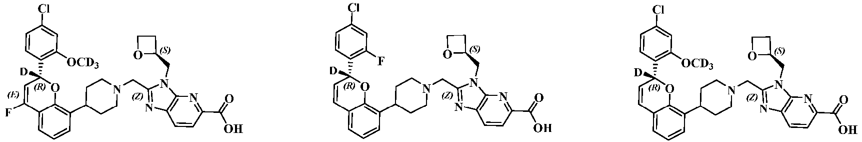

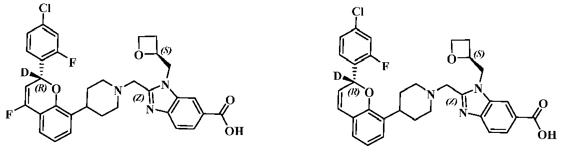

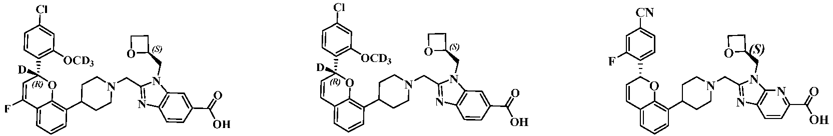

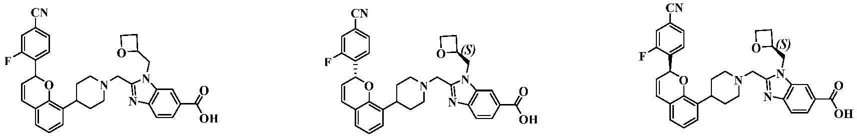

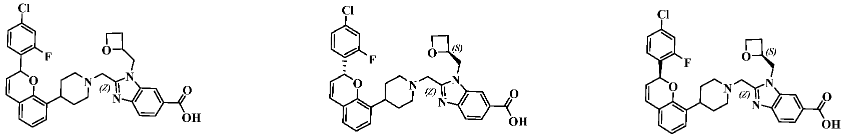

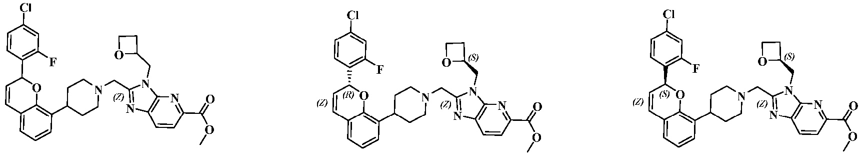

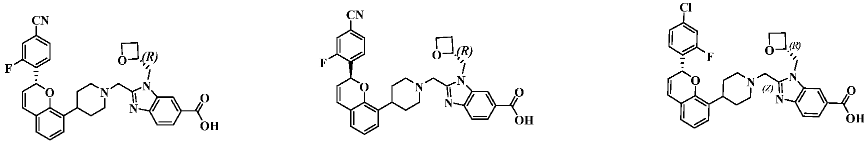

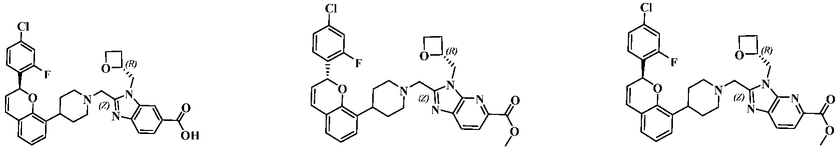

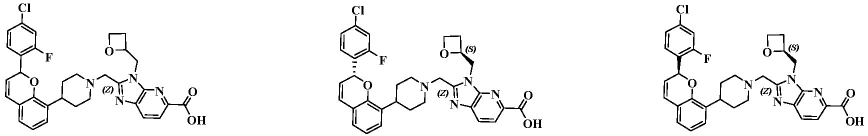

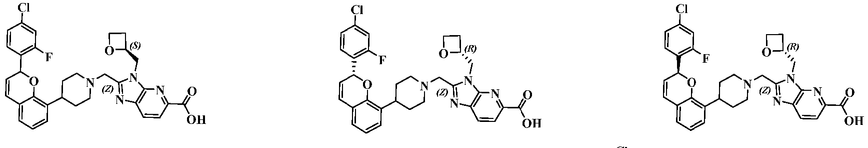

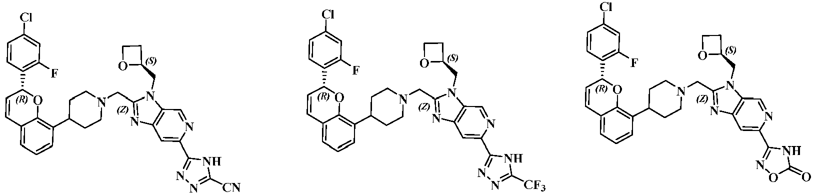

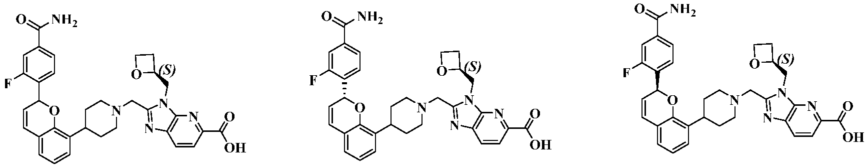

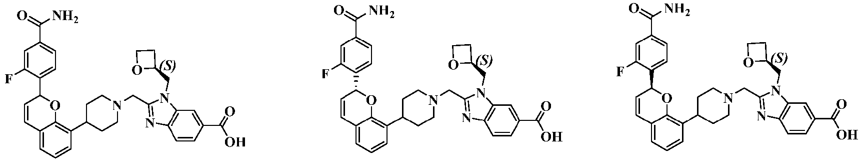

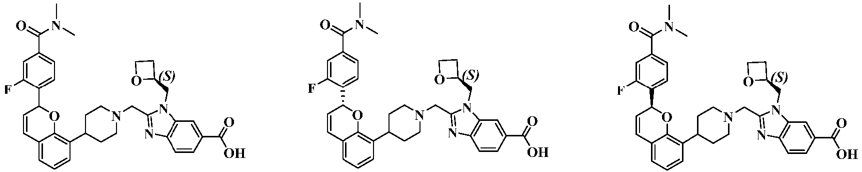

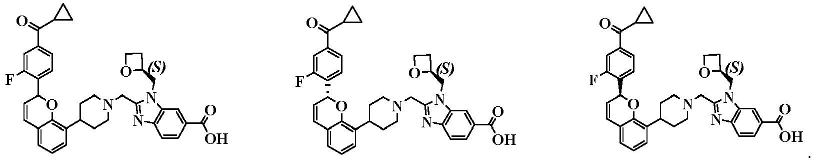

13. A pharmaceutical composition comprising a therapeutically effective dose of the compound, the stereoisomer thereof or the pharmaceutically acceptable salt thereof according to any one of claims 1 to 12, and one or more pharmaceutically acceptable carriers or excipients.

14. Use of the compound, the stereoisomer thereof or the pharmaceutically acceptable salt thereof according to any one of claims 1 to 12, or the pharmaceutical composition according to claim 13 in the preparation of a GLP-1 receptor agonist medicament.

15. Use of the compound, the stereoisomer thereof or the pharmaceutically acceptable salt thereof according to any one of claims 1 to 12, or the pharmaceutical composition according to claim 13 in the preparation of a medicament for treating metabolic related diseases; preferably wherein the metabolic related diseases are selected from diabetes, obesity or nonalcoholic steatohepatitis related diseases or other related diseases caused by diabetes, obesity or nonalcoholic steatohepatitis.
